# EUROPEAN PATENT APPLICATION

(11) **EP 4 763 837 A1**
(43) Date of publication of application: **24.06.2026**
(21) Application number: 24856315.7
(22) Date of filing: 08.08.2024
(51) Int. Cl.: C07D 311/94, C07D 405/04, C07D 413/04, C09K 9/02, G02B 5/23, G02C 7/10

(54) **PHOTOCHROMIC COMPOUND, CURABLE COMPOSITION, OPTICAL ARTICLE, LENS, AND EYEGLASSES**

(30) Priority: 18.08.2023 JP 2023133707
(71) Applicant: TOKUYAMA CORPORATION, Yamaguchi 745-8648 (JP)
(72) Inventor: MIYAZAKI, Masayuki, Shunan-shi, Yamaguchi 745-8648 (JP)
(74) Representative: Kraus & Lederer PartGmbB
(86) International application number: PCT/JP2024/028389
(87) International publication number: WO 2025/041624

(57) **Abstract**

The purpose of the present disclosure is to provide: a photochromic compound which has excellent repetition durability and excellent solubility in a matrix material; a curable composition which contains the photochromic compound; an optical article; a lens; and eyeglasses. An embodiment of the present invention provides a photochromic compound which has a skeleton represented by Formula (1). In Formula (1), M represents C, Si, or Ge. R¹ represents a hydrogen atom or a substituent. In cases where R¹ is a substituent, the substituent is not a halogen atom or a trifluoromethyl group. R² represents a hydrogen atom or a substituent. In cases where R² is a substituent, the substituent is not a halogen atom. R³ and R⁴ each independently represent a linear or branched alkyl group. R³ and R⁴ have structures that are different from each other.

## Description

### TECHNICAL FIELD

The present invention relates to a photochromic compound, a curable composition, an optical article, a lens, and eyeglasses.

### BACKGROUND ART

Photochromic compounds are compounds that can reversibly take two isomeric forms with different absorption spectra upon irradiation with light including ultraviolet light such as sunlight or light from a mercury vapor lamp. In general, a compound in a colorless or decolored state quickly changes in color and is isomerized (chromogenic reaction) to a colored or color-developed state by irradiation with ultraviolet light. For example, the photochromic compounds have been studied and developed as materials for photochromic lenses.

In applications of such photochromic lenses, the photochromic compounds may require the following properties:
(I) a degree of coloration in a visible light range before irradiation with ultraviolet light (hereinafter referred to as initial coloration) is low;
(II) a color developing density (hereinafter referred to as color developing density) quickly reaches a saturation level from the beginning of irradiation with ultraviolet light (hereinafter also referred to as high color developing sensitivity);
(III) a color quickly returns to its original state after irradiation with ultraviolet light is stopped (hereinafter referred to as color fading rate); and
(IV) repetition durability of the above-mentioned reversible action is high.

A number of chromene compounds have been studied as photochromic compounds that satisfy these properties. For example, a chromene compound represented by Formula (A) below (Patent Document 1), a chromene compound represented by Formula (B) below (Patent Document 2), and a chromene compound represented by Formula (C) below (Patent Document 3) have been known.

A photochromic lens into which such a photochromic compound is incorporated has been manufactured in various ways. For example, it is manufactured by a method for impregnating a surface of the lens with the photochromic compound. A cured product of a photochromic curable composition may be used as the photochromic lens. Furthermore, there is also a method for obtaining a photochromic laminate by laminating a photochromic layer, which is a cured product of a photochromic curable composition, on an optical substrate. Among these, the method for laminating a photochromic layer on an optical substrate is beneficial because it can be applied to a variety of optical substrates. A photochromic curable composition includes, for example, a matrix material such as (meth)acrylate or isocyanate and a photochromic compound (see Patent Documents 4, 5, 6).

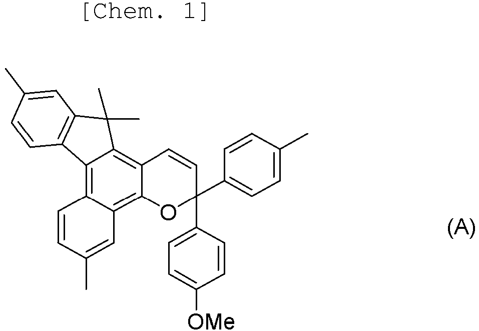

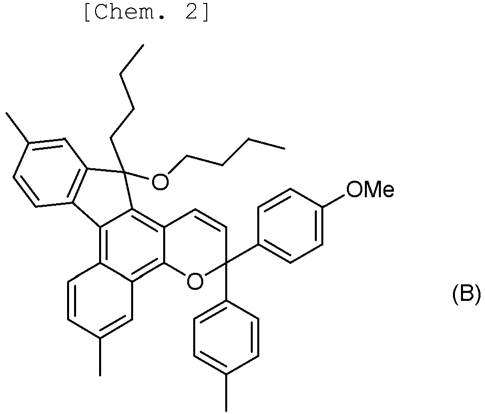

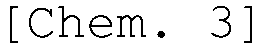

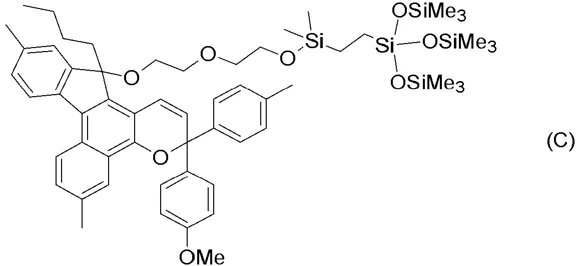

### Citation List

### Patent Documents

Patent Document 1: PCT International Publication No. WO1996/014596
Patent Document 2: PCT International Publication No. WO2004/085568
Patent Document 3: PCT International Publication No. WO2011/053615
Patent Document 4: PCT International Publication No. WO2011/125956
Patent Document 5: PCT International Publication No. WO2020/094772
Patent Document 6: Japanese Unexamined Patent Application, Publication No. 2021-59681
Patent Document 7: Japanese Unexamined Patent Application, Publication No. 2019-182866
Patent Document 8: PCT International Publication No. WO2013/042800
Patent Document 9: PCT International Publication No. WO2015/035325
Patent Document 10: PCT International Publication No. WO2018/235771
Patent Document 11: Japanese Unexamined Patent Application, Publication No. 2018-62496
Patent Document 12: PCT International Publication No. WO2021/075456
Patent Document 13: U.S. Published Patent Application Publication, No. 2006/0022176, Specification
Patent Document 14: PCT International Publication No. WO2016/143910

### DISCLOSURE OF THE INVENTION

### Problems to be Solved by the Invention

An object of the present invention is to provide a photochromic compound with excellent repetition durability and solubility in a matrix material; and a curable composition, an optical article, a lens, and eyeglasses including the photochromic compound.

### Means for Solving the Problems

According to the present disclosure, there is provided a photochromic compound having a backbone represented by Formula (3) below.

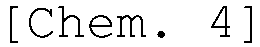

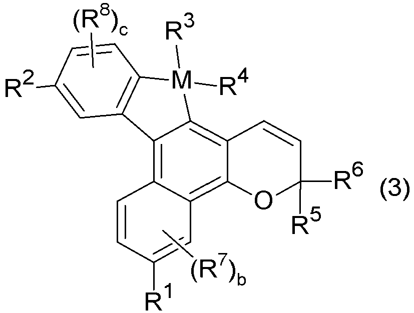

In Formula (3), M is C, Si, or Ge. R¹ is a hydrogen atom or a substituent. When R¹ is the substituent, it is not a halogen atom or a trifluoromethyl group. R² is a hydrogen atom or a substituent. When R² is the substituent, it is not a halogen atom. R³ and R⁴ are each independently a linear or branched alkyl group. R³ and R⁴ have different structures from each other. R⁵ and R⁶ are each independently a hydrogen atom or a substituent. R⁷ and R⁸ are each independently a substituent.
b and c are each independently an integer of 0, or 1 or more and 3 or less.

According to the present disclosure, a curable composition is provided. The curable composition includes a photochromic compound according to the embodiment; and at least one selected from the group consisting of a radical polymerizable monomer, a cationic polymerizable monomer, a compound having a polymerization reactive group, and a (thio)urethane(urea) polymer.

According to the present disclosure, an optical article is provided. The optical article includes a cured product of the curable composition.

According to the present disclosure, a lens is provided. The lens includes the photochromic compound according to the embodiment.

According to the present disclosure, eyeglasses are provided. The eyeglasses include the lens according to the embodiment.

### Effects of the Invention

The present invention provides a photochromic compound with excellent repetition durability and solubility; and a curable composition, an optical article, a lens, and eyeglasses including the photochromic compound.

### PREFERRED MODE FOR CARRYING OUT THE INVENTION

### [Photochromic compound]

A photochromic layer in a photochromic laminate may be required to be relatively thin in order to take advantage of a property of an optical substrate. However, a thinner photochromic layer may result in a decrease in repetition durability. Furthermore, a photochromic compound needs to be dispersed at a high concentration in the photochromic layer to exert a sufficient photochromic property in a thin film. Therefore, in addition to the above requirements, the photochromic compound may also be required to have high solubility in a matrix material and high dispersibility in a matrix. On the other hand, a photochromic compound with excessively high solubility in a matrix material is less likely to crystallize, and thus, a trace impurity is difficult to remove through purification. Such an impurity may degrade the photochromic compound, making it difficult for the photochromic compound to undergo an isomerization reaction. In other words, repetition durability is reduced.

According to an embodiment, there is provided a photochromic compound having a backbone represented by Formula (3) below:

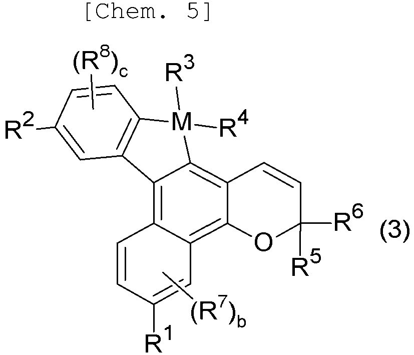

In Formula (3), M is C, Si, or Ge. R¹ is a hydrogen atom or a substituent. When R¹ is the substituent, it is not a halogen atom or a trifluoromethyl group. R² is a hydrogen atom or a substituent. When R² is the substituent, it is not a halogen atom. R³ and R⁴ are each independently a linear or branched alkyl group. R³ and R⁴ have different structures from each other. R⁵ and R⁶ are each independently a hydrogen atom or a substituent. R⁷ and R⁸ are each independently a substituent.
b and c are each independently an integer of 0, or 1 or more and 3 or less.

This photochromic compound has excellent repetition durability and solubility. The reason for this is unclear, but it is believed to be as follows by the present inventor.

R³ and R⁴ are attached to an atom M at position 13 of a photochromic compound according to the embodiment. R³ and R⁴ are alkyl groups characterized by having different structures from each other. Solubility of the photochromic compound in a matrix material is believed to be affected by both its affinity for the matrix material and flexibility of a crystal structure of the photochromic compound. The atom M at position 13 of the photochromic compound according to the embodiment is substituted with alkyl groups having different structures from each other, which moderately reduces symmetry of a molecule and weakens an intermolecular interaction of the photochromic compound. This is believed to result in a more flexible crystal structure of the photochromic compound. Since crystallinity of the photochromic compound is not completely lost, a photochromic compound with high purity can be obtained by, for example, a recrystallization method. Therefore, it is believed that a decrease in repetition durability due to degradation of the photochromic compound caused by the impurity may also be suppressed. Thus, the photochromic compound according to the embodiment can exhibit high durability while achieving high solubility in various matrix materials.

Furthermore, as a result of extensive studies, the present inventor has also found that durability of a photochromic compound is increased by introducing substituents R³ and R⁴ into an atom M at position 13 without an intervening oxygen atom (O). In other words, in a photochromic compound according to the embodiment, an alkyl group is directly attached to an atom M at position 13, and no oxygen atom is attached thereto.

Therefore, the photochromic compound according to the embodiment is more durable compared to a photochromic compound having a substituent introduced with an intervening oxygen atom such as an alkoxy group.

In light of the above, the photochromic compound according to the embodiment can be used to provide a photochromic curable compound with excellent solubility in a curable composition and thus achieve a cured product or an optical article with excellent durability.

A photochromic compound having a backbone represented by Formula (3) will be described in detail.

### <M>

In Formula (3), M is C, Si, or Ge. M is preferably C.

### <R¹>

R¹ is a hydrogen atom or a substituent. If R¹ is the substituent, it is a group other than a halogen atom or a trifluoromethyl group. In other words, R¹ is a substituent except for a halogen atom or a trifluoromethyl group, or a hydrogen atom.

R¹ is preferably a hydrogen atom, a hydroxyl group, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) below, a group represented by Formula (X) below, or a group represented by Formula (X3) below.

R¹ is more preferably a hydrogen atom, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a substituted or unsubstituted aryloxy group, a substituted or unsubstituted aryl group, a substituted or unsubstituted heteroaryl group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) below, a group represented by Formula (X) below, or a group represented by Formula (X3) below.

The alkyl group is preferably a substituted or unsubstituted alkyl group having 1 to 20 carbon atoms, more preferably an unsubstituted alkyl group having 1 to 10 carbon atoms, and further preferably an unsubstituted alkyl group having 1 to 6 carbon atoms. Specific examples of the alkyl group include a methyl group, an ethyl group, a propyl group, a butyl group, a pentyl group, or a hexyl group.

The cycloalkyl group is preferably a cycloalkyl group having 3 to 8 carbon atoms (a cycloalkyl group in which 3 to 8 carbon atoms form a ring). Examples of the cycloalkyl group having 3 to 8 carbon atoms may include a cyclopropyl group, a cyclobutyl group, a cyclopentyl group, or a cyclohexyl group, etc. Note that, the cycloalkyl group may have a substituent, but a number of carbon atoms (3 to 8 carbon atoms) shall not include a number of carbon atoms on the substituent.

The alkoxy group is preferably a substituted or unsubstituted alkoxy group having 1 to 20 carbon atoms, more preferably an unsubstituted alkoxy group having 1 to 10 carbon atoms, and particularly preferably an alkoxy group having 1 to 6 carbon atoms. Suitable examples of the alkoxy group having 1 to 6 carbon atoms include a methoxy group, an ethoxy group, an n-propoxy group, an isopropoxy group, an n-butoxy group, a sec-butoxy group, a tert-butoxy group, etc.

The amino group may be a primary amino group (-NH₂), or a secondary or tertiary amino group with one or two hydrogen atoms being substituted. Examples of a substituent that the substituted amino group has include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc. Suitable examples of the amino group include an amino group, a methylamino group, a dimethylamino group, an ethylamino group, a diethylamino group, a methylphenylamino group, a diphenylamino group, etc.

The heterocyclic group preferably has 3 to 10 atoms. A heteroatom in the heterocyclic group is preferably at least one selected from the group consisting of an oxygen atom, a nitrogen atom, a sulfur atom, and a phosphorus atom. A number of heteroatoms is, for example, 1 or more and 5 or less and preferably 1 or 2. Specifically, for example, an aliphatic heterocyclic group such as a morpholino group, a piperidino group, a pyrrolidinyl group, a piperazino group, or an N-methylpiperazino group; or an aromatic heterocyclic group such as an indolinyl group can be used. The heterocyclic group may be a 2,6-dimethylmorpholino group, a 2,6-dimethylpiperidino group, or a 2,2,6,6-tetramethylpiperidino group.

The alkylthio group is preferably an alkylthio group having 1 to 6 carbon atoms. The alkylthio group having 1 to 6 carbon atoms may include a methylthio group, an ethylthio group, an n-propylthio group, an isopropylthio group, an n-butylthio group, a sec-butylthio group, a t-butylthio group, etc.

The arylthio group is preferably an arylthio group having 6 to 10 carbon atoms. The arylthio group having 6 to 10 carbon atoms may include a phenylthio group, a 1-naphthylthio group, or a 2-naphthylthio group.

The alkylcarbonyl group is preferably an alkylcarbonyl group having 2 to 7 carbon atoms. The alkylcarbonyl group having 2 to 7 carbon atoms may include an acetyl group or an ethylcarbonyl group.

The alkoxycarbonyl group is preferably an alkoxycarbonyl group having 2 to 7 carbon atoms. Examples of the alkoxycarbonyl group having 2 to 7 carbon atoms include a methoxycarbonyl group, an ethoxycarbonyl group, etc.

The aralkyl group is preferably an aralkyl group having 7 to 11 carbon atoms. The aralkyl group having 7 to 11 carbon atoms may include a benzyl group, a phenylethyl group, a phenylpropyl group, a phenylbutyl group, or a naphthylmethyl group.

The aralkoxy group is preferably an aralkoxy group having 7 to 11 carbon atoms. The aralkoxy group having 7 to 11 carbon atoms may include a benzyloxy group or a naphthylmethoxy group.

The aryloxy group is preferably an aryloxy group having 6 to 12 carbon atoms. The aryloxy group having 6 to 12 carbon atoms may include a phenyloxy group or a naphthyloxy group.

The aryl group is not particularly limited, and is preferably an aryl group having 5 to 12 carbon atoms. The aryl group having 5 to 12 carbon atoms may include a phenyl group, a 1-naphthyl group, or a 2-naphthyl group.

The heteroaryl group is not particularly limited, and is preferably a heteroaryl group having 3 to 12 carbon atoms. A heteroatom may be at least one selected from the group consisting of an oxygen atom, a sulfur atom, a nitrogen atom, and a phosphorus atom. A number of heteroatoms in the substituted or unsubstituted heteroaryl group is, for example, 1 or more and 3 or less and preferably 1 or 2. A number of carbon atoms in the substituted or unsubstituted heteroaryl group is 4 or more and 11 or less carbon atoms and preferably 5 or more and 9 or less carbon atoms. The heteroaryl group having 3 to 12 carbon atoms may include a thienyl group, a furyl group, a pyrrolinyl group, a pyridyl group, a benzothienyl group, a benzofuranyl group, or a benzopyrrolinyl group.

The alkoxyalkylthio group is preferably an alkoxyalkylthio group having 2 to 9 carbon atoms. The alkoxyalkylthio group having 2 to 9 carbon atoms may include a methoxymethylthio group, a methoxyethylthio group, a methoxy n-propylthio group, a methoxy n-butylthio group, an ethoxyethylthio group, or an n-propoxypropylthio group.

The haloalkylthio group is preferably a haloalkylthio group having 1 to 6 carbon atoms. The haloalkylthio group having 1 to 6 carbon atoms may include a trifluoromethylthio group, a tetrafluoroethylthio group, a chloromethylthio group, a 2-chloroethylthio group, or a bromomethylthio group.

The cycloalkylthio group is preferably a cycloalkylthio group having 3 to 8 carbon atoms. The cycloalkylthio group having 3 to 8 carbon atoms may include a cyclopropylthio group, a cyclobutylthio group, a cyclopentylthio group, or a cyclohexylthio group. Note that, the cycloalkylthio group may have a substituent, but a number of carbon atoms (3 to 8 carbon atoms) shall not include a number of carbon atoms on the substituent.

The silyl group may be a substituted silyl group. A substituent that the substituted silyl group has is not particularly limited, and includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, or a heteroaryl group having 4 to 14 carbon atoms.

The oxysilyl group may be a substituted oxysilyl group. A substituent that the substituted oxysilyl group has is not particularly limited, and includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, or a heteroaryl group having 4 to 14 carbon atoms.

(Group represented by Formula (2a))

-Q¹-(X¹Q²)ₐ-X²Q³ (2a)

In Formula (2a), Q¹ is an alkylene group or a haloalkylene group.

The alkylene group has preferably 1 to 20 carbon atoms, more preferably 1 to 12 carbon atoms, further preferably 1 to 7 carbon atoms, and most preferably 2 to 6 carbon atoms. At least one halogen atom selected from the group consisting of I, Cl, Br, and F may be used in the haloalkylene group. The halogen atom is preferably at least one of Cl or F and more preferably F. The haloalkylene group preferably has a halogen atom bonded to a terminal carbon atom and more preferably has a terminal perfluoromethyl group.

Q² is an alkylene group or a haloalkylene group. A preferred aspect of the alkylene group or the haloalkylene group is the same as for Q¹. A number of carbon atoms in the alkylene group or the haloalkylene group for Q² may be the same as or different from that of the alkylene group or the haloalkylene group for Q¹.

Q³ is an alkyl group or a haloalkyl group and may be linear or branched, but is preferably linear.

The alkyl group has preferably 1 to 20 carbon atoms, more preferably 1 to 12 carbon atoms, and most preferably 1 to 7 carbon atoms. At least one halogen atom selected from the group consisting of I, Cl, Br, and F may be used in the haloalkyl group. The halogen atom is preferably at least one of Cl or F and more preferably F.

Q³ is preferably a linear alkyl group.

X¹ and X² are each independently O, S, NR⁷⁰⁰, PR⁷⁰¹, or P(=O). X¹ and X² are each preferably O, S, or NR⁷⁰⁰ and most preferably O.

R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.

R⁷⁰⁰ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

R⁷⁰¹ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

a is an integer of 0, or 1 or more and 3 or less.

Formula (2a) is preferably an alkylene alkoxy group, an alkylenethioalkyl group, or an alkyleneoxyalkoxyalkoxy group.

Specific examples of Formula (2a) include -CH₂OCH₃, - CH₂SCH₃, -CH₂CH₂OCH₃, -CH₂CH₂OCH₂CH₃, -CH₂CH₂SCH₃, -CH₂CH₂CH₂OCH₃, - CH₂CH₂CH₂SCH₃, -CH₂CH₂OCH₂CH₂OCH₃, and -CH₂CH₂OCH₂CH₂OCH₂CH₃.

The group represented by Formula (2a) is preferably - CH₂OCH₃, -CH₂CH₂OCH₃, -CH₂CH₂OCH₂CH₃, -CH₂CH₂CH₂OCH₃, - CH₂CH₂OCH₂CH₂OCH₃, or -CH₂CH₂OCH₂CH₂OCH₂CH₃.

### (Group represented by Formula (X))

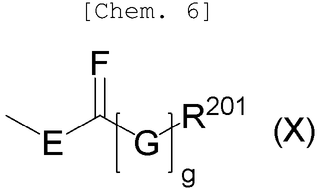

In Formula (X), E is an oxygen atom or NR¹⁰¹. R¹⁰¹ is a hydrogen atom or an alkyl group. E is NR¹⁰¹ in which R¹⁰¹ is preferably a hydrogen atom or an alkyl group having 1 to 6 carbon atoms.

F is an oxygen atom or a sulfur atom. F is preferably an oxygen atom.

G is an oxygen atom, a sulfur atom, or NR²⁰². R²⁰² is a hydrogen atom, an alkyl group, a cycloalkyl group, an aryl group, or a heteroaryl group. G is preferably NH.

### g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than a hydrogen atom. R²⁰¹ is preferably an alkyl group having 1 to 6 carbon atoms, an alkoxy group having 1 to 6 carbon atoms, or an aryl group having 6 to 12 carbon atoms.

A suitable group represented by Formula (X) is as follows:

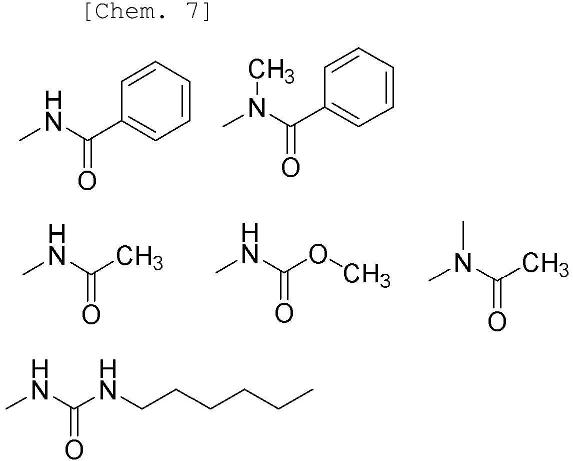

(Group represented by Formula (X3))

L¹-R⁴⁰⁰ (X3)

In Formula (X3),
R⁴⁰⁰ is a hydrogen atom, an alkyl group, an aryl group, a polymerizable group, a photochromic group, or a silyl group having an alkyl group, an alkoxyl group, or an aryl group as a substituent.

L¹ is a group represented by Formula (X2) below:

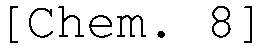

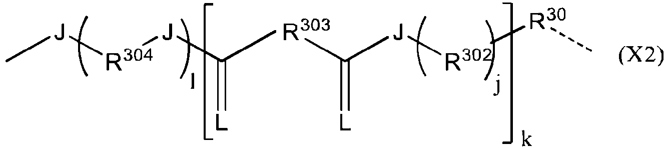

In Formula (X2), R³⁰ is a group represented by Formula (X2a) below.

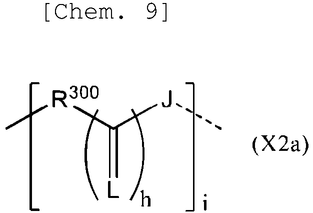

In Formulae (X2) and (X2a), J is a divalent group. A plurality of Js are each independently directly attached, a substituted or unsubstituted methylene group, an oxygen atom, a sulfur atom, or NR³⁰¹. R³⁰¹ is a hydrogen atom or an alkyl group. R³⁰¹ is preferably an alkyl group having 1 to 20 carbon atoms. The alkyl group preferably has a silyl group having an alkyl group having 1 to 10 carbon atoms; a polymerizable group, or a photochromic group as a substituent.

Examples of the polymerizable group include a radical polymerizable group such as a vinyl group, a 1-chlorovinyl group, an allyl group, a styryl group, a (meth)acryl group, a 2-(methacryloxy)ethylcarbamyl group, a 2-(methacryloxy)ethoxycarbonyl group, or a crotyl group. Other than the above-mentioned groups, an epoxy group, an episulfide group, a thietanyl group, an OH group, an SH group, an NH₂ group, a COOH group, an NCO group, or an NCS group may also be used. The polymerizable group is preferably at least one selected from the group consisting of a (meth)acrylic group, a 2-(methacryloxy)ethylcarbamyl group, a 2-(methacryloxy)ethoxycarbonyl group, an epoxy group, an OH group, an SH group, an NH₂ group, and a COOH group.

The photochromic group is a group containing a photochromic moiety. As a representative example, the photochromic group is exemplified by naphthopyran, spirooxazine, spiropyran, fulgide, fulgimide, or diarylethene. Indenonaphthopyran is preferred from the viewpoint of its ability to develop an excellent photochromic property, and indeno[2,1-f]naphtho[1,2-b]pyran is particularly preferred.

Indeno[2,1-f]naphtho[1,2-b]pyran is preferably a group represented by Formula (X4) below:

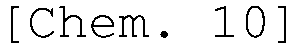

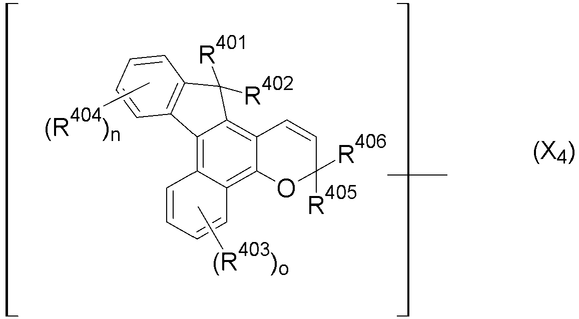

In Formula (X4), R⁴⁰¹ and R⁴⁰² may be the same as groups to be used for R³ and R⁴ shown below.

R⁴⁰³ and R⁴⁰⁴ each independently may be the same as groups to be used for R³ and R⁴ shown below.

R⁴⁰⁵ and R⁴⁰⁶ are each independently a substituted or unsubstituted aryl group or a substituted or unsubstituted heteroaryl group. R⁴⁰⁵ and R⁴⁰⁶ may be the same as groups to be used for R⁵ and R⁶ described below.

In Formula (X4), o is an integer of 0 or more and 4 or less. n is an integer of 0 or more and 4 or less. When o is 2 or more and 4 or less, a plurality of R⁴⁰³s may be the same as or different from each other. When n is 2 or more and 4 or less, a plurality of R⁴⁰⁴s may be the same as or different from each other.

When R⁴⁰¹, R⁴⁰², R⁴⁰³, R⁴⁰⁴, or R⁴⁰⁵ is the group represented by Formula (X3), at least one of substituents on the aryl group or the heteroaryl group is attached to L¹.

A particularly suitable group represented by Formula (X2) is represented by any of the following formulae:

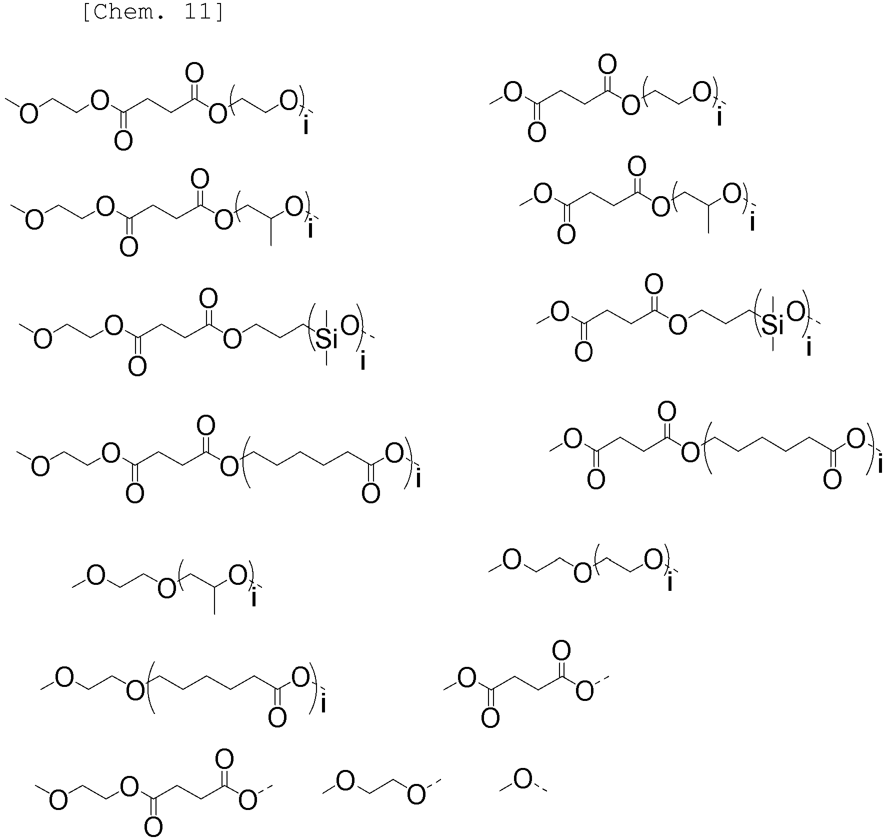

In Formula (X2), L is an oxygen atom or a sulfur atom.

R³⁰⁰ is an alkylene group, or a silylene group having an alkyl group or an aryl group as a substituent. R³⁰⁰ is preferably an alkylene group having 1 to 6 carbon atoms or a silylene group having an alkyl group having 1 to 6 carbon atoms as a substituent.

R³⁰², R³⁰³, and R³⁰⁴ are each independently an alkylene group. R³⁰² is preferably an alkylene group having 1 to 6 carbon atoms. R³⁰³ is preferably an alkylene group having 1 to 6 carbon atoms. R³⁰⁴ is preferably an alkylene group having 1 to 6 carbon atoms.

h, j, k, and 1 are each independently 0 or 1.

i is an integer of 0 or more and 200 or less. A plurality of groups each with a subscript i may have structures identical to or different from each other.
i is preferably in a range of 5 to 100, more preferably 8 to 75, and most preferably 10 to 70.

In Formula (X2), a dashed line represents an attachment to R⁴⁰⁰.

### <R²>

R² is a hydrogen atom or a substituent. If R² is the substituent, it is a group other than a halogen atom. In other words, R² is a substituent except for a halogen atom, or a hydrogen atom.

R² is preferably a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a), a group represented by Formula (X), or a group represented by Formula (X3).

The haloalkyl group is preferably a haloalkyl group having 1 to 6 carbon atoms. A number of halogen atoms is preferably 1 or more and 10 or less and more preferably 2 or more and 5 or less. The haloalkyl group having 1 to 6 carbon atoms is preferably an alkyl group substituted with a fluorine atom, a chlorine atom, or a bromine atom. The haloalkyl group preferably has a terminal perfluoromethyl group. Suitable examples of the haloalkyl group may include a trifluoromethyl group, a trifluoroethyl group, a trifluoropropyl group, a tetrafluoroethyl group, a chloromethyl group, a 2-chloroethyl group, or a bromomethyl group.

Specific suitable examples of the alkyl group, the cycloalkyl group, the alkoxy group, the amino group, the heterocyclic group, the alkylthio group, the arylthio group, the alkyl carbonyl group, the alkoxycarbonyl group, the aralkyl group, the aralkoxy group, the aryloxy group, the heteroaryl group, the silyl group, the oxysilyl group, the group represented by Formula (2a), the group represented by Formula (X), or the group represented by Formula (X3) are the same as those exemplified for R¹.

### <R³ and R⁴>

R³ and R⁴ are each independently a linear or branched unsubstituted alkyl group and have different structures from each other. A number of carbon atoms in the alkyl group for R⁴ can be equal to or larger than a number of carbon atoms in the alkyl group for R³.

In other words, if R³ is a linear alkyl group, then R⁴ is a linear alkyl group having a larger number of carbon atoms than R³, or a branched alkyl group in which a number of carbon atoms is equal to or larger than in R³.

If R³ is a branched alkyl group, then R⁴ is a linear alkyl group in which a number of carbon atoms is equal to or larger than in R³. Alternatively, R⁴ is a branched alkyl group in which a number of carbon atoms is equal to or larger than in R³ and which has a structure different from R³.

Furthermore, if both R³ and R⁴ are branched alkyl groups each having 4 or more carbon atoms, a number of carbon atoms in a main chain of R³ may be larger than a number of carbon atoms in a main chain of R⁴, with the proviso that a number of carbon atoms in the alkyl group for R⁴ equals to or larger than a number of carbon atoms in the alkyl group for R³. When R³ and R⁴ have the same number of carbon atoms in their main chains and are alkyl groups having the same number of carbon atoms, a branched alkyl group in which an alkyl group branched from the main chain is substituted at a position closer to M in Formula (3) is determined as R³.

For example, when branched alkyl groups substituted at M are an s-butyl group and an isobutyl group, the s-butyl group with a methyl group in a branched chain being substituted at a position closer to M is determined as R³ and the isobutyl group is determined as R⁴.

R³ preferably has 1 to 19 carbon atoms, more preferably 1 to 10 carbon atoms, further preferably 1 to 9 carbon atoms, particularly preferably 1 to 6 carbon atoms, and most preferably 1 to 3 carbon atoms. A smaller number of carbon atoms tends to increase repetition durability. A larger number of carbon atoms tends to increase solubility. R³ is preferably a linear alkyl group.

When R³ is a branched alkyl group, a number of branches is preferably 5 or less, preferably 3 or less, and more preferably 1.

A number of carbon atoms in a side chain is preferably 5 or less, preferably 3 or less, more preferably 2 or less, and particularly preferably 1.

R⁴ preferably has 2 to 20 carbon atoms, more preferably 2 to 11 carbon atoms, further preferably 2 to 10 carbon atoms, particularly preferably 2 to 7 carbon atoms, and most preferably 2 to 4 carbon atoms. A smaller number of carbon atoms tends to increase repetition durability. A larger number of carbon atoms tends to increase solubility. R⁴ is preferably a linear alkyl group.

A value obtained by subtracting a number of carbon atoms in the alkyl group for R³ from a number of carbon atoms in the alkyl group for R⁴ is preferably 1 or more and 15 or less, more preferably 1 or more and 10 or less, more preferably 1 or more and 9 or less, further preferably 1 or more and 5 or less, particularly preferably 1 or more and 3 or less, and most preferably 1 or more and 2 or less. The smaller the value is, the higher repetition durability tends to be. The larger the value is, the higher solubility tends to be.

For a combination of R³ and R⁴, preferably, R³ is a linear or branched alkyl group having 1 to 19 carbon atoms and R⁴ is a linear or branched alkyl group having 2 to 20 carbon atoms; more preferably, R³ is a linear alkyl group having 1 to 19 carbon atoms and R⁴ is a linear or branched alkyl group having 2 to 20 carbon atoms; further preferably, R³ is a linear alkyl group having 1 to 19 carbon atoms and R⁴ is a linear alkyl group having 2 to 20 carbon atoms; more preferably, R³ is a linear alkyl group having 1 to 9 carbon atoms and R⁴ is a linear alkyl group having 2 to 10 carbon atoms; more preferably, R³ is a linear alkyl group having 1 to 6 carbon atoms and R⁴ is a linear alkyl group having 2 to 10 carbon atoms; more preferably, R³ is a linear alkyl group having 1 to 3 carbon atoms and R⁴ is a linear alkyl group having 2 to 10 carbon atoms; more preferably, R³ is a linear alkyl group having 1 to 3 carbon atoms and R⁴ is a linear alkyl group having 2 to 7 carbon atoms; more preferably, R³ is a linear alkyl group having 1 to 3 carbon atoms and R⁴ is a linear alkyl group having 2 to 4 carbon atoms; and most preferably, one of R³ or R⁴ is an ethyl group.

### <R⁷ and R⁸>

R⁷ and R⁸ are each independently a substituent. b and c are each independently an integer of 0, or 1 or more and 3 or less. In Formula (2), for example, R⁷ and R⁸ each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a), a group represented by Formula (X), or a group represented by Formula (X3). These groups may be the same as those exemplified for R¹ to R⁴.

In Formula (2), when b is 2 to 3, a plurality of R⁷s may be the same as or different from each other.

In Formula (2), when b is 2 to 3 and adjacent R⁷s are present, two adjacent R⁷s may be taken together with a carbon atom to which R⁷s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings. A combination of the adjacent R⁷s may be R⁷s at positions 7 and 8 of the naphthopyran backbone.

Furthermore, when R¹ and R⁷ adjacent thereto are present, the adjacent R¹ and R⁷ may be taken together with a carbon atom to which they are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings. A combination of the adjacent R¹ and R⁷ may be R¹ and R⁷ at positions 5 and 6 or positions 6 and 7 of the naphthopyran backbone.

In Formula (2), when c is 2 to 3, a plurality of R⁸s may be the same as or different from each other.

In Formula (2), when c is 2 to 3 and adjacent R⁸s are present, two adjacent R⁸s may be taken together with a carbon atom to which R⁸s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings. A combination of the adjacent R⁸s may be R⁸s at positions 11 and 12 of the naphthopyran compound.

Furthermore, when R² and R⁸ adjacent thereto are present, the adjacent R² and R⁸ may be taken together with a carbon atom to which they are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings. A combination of the adjacent R² and R⁸ may be R² and R⁸ at positions 9 and 10 or positions 10 and 11 of the naphthopyran backbone.

A ring having 5 to 8 atoms including a carbon atom to which R⁷ and R⁸ are attached may be formed. The ring may have a substituent and the substituent may be a substituent selected from a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of the substituent will be described below.

Suitable ring that R⁷ or R⁸ forms includes a ring represented by Formula (X5) below.

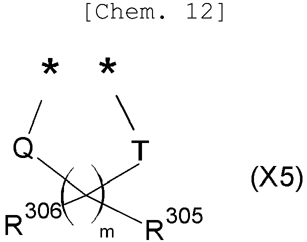

In Formula (X5), Q and T are each independently a sulfur atom, a substituted or unsubstituted methylene group, an oxygen atom, or a group represented by NR³⁰⁷. R³⁰⁷ is a hydrogen atom, a hydroxyl group, an alkyl group, a haloalkyl group, a cycloalkyl group, an alkoxy group, a substituted or unsubstituted aryl group, a substituted or unsubstituted heteroaryl group, or a group represented by Formula (2a).

R³⁰⁵ and R³⁰⁶ are each independently preferably a hydroxy group, an alkyl group, a haloalkyl group, a cycloalkyl group, an alkoxy group, an amino group, a substituted amino group, a substituted or unsubstituted heterocyclic group, a cyano group, a nitro group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryl group, a thiol group, an alkylthio group, an alkoxyalkylthio group, a haloalkylthio group, a cycloalkylthio group, or a substituted or unsubstituted arylthio group.

In addition, R³⁰⁵ and R³⁰⁶ may be taken together with a carbon atom to which they are attached to form a substituted or unsubstituted aliphatic ring. Specific examples of the aliphatic ring include a cyclopentane ring, a cyclohexane ring, etc. For the aliphatic ring, 1 to 8 hydrogen atoms, particularly preferably 1 to 4 hydrogen atoms may be substituted with at least one group selected from the group consisting of a hydroxyl group, an alkyl group, a haloalkyl group, a cycloalkyl group, an alkoxy group, an amino group, a substituted amino group, a heterocyclic group, a cyano group, a nitro group, and a halogen atom. Specific examples of the substituent will be described below.

In Formula (X5), m is an integer of 1 or more and 4 or less.

### <R⁵ and R⁶>

R⁵ and R⁶ are each independently a hydrogen atom or a substituent. The substituent may be those exemplified for R¹. In Formula (3), for example, R⁵ and R⁶ are each independently a substituted or unsubstituted aryl group or a substituted or unsubstituted heteroaryl group. These groups may be the same as those exemplified for R¹.

R⁵ and R⁶ are each independently preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or substituted or unsubstituted benzopyrrolinyl group. Furthermore, it is preferred that at least one of R⁵ or R⁶ be a substituted or unsubstituted phenyl group, and it is more preferred that both R⁵ and R⁶ be substituted phenyl groups.

A substituent that the phenyl group has is preferably a group represented by Formula (2a), a hydroxyl group, an alkyl group, a haloalkyl group, a substituted or unsubstituted cycloalkyl group, an alkoxy group, an amino group, a substituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, or a group represented by Formula (X3).

The substituent that the phenyl group has is more preferably an alkyl group, a haloalkyl group, an alkoxy group, an amino group, a substituted amino group, a substituted or unsubstituted heterocyclic group, a cyano group, a halogen atom, an alkylthio group, a substituted or unsubstituted arylthio group, a substituted or unsubstituted aryloxy group, a substituted or unsubstituted aryl group, a substituted or unsubstituted heteroaryl group, an alkoxyalkylthio group, a haloalkylthio group, a substituted or unsubstituted silyl group, a substituted or unsubstituted oxysilyl group, or a group represented by Formula (X3).

### <Photochromic compound represented by Formula (4)>

A suitable photochromic compound includes a compound represented by Formula (4) below:

In Formula (4), R¹, R², R³, R⁴, R⁷, R⁸, b, c, and M are each independently the same as in Formula (3).

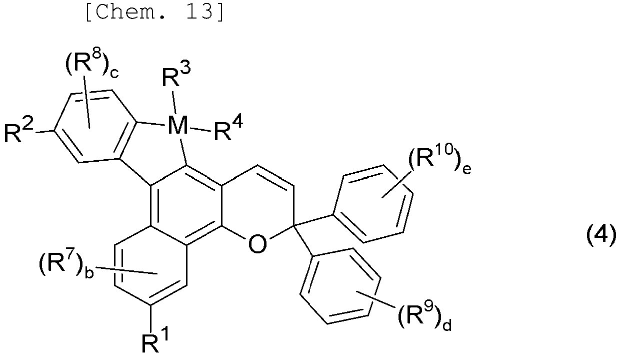

### <R⁹ and R¹⁰>

R⁹ and R¹⁰ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by Formula (2a), or a group represented by Formula (X3).

d denotes a number of R⁹ and is an integer of 0 or more and 5 or less. When d is 2 or more, R⁹s may be the same as or different from each other.

When d is 2 or more and 5 or less and adjacent R⁹s are present, two adjacent R⁹s may be taken together with a carbon atom to which R⁹s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings.

e denotes a number of R¹⁰ and is an integer of 0 or more and 5 or less. When e is 2 or more, R¹⁰s may be the same as or different from each other.

When e is 2 or more and 5 or less and adjacent R¹⁰s are present, two adjacent R¹⁰s may be taken together with a carbon atom to which R¹⁰s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings.

For R⁹ and R¹⁰, each independently, adjacent two may be taken together to form a ring group optionally including at least one selected from the group consisting of an oxygen atom, a sulfur atom, a carbon atom, and a nitrogen atom. This ring group is not particularly limited, and is preferably a ring having 5 to 8 atoms including a carbon atom to which R⁹ and R¹⁰ are attached. The ring may have a substituent. The substituent may be a substituent selected from a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of these substituents are the same groups as exemplified for R¹ to R⁴. R⁹ and R¹⁰ are preferably taken together to form the ring represented by Formula (X5).

### <Photochromic compound represented by Formula (5)>

A particularly suitable photochromic compound includes a compound represented by Formula (5) below:
In Formula (5), R¹, R², R³, R⁴, R⁷, R⁸, R⁹, R¹⁰, d, and e are each independently the same as in Formula (4).

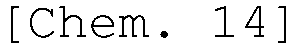

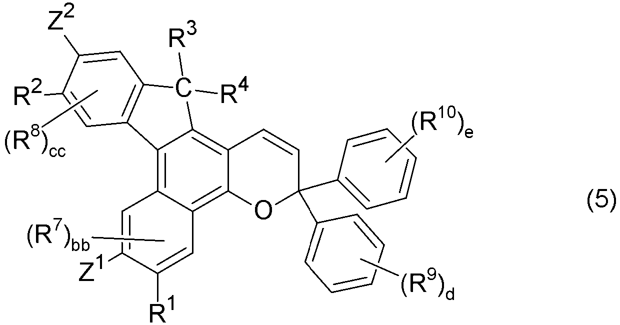

### <2¹ and Z²>

Z¹ and Z² are each independently a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a), a group represented by Formula (X), or a group represented by Formula (X3).

In Formula (5), bb denotes a number of R⁷ and is an integer of 0 or more and 2 or less. When bb is 2, R⁷s may be the same as or different from each other.
cc denotes a number of R⁸ and is an integer of 0 or more and 2 or less. When cc is 2, R⁸s may be the same as or different from each other.

Among photochromic compounds represented by Formula (5), a structure in which R² is a hydrogen atom, i.e., a structure in which a position 10 is unsubstituted is preferred and a structure in which R² is a hydrogen atom, bb is 0, and cc is 0, i.e., a structure represented by Formula (6) below is further preferred.

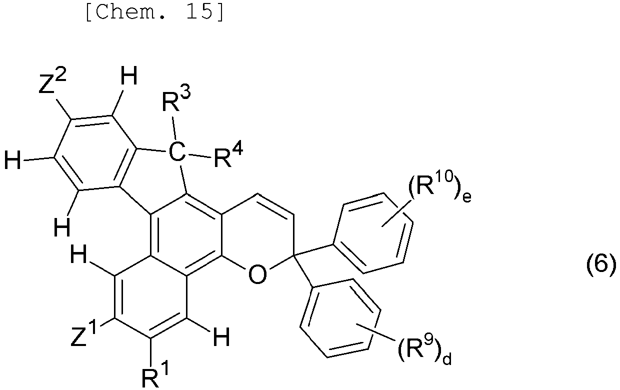

In Formula (6), R¹, R², R³, R⁴, R⁹, R¹⁰, Z¹, Z², d, and e are each independently the same as in Formula (5).

Z² is preferably a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a), a group represented by Formula (X), or a group represented by Formula (X3).

Z² is more preferably a hydrogen atom, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryl group, a substituted or unsubstituted heteroaryl group, a substituted or unsubstituted alkoxyalkylthio group, a haloalkylthio group, a substituted or unsubstituted silyl group, a group represented by Formula (2a), a group represented by Formula (X), or a group represented by Formula (X3) and further preferably a hydrogen atom, a substituted or unsubstituted alkyl group, a haloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted aryl group, a substituted or unsubstituted heteroaryl group, a haloalkylthio group, a group represented by Formula (2a), or a group represented by Formula (X3).

### <Detailed description of substituent>

Examples of the substituent that above-mentioned groups R¹ to R¹⁰, Z¹, and Z² may have include a hydroxyl group, a cyano group, a halogen atom, a nitro group, a formyl group, a hydroxycarbonyl group, a thiol group, a group represented by Formula (2a), a group represented by Formula (X), a group represented by Formula (X3), an alkyl group, a haloalkyl group, a cycloalkyl group, an alkoxy group, an amino group, a substituted amino group, a heterocyclic group, a halogen atom, an alkylthio group, an arylthio group, an alkylcarbonyl group, an alkoxycarbonyl group, an aralkyl, an aralkoxy group, an aryloxy group, an aryl group, a heteroaryl group, an alkoxyalkylthio group, a haloalkylthio group, a cycloalkylthio group, a silyl group, or an oxysilyl group. These groups may be the same as those detailed for R¹ to R⁴.

### <Specific example of suitable photochromic compound>

Specific examples of a particularly suitable photochromic compound include photochromic compounds represented by the following formulae:

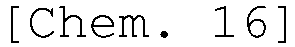

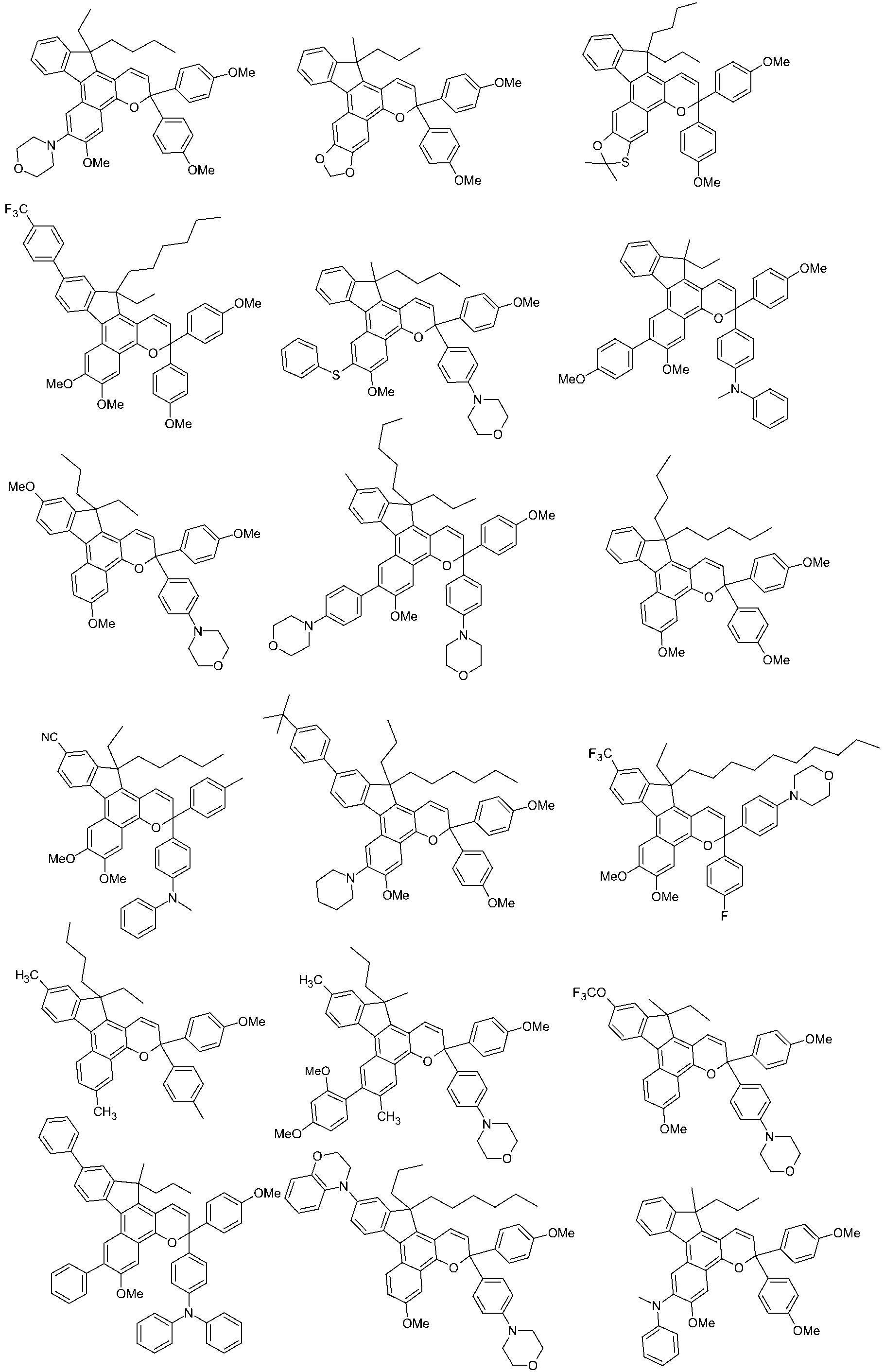

### [Method for producing photochromic compound]

A photochromic compound according to the embodiment may be produced by any synthetic method. Representative examples of a method for producing the photochromic compound will be described, but the method is not limited thereto. Note that, in the following description, a symbol in each formula has the same meaning as described for any of the above-mentioned formulae, unless otherwise noted.

The photochromic compound may be produced by a method in which a naphthol derivative represented by Formula (7) below and a propargyl alcohol compound represented by Formula (8) below are reacted in the presence of an acid catalyst.

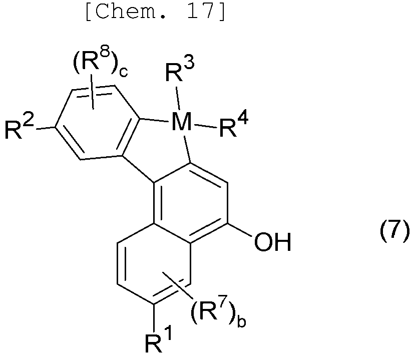

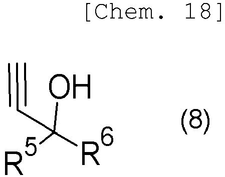

A reaction ratio of the naphthol derivative to the propargyl alcohol compound is preferably selected from a range of 1:10 to 10:1 (molar ratio). For example, sulfuric acid, benzene sulfonic acid, p-toluenesulfonic acid, acidic alumina, etc., are used as the acid catalyst. The acid catalyst is preferably used in a range of 0.1 to 10 parts by weight per 100 parts by weight of a total of the naphthol derivative and the propargyl alcohol compound. A reaction temperature is preferably 0 to 200°C. A solvent is preferably an aprotic organic solvent such as N-methylpyrrolidone, dimethylformamide, tetrahydrofuran, benzene, toluene, etc. A method for purifying a product obtained from such a reaction is not particularly limited. For example, the product can be purified by silica gel column purification and then recrystallization.

### <Method for synthesizing naphthol derivative>

A naphthol derivative can be synthesized using any of known methods.

A method for synthesizing the naphthol derivative represented by Formula (7) is not particularly limited. For example, when M is a carbon atom, the naphthol compound can be synthesized as follows. First, a benzene compound represented by Formula (9) below is reacted with an acid chloride compound represented by Formula (10) below to obtain a benzophenone compound represented by Formula (11) below. Note that, in Formula (11), R¹, R², R⁷, R⁸, b, and c are the same as in Formula (3).

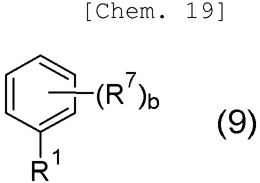

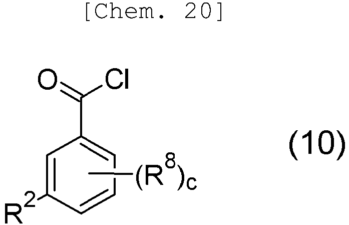

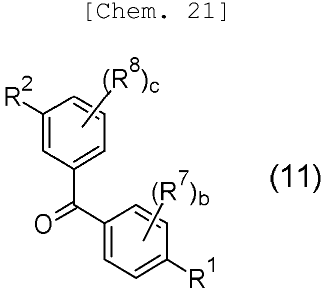

Note that, a substituent of the acid chloride compound and a substituent of the benzene compound may be exchanged depending on a structure and a substituent of a photochromic compound to be synthesized.

Furthermore, the benzophenone compound (11) is subjected to a Stobbe reaction, a cyclization reaction, a hydrolysis reaction using an alkali or an acid, benzyl protection, or debenzylation by a hydrolysis reaction using an alkali or an acid to obtain a carboxylic acid of which a hydroxyl group is protected with benzyl (Bn) as represented by Formula (12) below.

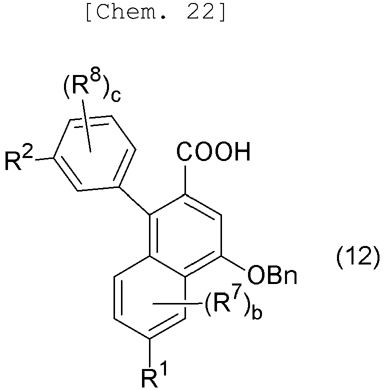

The thus-benzyl-protected carboxylic acid represented by Formula (12) is then converted to an amine by Curtius rearrangement, Hofmann rearrangement, Lossen rearrangement, etc., from which a diazonium salt is prepared. The diazonium salt is converted to a halide such as a bromide or an iodide by a Sandmeyer reaction, etc., to obtain a halide represented by Formula (13) below in which Hal represents a halogen.

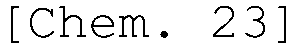

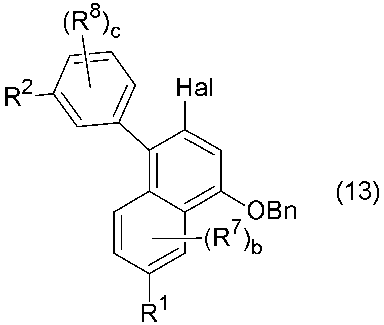

The thus-obtained halide is reacted with magnesium, lithium, etc., to prepare an organometallic reagent. This organometallic reagent is reacted with a ketone represented by Formula (14) below in which R³ and R⁴ are the same as in Formula (3) in an organic solvent at -100 to 70°C to obtain a compound represented by Formula (15) below.

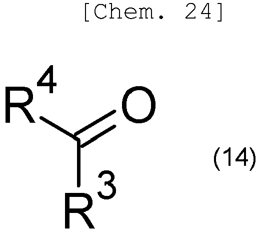

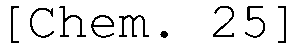

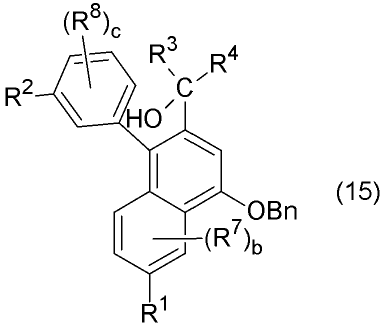

The thus-obtained compound represented by Formula (15) is debenzylated and then reacted under a neutral to acidic condition at 10 to 120°C for 10 minutes to 2 hours to thereby convert alcohol into its spiro form. Thus, the naphthol derivative represented by Formula (7) of interest can be synthesized. In such a reaction, a reaction ratio of the organometallic reagent to the ketone represented by Formula (14) may be selected from a wide range, but preferably from a range of 1:10 to 10:1 (molar ratio). A reaction temperature is preferably -100 to 70°C. An aprotic organic solvent such as diethyl ether, tetrahydrofuran, benzene, or toluene is preferably used as the solvent. The conversion of the alcohol into its spiro form under a neutral to acidic condition is preferably performed in the presence of an acid catalyst. For example, acetic acid, hydrochloric acid, sulfuric acid, benzene sulfonic acid, p-toluenesulfonic acid, acidic alumina, etc., are used as the acid catalyst. Such an acid catalyst is suitably used in a range of 0.1 to 10 parts by weight per 100 parts by weight of the alcohol. The alcohol is preferably converted into its spiro form in the presence of a solvent such as tetrahydrofuran, benzene, or toluene.

### <Method for synthesizing naphthol derivative including Si or Ge>

One exemplary method for producing a naphthol derivative represented by Formula (7) in which M is Si or Ge will be described.

First, the halide represented by Formula (13) is reacted with magnesium, lithium, etc., to prepare an organometallic reagent. This organometallic reagent is reacted with a monohalide represented by Formula (16) below in which R³ and R⁴ are the same as in Formula (7) in an organic solvent at -100 to 70°C to obtain a compound represented by Formula (17) below.

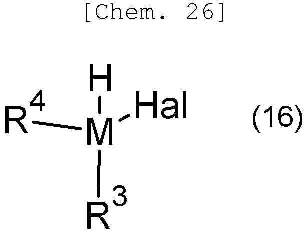

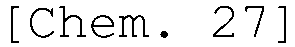

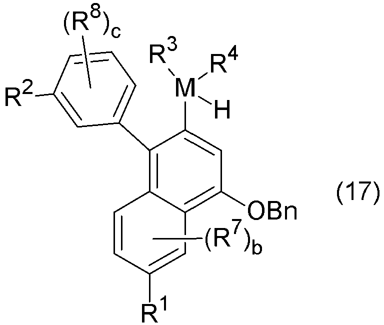

The resulting compound represented by Formula (17) is used to obtain a cyclized form represented by Formula (18) below by a known method.

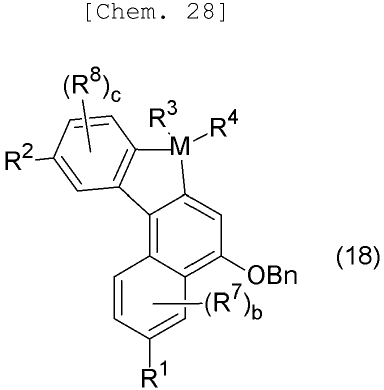

The thus-obtained cyclized form can be debenzylated to obtain the naphthol derivative represented by Formula (7).

### <Specific examples of naphthol derivative>

Specific examples of the naphthol derivative include compounds represented by the following formulae:

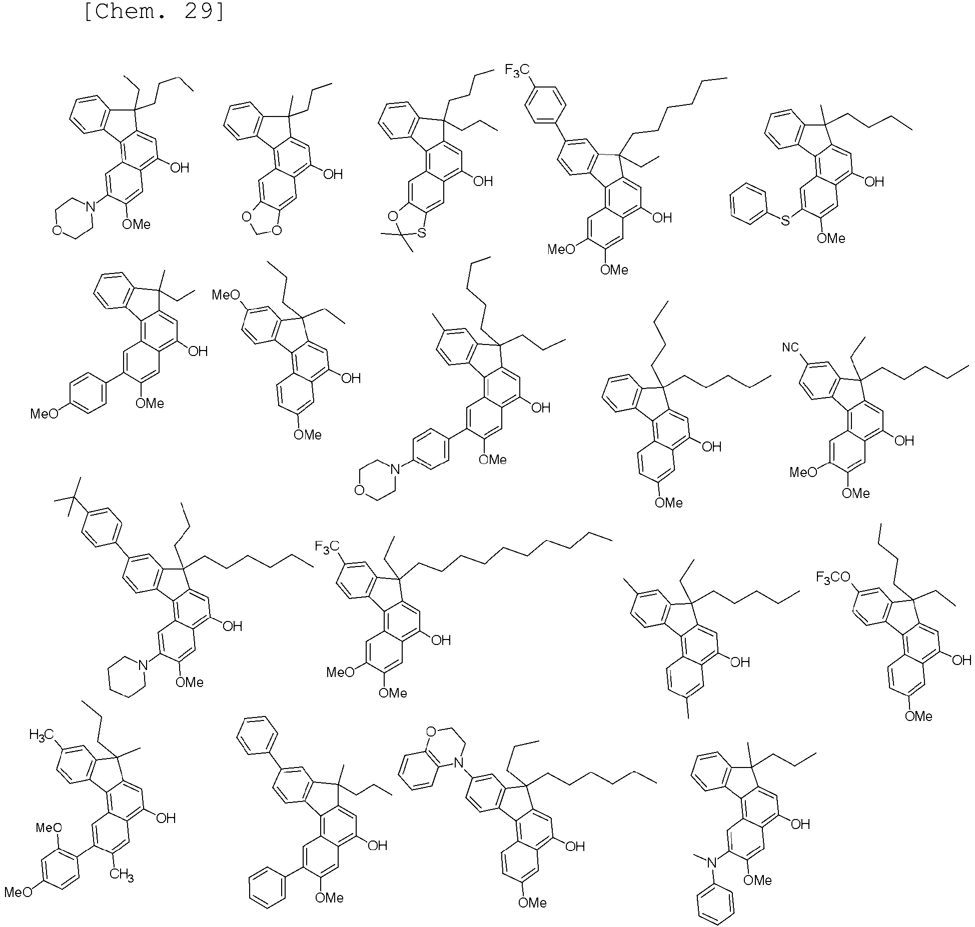

### <Identification of photochromic compound>

A photochromic compound according to the embodiment is, for example, in a solid or viscous liquid form at room temperature and atmospheric pressure. The photochromic compound may be isolated from such a solid or liquid by separation operations such as thin-layer chromatography, silica gel column chromatography, high-performance liquid chromatography, gas chromatography, etc. The absence of a byproduct such as a raw material compound or a coloring matter other than the photochromic compound is confirmed.

The thus-obtained photochromic compound is characterized by proton nuclear magnetic resonance spectroscopy (¹H-NMR) and a peak based on an aromatic proton and an alkene proton appears around δ: 5.0 to 9.0 ppm and a peak based on protons of an alkyl group and an alkylene group appears around δ: 1.0 to 4.0 ppm. By relatively comparing spectral intensities of the peaks, a number of protons on each attached group can be determined. This allows identification of a backbone and a substituent that the photochromic compound has.

When the photochromic compound is included in a cured product such as a resin, the photochromic compound can be isolated by dissolving the resin and using the above-mentioned separation operations.

### <Photochromic composition>

A photochromic compound according to the embodiment can be dissolved in a common organic solvent such as toluene, chloroform, or tetrahydrofuran. When a photochromic compound having a backbone represented by Formula (3) is dissolved in such a solvent, a clear, colorless solution is obtained. This solution exhibits good photochromic behavior, that is, when the solution is irradiated with sunlight or ultraviolet light, it quickly develops a color and when the sunlight, etc., is blocked, it quickly reversibly returns to its original colorless state.

The photochromic compound according to the embodiment can be used in combination with a photochromic compound having another structure, depending on the intended use. For example, it can be used in combination with another photochromic compound to obtain various color tones required for a photochromic lens. A photochromic compound to be combined may be any known compound without any limitation. Examples thereof include indenonaphthopyran, naphthopyran, spirooxazine, spiropyran, fulgide, fulgimide, diarylethene, etc. Among these, an indenonaphthopyran compound is particularly preferred since it can maintain a uniform color tone upon color development and fading, can suppress a color shift upon color development associated with degradation of a photochromic property, and can reduce initial coloration. It is preferred to use a plurality of types of photochromic compounds to adjust a color tone since they can achieve both a color density under a high temperature and a fast color fading rate and also give excellent durability.

When a photochromic composition including the photochromic compound according to the embodiment and another photochromic compound is produced, a blending ratio of the photochromic compounds is appropriately determined in accordance with a desired color tone.

### <Photochromic curable composition>

A curable composition according to the embodiment includes a photochromic compound according to the embodiment; and at least one selected from the group consisting of a radical polymerizable monomer, a cationic polymerizable monomer, a compound having a polymerization reactive group, and a (thio)urethane (urea) polymer. Here, the (thio) urethane (urea) polymer includes at least one selected from the group consisting of a urethane polymer, a thiourethane polymer, a urethane urea polymer, and a thiourethaneurea polymer.

The photochromic compound according to the embodiment and the photochromic composition are preferably used in combination with a polymerizable compound as a photochromic curable composition.

For the photochromic curable composition, although it all depends on a color developing intensity of a photochromic compound, a lens material selected, and a thickness of a lens, the photochromic compound (or photochromic composition) is preferably used in an amount of 0.001 to 10 parts by mass relative to 100 parts by mass of the polymerizable compound. An optimal amount to be incorporated depends on application for which the photochromic curable composition is used. For example, the case where the photochromic curable composition is used as a thin-film optical article or as a thick-film optical article will be described below.

### (Use as thin-film optical article)

For example, when a thin film of 10 µm or more and less than 1000 µm, e.g., about 100 µm (polymer film made by polymerization of the photochromic curable composition) is formed from the photochromic curable composition, 0.001 to 10 parts by mass of a photochromic compound (or photochromic composition) according to the embodiment relative to 100 parts by mass of another polymerizable monomer may be incorporated to adjust a color tone.

### (Use as thick-film optical article)

In the case of a thick cured product (polymeric molded product made by polymerization of the photochromic curable composition), e.g., a cured product having a thickness of 1 mm or more, 0.001 to 1 part by mass of a photochromic compound (or photochromic composition) according to the embodiment relative to 100 parts by mass of the thick cured product or another polymerizable monomer that gives the thick cured product may be incorporated to adjust a color tone.

### <Polymerizable compound>

As mentioned above, a photochromic compound is preferably used as a photochromic curable composition in combination with a polymerizable compound. Examples of the polymerizable compound may include a urethane- or urea-based polymerizable compound that can form a urethane bond or a urea bond, a compound having a polymerization reactive group, a radical polymerizable compound, an epoxy-based polymerizable compound, etc. These polymerizable compounds are not particularly limited, and, for example, the polymerizable compound described in Patent Document 10 may be suitably used.

Among these, the below-mentioned polymerizable compounds are particularly suitably used.

### <Compound having polymerization reactive group>

An example of a compound having a polymerization reactive group includes a compound having an iso(thio)cyanate group (iso(thio)cyanate compound). An iso(thio)cyanate compound is a compound having an isocyanate group or an isothiocyanate group and may include both an isocyanate group and an isothiocyanate group. This compound is suitable for use in combination with an active hydrogen-containing compound as described below. Examples of such an iso(thio)cyanate compound include, but are not limited to, the below-mentioned compounds.

### (Polyiso(thio)cyanate)

Polyiso(thio)cyanate is a compound having at least two or more iso(thio)cyanate groups per molecule. Examples of the polyiso(thio)cyanate include an aromatic polyiso(thio)cyanate having an aromatic ring such as m-xylene diisocyanate or 4,4'-diphenylmethane diisocyanate; or an aliphatic polyiso(thio)cyanate such as norbornane diisocyanate or dicyclohexylmethane-4,4'-diisocyanate.

### (Active hydrogen-containing compound)

An active hydrogen-containing compound is preferably a compound having a hydroxyl group and/or a thiol group and particularly preferably a polyfunctional compound having two or more active hydrogens per molecule, but is not limited thereto. Specific examples of the active hydrogen-containing compound may include a polyfunctional thiol compound such as pentaerythritol tetrakis(3-mercaptopropionate) or 4-mercaptomethyl-3,6-dithia-octanedithiol; or a polyfunctional alcohol such as trimethylolpropane or pentaerythritol.

### (Radical polymerizable compound)

A radical polymerizable compound includes a polyfunctional radical polymerizable compound and a monofunctional radical polymerizable compound. Each of these can be used alone or a plurality of them can be used in combination. Examples of a radical polymerizable substituent include a group having an unsaturated double bond, i.e., a vinyl group including a styryl group, a (meth)acryl group, or an allyl group, etc.

The polyfunctional radical polymerizable compound is a compound having two or more radical polymerizable substituents per molecule. This polyfunctional radical polymerizable compound includes a first polyfunctional radical polymerizable compound having 2 to 10 radical polymerizable substituents and a second polyfunctional radical polymerizable compound having more than 10 radical polymerizable substituents.

The first polyfunctional radical polymerizable compound is not particularly limited, but more preferably has 2 to 6 radical polymerizable substituents. Specific examples thereof are as follows.

### (Polyfunctional (meth)acrylic acid ester compound)

Ethylene glycol di(meth)acrylate, diethylene glycol di(meth)acrylate, triethylene glycol di(meth)acrylate, tetraethylene glycol di(meth)acrylate, ethylene glycol bisglycidyl(meth)acrylate, bisphenol A di(meth)acrylate, 2,2-bis(4-(meth)acryloyloxyethoxyphenyl)propane, 2,2-bis(3,5-dibromo-4-(meth)acryloyl, oxyethoxyphenyl)propane.

### (Polyfunctional allyl compound)

Diallyl phthalate, diallyl terephthalate, diallyl isophthalate, diallyl tartrate, diallyl epoxysuccinate, diallyl fumarate, diallyl chlorendate, diallyl hexaphthalate, diallyl carbonate, allyl diglycol carbonate, trimethylolpropane triallyl carbonate.

(Polyfunctional thio(meth)acrylic acid ester compound) 1,2-bis(methacryloylthio)ethane, bis(2-acryloylthioethyl)ether, 1,4-bis(methacryloylthiomethyl)benzene.

### (Radical polymerizable compound)

Examples of the second polyfunctional radical polymerizable compound having more than 10 radical polymerizable substituents include a compound having a relatively large molecular weight such as a silsesquioxane compound having a radical polymerizable substituent or a polyrotaxane compound having a radical polymerizable substituent.

The monofunctional radical polymerizable compound is a compound having one radical polymerizable substituent in a molecule, and specific examples thereof include, but are not limited to, the below-mentioned compounds.

### (Unsaturated carboxylic acid)

Acrylic acid, methacrylic acid, maleic anhydride.

### ((Meth)acrylic acid ester)

Methyl (meth)acrylate, benzyl methacrylate, phenyl methacrylate.

### (Monofunctional (meth)acrylic acid ester compound)

2-Hydroxyethyl methacrylate, glycidyl (meth)acrylate, β-methyl glycidyl (meth)acrylate, bisphenol A-monoglycidyl ether methacrylate, 4-glycidyloxy methacrylate, 3-(glycidyl-2-oxyethoxy)-2-hydroxypropyl methacrylate, 3-(glycidyloxy-1-isopropyloxy)-2-hydroxypropyl acrylate, 3-glycidyloxy-2-hydroxypropyloxy)-2-hydroxypropyl acrylate.

### (Fumaric acid ester)

Diethyl fumarate, diphenyl fumarate.

### (Thio(meth)acrylic acid)

Methyl thioacrylate, benzyl thioacrylate, benzyl thiomethacrylate.

### (Vinyl Compound)

Divinylbenzene, styrene, chlorostyrene, tylstyrene, vinylnaphthalene, α-methylstyrene dimer, bromostyrene.

The radical polymerizable compound may be used alone or a mixture of a plurality of types of them may also be used. In this case, preferably 80 to 100 parts by mass of the polyfunctional radical polymerizable compound and 0 to 20 parts by mass of the monofunctional radical polymerizable compound, and more preferably 90 to 100 parts by mass of the polyfunctional radical polymerizable compound and 0 to 10 parts by mass of the monofunctional radical polymerizable compound are used per 100 parts by mass of a total of the radical polymerizable compounds. Furthermore, preferably 80 to 100 parts by mass of the first polyfunctional radical polymerizable compound, 0 to 20 parts by mass of the second polyfunctional radical polymerizable compound, and 0 to 20 parts by mass of the monofunctional radical polymerizable compound, and further preferably 85 to 100 parts by mass of the first polyfunctional radical polymerizable compound, 0 to 10 parts by mass of the second polyfunctional radical polymerizable compound, and 0 to 10 parts by mass of the monofunctional radical polymerizable compound are used per 100 parts by mass of a total of the radical polymerizable compounds.

### (Various compounding agents)

Various known compounding agents may be incorporated into a curable composition as long as the effect is not impaired. Examples of the compounding agent include, for example, various other stabilizers such as a releasing agent, a UV absorber, an IR absorber, a UV stabilizer, an antioxidant, an anti-coloring agent, an antistatic agent, a fluorescent dye, a dye, a pigment, a perfume, etc. A solvent or a leveling agent can also be incorporated. A thiol such as t-dodecyl mercaptan can be incorporated as a polymerization regulator.

Among the above-mentioned compounding agents, the UV stabilizer is suitably used from the viewpoint of improving durability of a photochromic moiety. A hindered amine light stabilizer, a hindered phenol antioxidant, a sulfur antioxidant, etc., are known as such a UV stabilizer. A particularly suitable UV stabilizer is as follows:
Bis(1,2,2,6,6-pentamethyl-4-piperidyl)sebacate; ADK STAB LA-52, LA-57, LA-62, LA-63, LA-67, LA-77, LA-82, LA-87 manufactured by ADEKA CORPORATION; 2,6-di-tert-butyl-4-methylphenol, ethylene bis(oxyethylene) bis[3-(5-tert-butyl-4-hydroxy-m-tolyl)propionate]; IRGANOX 1010, 1035, 1075, 1098, 1135, 1141, 1222, 1330, 1425, 1520, 259, 3114, 3790, 5057, 565 manufactured by BASF Japan. An amount of such a UV stabilizer to be used is not particularly limited unless the effect is impaired, but is usually in a range of 0.001 to 10 parts by mass, especially 0.01 to 1 part by mass per 100 parts by mass of a photochromic curable composition.

A UV absorber may also be used in addition to the UV stabilizer. Known UV absorbers such as a benzophenone compound, a benzotriazole compound, a cyanoacrylate compound, a triazine compound, or a benzoate compound can be used as the UV absorber, with a cyanoacrylate compound or a benzophenone compound being particularly preferred. The above-mentioned UV stabilizer is preferably used in a range of 0.001 to 5 parts by mass relative to 100 parts by mass of a photochromic curable composition including a photochromic compound and a polymerizable compound.

### <Method for using photochromic curable composition; optical article>

A photochromic curable composition is cured to obtain a photochromic cured product. Polymerization curing for producing the photochromic cured product is performed by radical polymerization, ring-opening polymerization, anionic polymerization, or condensation polymerization using irradiation with an active energy ray such as an ultraviolet ray, an α-ray, a β-ray, or a γ-ray; heat; or a combination thereof. In other words, an appropriate polymerization method may be employed depending on a type of a polymerizable compound or a polymerization accelerator and a form of a photochromic cured product to be formed.

When a curable composition containing a polymerizable compound is thermally polymerized, a temperature affects a property of the resulting photochromic cured product.

Such a temperature condition cannot be generally limited since it is affected by a type or amount of a thermal polymerization initiator or a type of a polymerizable compound. However, in general, a method in which polymerization is started at a relatively low temperature, followed by heating slowly is suitable. Polymerization time also varies depending on various factors, as is the case with the temperature. Therefore, although it is suitable to determine an optimum time in advance in accordance with these conditions, in general, the conditions are preferably selected so that polymerization is completed in 2 to 48 hours. When obtaining a photochromic laminated sheet, polymerization is performed at a temperature at which a reaction between polymerizable functional groups proceeds, and optimal temperature and time are preferably determined to achieve a desired molecular weight.

When a curable composition is photo-polymerized, among polymerization conditions, especially UV intensity affects a property of the resulting photochromic cured product. Such an irradiation condition is affected by a type or amount of a photoinitiator or a type of a polymerizable monomer, so it cannot be generally limited, but in general, the conditions are preferably selected so as to give photoirradiation with 50 to 500 mW/cm² of UV light at a wavelength of 365 nm for 0.5 to 5 minutes.

### [Optical article]

A photochromic compound according to the embodiment can be widely used as a photochromic material, and can be used, for example, as various memory materials as an alternative to a silver halide photosensitive material, a reprographic material, a photosensitive material for printing, a memory material for a cathode ray tube, a photosensitive material for a laser, a photosensitive material for holography, and various other memory materials. The photochromic material can also be used as a material for a photochromic lens material, an optical filter material, a display material, a light meter, or a decoration material.

The photochromic compound according to the embodiment is particularly suitable for a photochromic lens application. A photochromic lens is suitable as a lens for eyeglasses such as sunglasses. Any known method for producing a photochromic lens can be employed, as long as the method provides uniform photochromic performance.

In the case where a photochromic property is developed by a kneading method, a photochromic cured product in a form of an optical material such as a lens may be obtained by injecting the above-mentioned curable composition between glass molds held with an elastomeric gasket or a spacer and then heating the curable composition in an air furnace or cast-polymerizing the curable composition by irradiation with an active energy ray such as ultraviolet ray depending on a type of a polymerizable compound or a polymerization accelerator.

In the case where a photochromic property is developed by a lamination method, a photochromic layer made of a photochromic cured product is formed on a surface of an optical substrate by dissolving a curable composition in an organic solvent as appropriate to prepare a coating solution; coating a surface of an optical substrate such as a lens substrate with the coating solution by spin coating, dipping, etc.; drying to remove the organic solvent; and then polymerization-curing the curable composition with UV irradiation, heating, etc. in an inert gas such as nitrogen (coating method).

Furthermore, a photochromic layer made of a photochromic cured product can also be formed on a surface of an optical substrate by casting polymerization using an inner mold in which an optical substrate such as a lens substrate is placed face-to-face in a glass mold so that a predetermined void is formed, a curable composition is injected into this void, and the curable composition is polymerization-cured in this state by UV irradiation, heating, etc. (casting polymerization method).

In the case where a photochromic layer is formed on a surface of an optical substrate by the lamination method (coating method and casting polymerization method) as described above, the surface of the optical substrate may be chemically treated with an alkali solution, an acid solution, etc., or physically treated with corona discharge, plasma discharge, polishing, etc. in advance in order to enhance adhesion between the photochromic layer and the optical substrate. Of course, it is also possible to provide a transparent adhesive resin layer on the surface of the optical substrate.

In addition, in the case where a photochromic property is developed by a binder method, a photochromic sheet is made by sheet-molding using a curable composition, sandwiched between two transparent sheets (optical sheets), and polymerization-cured as described above to obtain a photochromic laminate with a photochromic layer as an adhesive layer.

In this case, coating with a coating solution in which a curable composition is dissolved in an organic solvent can also be employed to produce the photochromic sheet.

The thus-produced photochromic laminate is, for example, mounted in a mold and then a thermoplastic resin (e.g., polycarbonate) for an optical substrate such as a lens is injection-molded thereon to obtain an optical substrate such as a lens with a photochromic property in a predetermined shape.

The photochromic laminate can also be adhered to a surface of an optical substrate with, for example, an adhesive to obtain a photochromic lens.

Note that, when a photochromic laminate is produced as described above, it is preferred to use, as a polymerizable compound, a urethane- or urea-based polymerizable compound, especially a urethane-based polymerizable compound to adjust so as to form polyurethane from the viewpoint of high adhesion especially to an optical substrate.

The above-mentioned curable composition can develop a photochromic property with an excellent color developing density at a high temperature.

A photochromic layer or a photochromic cured product formed from a curable composition can be subjected to post-processing such as staining using a dye such as a disperse dye; formation of a hard coat film using a silane coupling agent or a hard coat agent containing as a main component a sol of silicon, zirconium, antimony, aluminum, tin, tungsten, etc.; formation of a thin film by vapor deposition of a metal oxide such as SiO₂, TiO₂, ZrO₂, etc.; antireflection treatment by a thin film coated with an organic polymer; or antistatic treatment depending on its application.

### EXAMPLES

The present invention will be described in more detail with reference to Examples exemplified below. These Examples are for illustrative purposes only and the spirit and the scope of the present invention are not limited to Examples.

### Example 1

### First step

First, 200 mL of toluene was added to 9.3 g (20.0 mmol) of a halogen compound represented by Formula (1-1) below, which was synthesized from 4,4'-dimethylbenzophenone with reference to the method described in Patent Document 11, and heated to distill off 50 mL of the toluene under atmospheric pressure. After the toluene was distilled off, the resultant was cooled until an internal temperature was -20°C and 15 mL of n-BuLi (1.6 M hexane solution) was added dropwise thereto while maintaining -15°C or less. After consumption of the raw materials was confirmed, 2.6 g (30.2 mmol) of methyl propyl ketone was added dropwise thereto while maintaining -15°C or less. After stirring for 1 hour, the resultant was slowly heated to room temperature. Then, the resultant was partitioned by adding 150 mL of water thereto. Washing with water was repeated until a pH of an aqueous layer was 7, and the resulting organic layer was concentrated, followed by purification by chromatography on silica gel to obtain a compound represented by Formula (1-2) below at a yield of 87%.

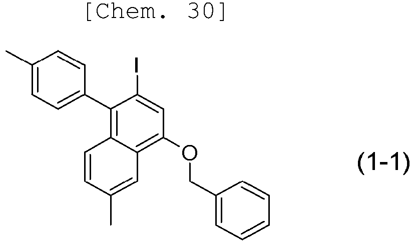

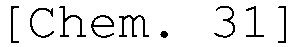

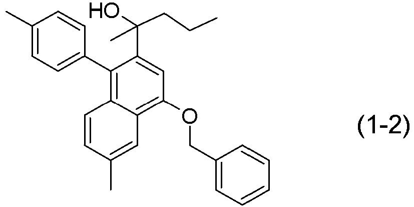

### Second step

Then, 7.12 g (17.4 mmol) of a compound represented by Formula (1-2) was dissolved in 75 mL of tetrahydrofuran, 1.4 g of 5% Pd/C (50% water content) was added thereto, followed by reacting under pressure with hydrogen gas at 0.05 to 0.1 Mpa. After consumption of the raw materials was confirmed, the Pd/C was filtered off and a solvent was removed from the resulting organic layer to obtain a compound represented by Formula (1-3) below at a yield of 100%.

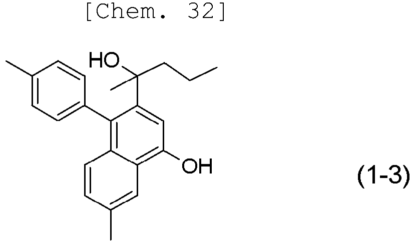

### Third step

One hundred milliliters of toluene was added to 5.0 g (26.1 mmol) of p-toluenesulfonic acid monohydrate, and azeotropic dehydration was performed until a water content in the toluene was 92 ppm. After the azeotropic dehydration, 5.8 g of the compound represented by Formula (1-3) was added thereto and heated at 100°C. After consumption of the raw materials, the resultant was cooled to room temperature and partitioned by adding 48 g of a 5% sodium hydrogen carbonate aqueous solution thereto and stirring. The resultant was partitioned by adding 100 mL of water thereto. Washing with water was repeated until a pH of an aqueous layer was 7, and the resulting organic layer was concentrated, followed by purification by chromatography on silica gel to obtain a compound represented by Formula (1-4) below at a yield of 82%.

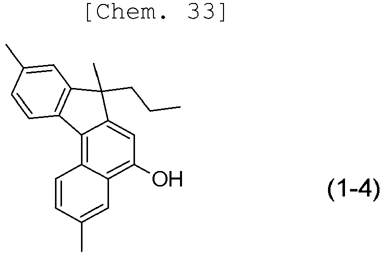

### Fourth step

First, 3.2 g (10.0 mmol) of a compound represented by Formula (1-4) and 3.0 g (12.0 mmol) of propargyl alcohol represented by Formula (1-5) below were dissolved in 80 mL of toluene, and 0.25 g (1.0 mmol) of pyridinium p-toluenesulfonate was added thereto and stirred at 85°C for 1 hour. After the compound represented by Formula (1-4) was consumed, the resultant was cooled to room temperature and partitioned by adding 80 mL of water thereto. A solvent was removed from the resulting organic layer, followed by purification by chromatography on silica gel and recrystallization with a mixed solvent of ethanol/acetonitrile to obtain a photochromic compound represented by Formula (1-6) below at a yield of 67%.

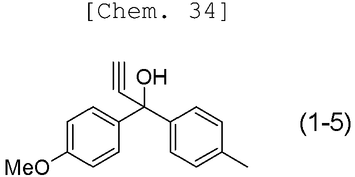

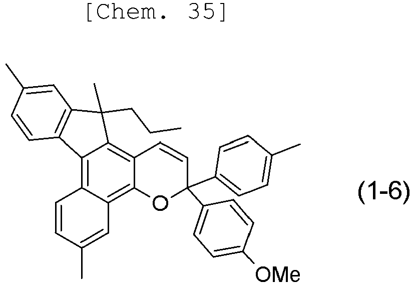

Elemental analysis values for the photochromic compound represented by Formula (1-5) were C: 87.24%, H: 6.95%, which are in good agreement with the calculated values for C₄₀H₃₈O₂ of C: 87.23%, H: 6.95%.

Proton nuclear magnetic resonance spectra were measured and showed a 19H peak based on a propyl group, a methyl group around δ: 0.0 to 3.5 ppm, a 3H peak based on a methoxy group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 2)

A photochromic compound represented by Formula (2-2) below was obtained at a yield of 71% in the same manner as Example 1, except that 3-methyl-4-methoxybenzophenone was used instead of 4,4'-dimethylbenzophenone and 3-heptanone was used instead of methyl propyl ketone in the first step and a compound represented by Formula (1-2) below was used instead of the compound represented by Formula (1-5) in fifth step.

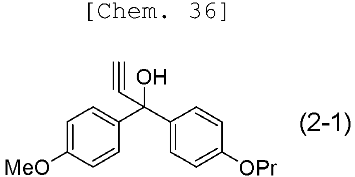

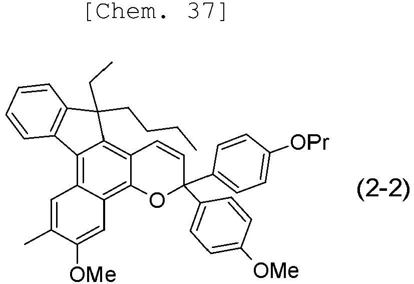

Elemental analysis values for the photochromic compound represented by Formula (2-2) were C: 82.70%, H: 7.25%, which are in good agreement with the calculated values for C₄₄H₄₆O₄ of C: 82.72%, H: 7.26%.

Proton nuclear magnetic resonance spectra were measured and showed a 22H peak based on a methyl group, an ethyl group, a butyl group, a propoxy group around δ: 0.0 to 3.5 ppm, a 8H peak based on a methoxy group, a propoxy group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 3)

A photochromic compound represented by Formula (3-2) below was obtained at a yield of 43% in the same manner Example 1, except that 4,4'-dimethoxybenzophenone was used instead of 4,4'-dimethylbenzophenone and 4-methyl-3-hexanone was used instead of methyl propyl ketone in the first step and a compound represented by Formula (3-1) below was used instead of the compound represented by Formula (1-5) in the fifth step.

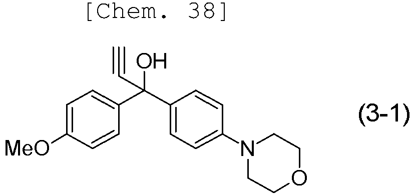

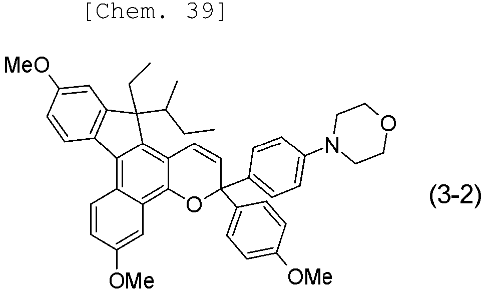

Elemental analysis values for the photochromic compound represented by Formula (3-2) were C: 79.28%, H: 6.95%, N: 2.05%, which are in good agreement with the calculated values for C₄₅H₄₇NO₃ of C: 79.27%, H: 6.95%, N: 2.05%.

Proton nuclear magnetic resonance spectra were measured and showed a 18H peak based on an ethyl group, a 2-butyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 4)

### First step

First, 9.3 g (20.0 mmol) of a carboxylic acid represented by Formula (4-1) below, which was synthesized from 3-bromo-4-methoxybenzophenone with reference to the method described in Patent Document 11, was synthesized and reacted with 2,6-dimethylbenzenethiol with reference to the method described in Patent Document 8 to obtain a carboxylic acid compound represented by Formula (4-2) below at a yield of 92%.

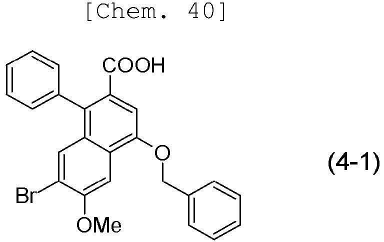

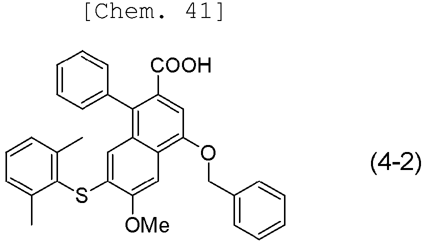

### Second step

A naphthol compound represented by Formula (4-4) below was synthesized in the same manner as Example 1, except that a iodine compound represented by Formula (4-3) below, which was synthesized from the carboxylic acid compound represented by Formula (4-2) with reference to the method described in Patent Document 11, was used instead of the compound represented by Formula (1-1) and ethyl propyl ketone was used instead of methyl propyl ketone in the first step.

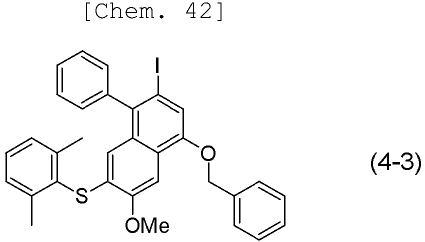

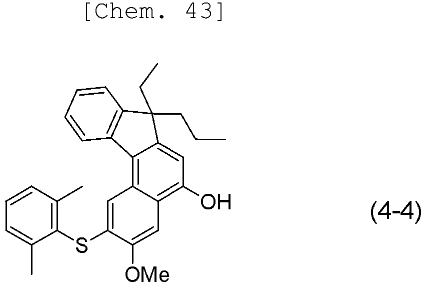

### Third step

A photochromic compound represented by Formula (4-5) below was obtained at a yield of 60% in the same manner as Example 1, except that a compound represented by Formula (4-4) was used instead of the compound represented by Formula (1-4) and the compound represented by Formula (3-1) was used instead of the compound represented by Formula (1-5) in the fourth step.

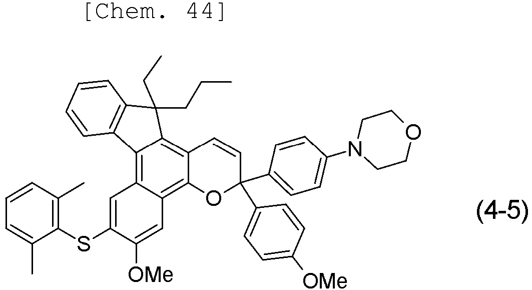

Elemental analysis values for the photochromic compound represented by Formula (4-5) were C: 79.14%, H: 6.65%, N: 1.80%, S: 4.14%, which are in good agreement with the calculated values for C₃₁H₃₁NO₄S of C: 79.14%, H: 6.64%, N: 1.81%, S: 4.14%.

Proton nuclear magnetic resonance spectra were measured and showed a 22H peak based on a methyl group, an ethyl group, a propyl group, a morpholino group around δ: 0.0 to 3.5 ppm, a 10H peak based on a methoxy group, a morpholino group around δ: 3.5 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 5)

### First step

A carboxylic acid compound represented by Formula (5-1) below was obtained in the same manner as Example 4, except that 4-bromo-3',4'-dimethoxybenzophenone was used instead of 4,4'-dimethylbenzophenone in the first step.

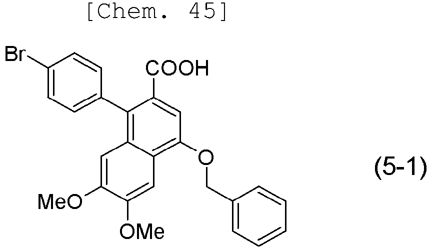

### Second step

First, 7.4 g (54.3 mmol) of 4-methylphenylboronic acid, 11.5 g (108.7 mmol) of sodium carbonate, 103.5 mL of water, 121.5 mL of 1,2-dimethoxyethane, and 12.2 mL of ethanol were added to 24.6 g (49.9 mmol) of the carboxylic acid compound represented by Formula (5-1) and the resultant was stirred while bubbling with nitrogen. The nitrogen bubbling was continued for about 20 minutes, and then 142.7 mg (0.1 mmol) of Pd(PPh₃)₄ was added thereto and the resultant was reacted at 75°C for 2 hours. After the above reaction, the resultant was cooled to room temperature, 650 mL of THF was added thereto, and the resultant was cooled to 0 to 5°C and partitioned by adding concentrated hydrochloric acid thereto to pH 1. A solvent was removed by washing with 500 mL of water twice. The resultant was purified by reslurrying with 300 mL of methanol to obtain a carboxylic acid represented by Formula (5-2) below at a yield of 93%.

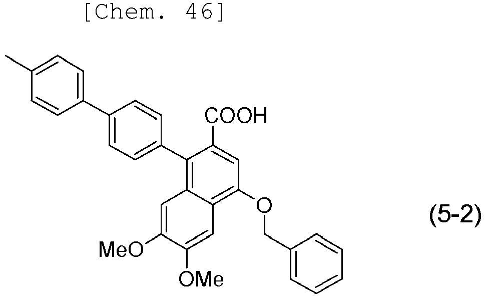

### Third step

An iodine compound represented by Formula (5-3) below was obtained from the compound represented by Formula (5-2) below at a yield of 76% with reference to the method described in Patent Document 11.

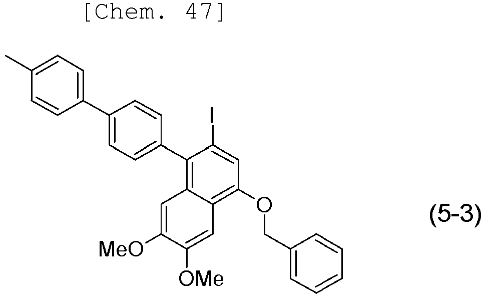

### Fourth step

A naphthol compound represented by Formula (5-4) below was synthesized in the same manner as Example 1, except that Formula (5-3) was used instead of the compound represented by Formula (1-1) and methyl butyl ketone was used instead of methyl propyl ketone in the first step.

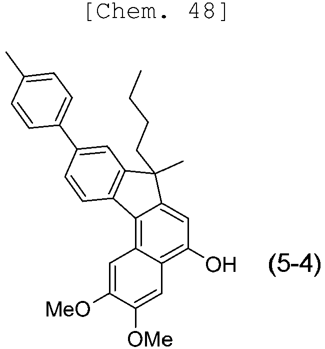

### Fifth step

A photochromic compound represented by Formula (5-6) below was obtained at a yield of 63% in the same manner as Example 1, except that the compound represented by Formula (5-4) was used instead of the compound represented by Formula (1-4) and a compound represented by Formula (5-5) was used instead of the compound represented by Formula (1-5) in the fourth step.

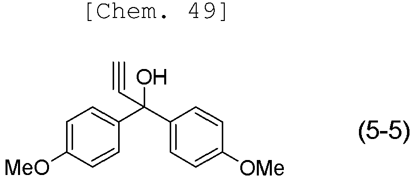

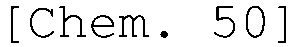

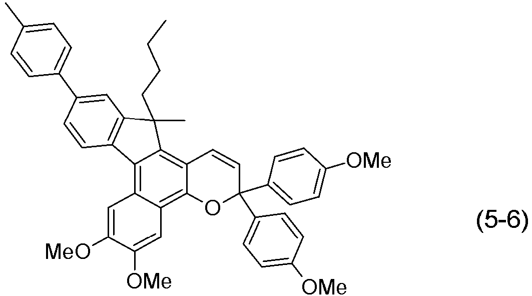

Elemental analysis values for the photochromic compound represented by Formula (5-6) were C: 82.04%, H: 6.59%, which are in good agreement with the calculated values for C₄₈H₄₆O₅ of C: 82.02%, H: 6.60%.

Proton nuclear magnetic resonance spectra were measured and showed a 15H peak based on a methyl group, a butyl group around δ: 0.0 to 3.5 ppm, a 12H peak based on a methoxy group around δ: 3.5 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 6)

### First step

First, 6.9 g (15.0 mmol) of the carboxylic acid represented by Formula (4-1), 1.6 g (18.0 mmol) of morpholine, 140 mL of toluene, and 5.0 g (45.0 mmol) of tertiary-butoxycalcium were added and stirred under reduced pressure to remove dissolved oxygen. Then, 13.7 mg (0.015 mmol) of Pd₂(dba)₃ and 28.6 mg (0.06 mmol) of X-phos were added to the resulting reaction liquid and heated to 85°C. Heating was continued until the raw materials were exhausted. After the reaction was completed, the resultant was cooled to room temperature and filtered. Then, 150 mL of tetrahydrofuran and 10% hydrochloric acid were added to the resulting filtrate for neutralization and then the resultant was partitioned. The resulting organic layer was concentrated and then purified by reslurrying with 70 mL of 2-propanol to obtain a carboxylic acid represented by Formula (6-1) below at a yield of 92%.

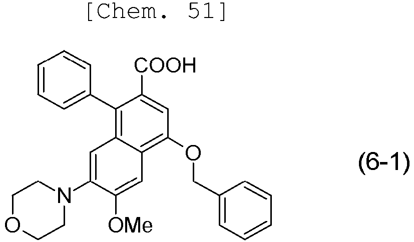

### Second step

A photochromic compound represented by Formula (6-2) below was obtained at a yield of 61% in the same manner as Example 5, except that a compound represented by Formula (6-1) was used instead of the compound represented by Formula (5-2) in the third step and 4-octanone was used instead of methyl butyl ketone in the fourth step.

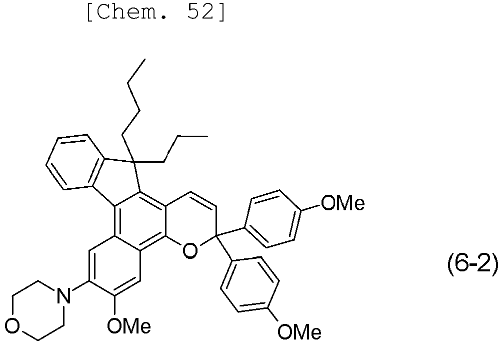

Elemental analysis values for the photochromic compound represented by Formula (6-2) were C: 79.40%, H: 7.10%, N: 2.00%, which are in good agreement with the calculated values for C₄₆H₄₉NO₅ of C: 79.39%, H: 7.10%, N: 2.01%.

Proton nuclear magnetic resonance spectra were measured and showed a 20H peak based on a propyl group, a butyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 7)

### First step

First, 2.75 g (10.0 mmol) of 4-bromo-4'-methylbenzophenone, 1.50 g (14.0 mmol) of N-methylaniline, and 0.29 g (30.0 mmol) of sodium tert-butoxide were added to 50 mL of toluene and the resultant was subject to nitrogen bubbling for 20 minutes. Then, 46 mg (0.05 mmol) of tris(dibenzylideneacetone)dipalladium(0) and 95 mg (0.20 mmol) of 2-dicyclohexylphosphino-triisopropylbiphenyl were added thereto and the resultant was reacted at 80°C for 3 hours. After the reaction was completed, the resultant was cooled to room temperature and 30 mL of THF was added thereto, followed by filtering. The resultant was cooled at 0 to 5°C, and 10% hydrochloric acid was added thereto to a pH of 6 to 7. The resultant was partitioned and washed with 100 mL of water three times. A solvent was removed from the resulting organic layer and the resultant was purified by chromatography on silica gel to obtain benzophenone represented by Formula (7-1) below at a yield of 92%.

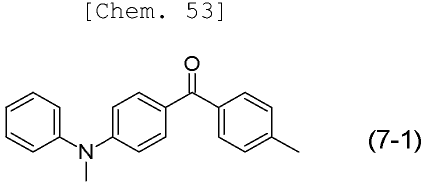

### Second step

The reaction was performed with reference to the method described in Patent Document 12 to obtain propargyl alcohol represented by Formula (7-2) below at a yield of 86%.

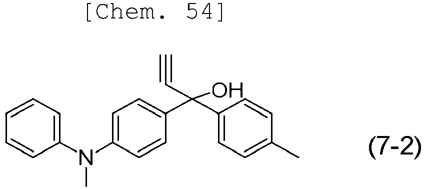

### Third step

A naphthol derivative represented by Formula (7-3) below was synthesized in the same manner as Example 1, except that 4-methoxybenzophenone was used instead of 4,4'-dimethylbenzophenone and 2-octanone was used instead of methyl propyl ketone in the first step. Then, 2.5 g (6.7 mmol) of the resulting naphthol compound, 5.0 g of WakoGel C300, and 80 mL of toluene were added thereto and heated to 100°C. Next, 2.85 g (8.7 mmol) of the propargyl alcohol represented by Formula (7-2) dissolved in 20 mL of toluene was added thereto and heated to 100°C. After consumption of the naphthol derivative serving as a raw material, the resultant was cooled to room temperature and filtered. A solvent was removed from the resulting organic layer, followed by purification by chromatography on silica gel and recrystallization with a mixed solvent of ethanol/acetonitrile to obtain a photochromic compound represented by Formula (7-4) below at a yield of 58%.

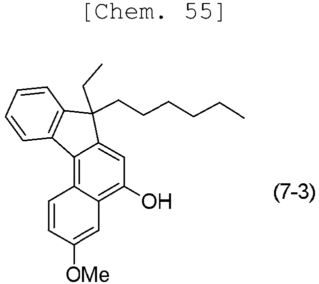

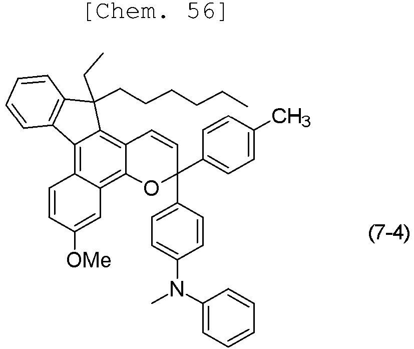

Elemental analysis values for the photochromic compound represented by Formula (7-4) were C: 86.05%, H: 7.21%, N: 2.06%, which are in good agreement with the calculated values for C₄₉H₄₉NO₂ of C: 86.05%, H: 7.22%, N: 2.05%.

Proton nuclear magnetic resonance spectra were measured and showed a 24H peak based on a methyl group, an ethyl group, a hexyl group around δ: 0.0 to 3.5 ppm, a 3H peak based on a methoxy group around δ: 3.5 to 5.0 ppm, and a 22H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 8)

### First step

A carboxylic acid compound represented by Formula (8-1) below was synthesized in the same manner as Example 5, except that 4-bromo-4'-methoxybenzophenone was used instead of 4-bromo-3',4'-dimethylbenzophenone in the first step.

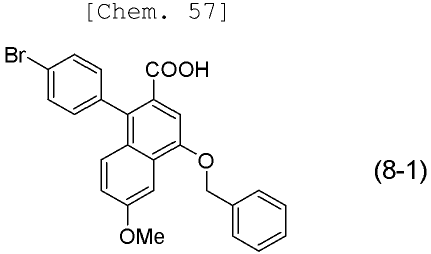

### Second step

A carboxylic acid represented by Formula (8-2) below was synthesized at a yield of 91% in the same manner as Example 7, except that the compound represented by Formula (7-1) was used instead of 4-bromo-4'-methylbenzophenone and diphenylamine was used instead of N-methylaniline in the first step.

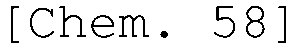

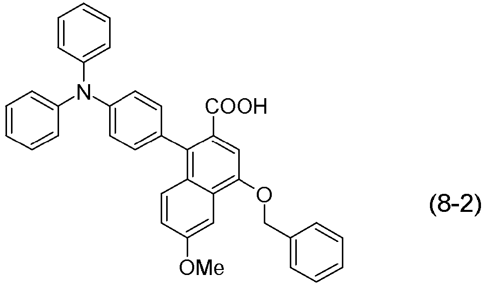

### Third step

A photochromic compound represented by Formula (7-3) below was obtained at a yield of 57% in the same manner as Example 5, except that a compound represented by Formula (7-2) was used instead of the compound represented by Formula (5-2) in the third step and 4-nonanone was used instead of methyl butyl ketone in the fourth step.

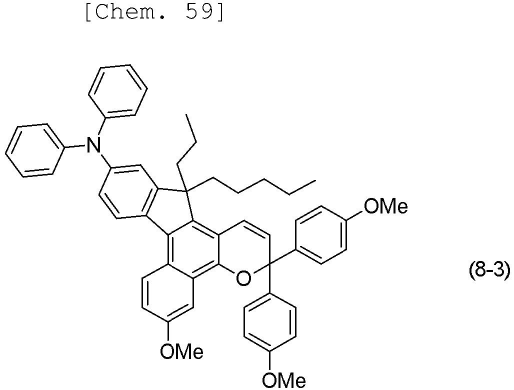

Elemental analysis values for the photochromic compound represented by Formula (8-3) were C: 83.42%, H: 6.76%, N: 1.78%, which are in good agreement with the calculated values for C₃₃H₅₃NO₄ of C: 83.41%, H: 6.74%, N: 1.77%.

Proton nuclear magnetic resonance spectra were measured and showed an 18H peak based on a propyl group, a pentyl group around δ: 0.0 to 3.5 ppm, a 9H peak based on a methoxy group around δ: 3.5 to 5.0 ppm, and a 26H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 9)

A photochromic compound represented by Formula (9-1) below was obtained at a yield of 66% in the same manner as Example 6, except that N-methylaniline was used instead of morpholine in the first step and methyl ethyl ketone was used instead of 4-octanone in the second step.

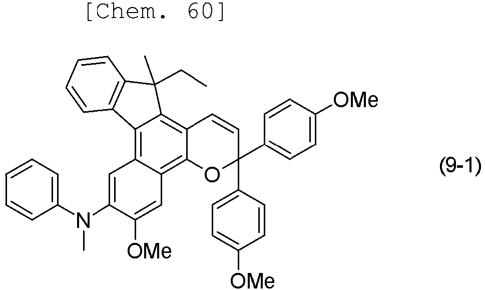

Elemental analysis values for the photochromic compound represented by Formula (9-1) were C: 81.92%, H: 6.26%, N: 2.11%, which are in good agreement with the calculated values for C₄₅H₅₁NO₄ of C: 81.91%, H: 6.26%, N: 2.12%.

Proton nuclear magnetic resonance spectra were measured and showed an 11H peak based on a methyl group, an ethyl group around δ: 0.0 to 3.5 ppm, a 9H peak based on a methoxy group around δ: 3.5 to 5.0 ppm, and a 21H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 10)

A photochromic compound represented by Formula (10-1) below was obtained at a yield of 56% in the same manner as Example 5, except that 3-bromo-4-methoxy-4'-methylbenzophenone was used instead of 4-bromo-3',4'-dimethylbenzophenone in the first step , 4-morpholinopheylboronic acid was used instead of 4-methylphenylboronic acid in the second step, 4-octaonne was used instead of methyl butyl ketone in the fourth step, and the compound represented by Formula (3-1) was used instead of the compound represented by Formula (5-5) in the fifth step.

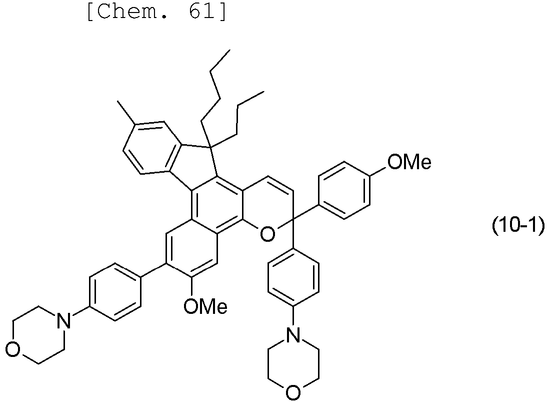

Elemental analysis values for the photochromic compound represented by Formula (10-1) were C: 79.99%, H: 7.17%, N: 3.34%, which are in good agreement with the calculated values for C₅₆H₆₀N₂O₅ of C: 79.97%, H: 7.19%, N: 3.33%.

Proton nuclear magnetic resonance spectra were measured and showed a 27H peak based on a methyl group, a propyl group, a butyl group, a morpholino group around δ: 0.0 to 3.5 ppm, a 14H peak based on a methoxy group, a morpholino group around δ: 3.5 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 11)

A photochromic compound represented by Formula (11-1) below was obtained at a yield of 54% in the same manner as Example 10, except that 3-bromo-4-methoxy-4'-bromobenzophenone was used instead of 3-bromo-4-methoxy-4'-methylbenzophenone, an excessive amount of 4-isopropoxypheylboronic acid was used instead of 4-morpholinophenylboronic acid, 3-nonanon was used instead of 4-octanone, and the compound represented by Formula (2-1) was used instead of the compound represented by Formula (3-1).

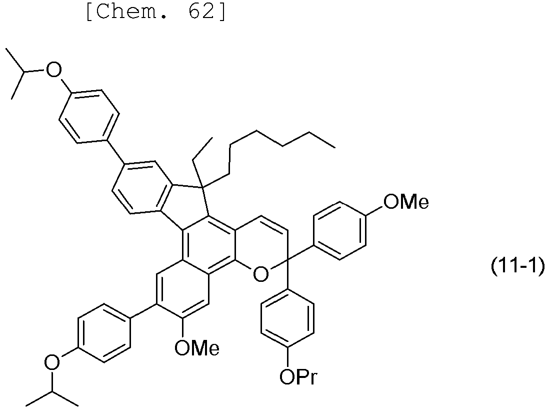

Elemental analysis values for the photochromic compound represented by Formula (11-1) were C: 82.16%, H: 7.43%, N: 3.34%, which are in good agreement with the calculated values for C₅₆H₆₀N₂O₅ of C: 82.14%, H: 7.44%.

Proton nuclear magnetic resonance spectra were measured and showed a 35H peak based on an ethyl group, a hexyl group, an isopropoxy group, a propoxy group around δ: 0.0 to 3.5 ppm, a 10H peak based on a methoxy group, an isopropoxy group, a propoxy group around δ: 3.5 to 5.0 ppm, and a 23H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 12)

### First step

Propargyl alcohol represented by Formula (12-1) below was synthesized in the same manner as Example 7, except that 4-bromo-4'-methoxybenzophenone was used instead of 4-bromo-4'-methylbenzophenone and diphenylamine was used instead of N-methylaniline in the first step.

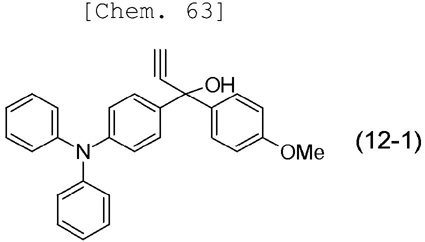

### Second step

A photochromic compound represented by Formula (12-2) below was obtained at a yield of 62% in the same manner as Example 10, except that 3-bromo-4,4'dimethylbenzophenone was used instead of 3-bromo-4-methoxy-4'-methylbenzophenone, 4-methoxyphenylboronic acid was used instead of 4-morpholinophenylboronic acid, ethyl propyl ketone was used instead of 4-octanone, and a compound represented by Formula (12-1) was used instead of the compound represented by Formula (3-1) .

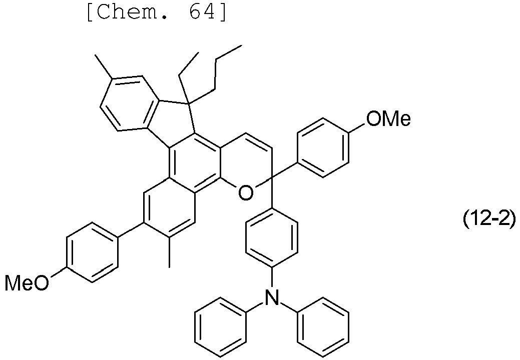

Elemental analysis values for the photochromic compound represented by Formula (12-2) were C: 86.01%, H: 6.46%, N: 1.71%, which are in good agreement with the calculated values for C₅₈H₅₁NO₃ of C: 85.99%, H: 6.48%, N: 1.70%.

Proton nuclear magnetic resonance spectra were measured and showed a 18H peak based on a methyl group, an ethyl group, a propyl group around δ: 0.0 to 3.5 ppm, a 6H peak based on a methoxy group, a morpholino group around δ: 3.5 to 5.0 ppm, and a 29H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 13)

### First step

Propargyl alcohol represented by Formula (13-1) below was synthesized in the same manner as Example 12, except that N-methylaniline was used instead of diphenylamine in the first step.

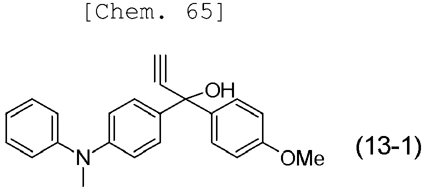

### Second step

A photochromic compound represented by Formula (13-2) below was obtained at a yield of 56% in the same manner as Example 10, except that 3-bromo-4-methoxybenzophenone was used instead of 3-bromo-4-methoxy-4'-methylbenzophenone, 2,4-dimethoxyphenylboronic acid was used instead of 4-morpholinophenylboronic acid, methyl ethyl ketone was used instead of 4-octanone, and a compound represented by Formula (13-1) was used instead of the compound represented by Formula (3-1).

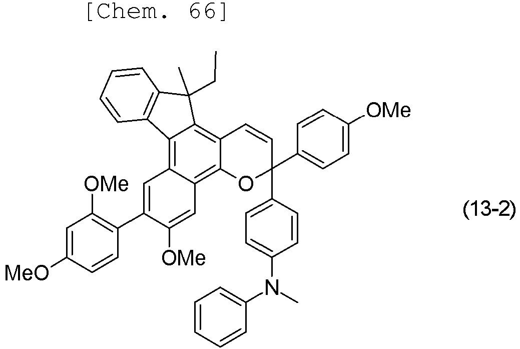

Elemental analysis values for the photochromic compound represented by Formula (13-2) were C: 81.56%, H: 6.17%, N: 1.82%, which are in good agreement with the calculated values for C₅₂H₄₇NO₅ of C: 81.54%, H: 6.19%, N: 1.83%.

Proton nuclear magnetic resonance spectra were measured and showed an 11H peak based on a methyl group, an ethyl group around δ: 0.0 to 3.5 ppm, a 12H peak based on a methoxy group around δ: 3.5 to 5.0 ppm, and a 24H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 14)

A photochromic compound represented by Formula (14-1) below was obtained at a yield of 64% in the same manner as Example 6, except that methyl propyl ketone was used instead of 4-octanone in the second step.

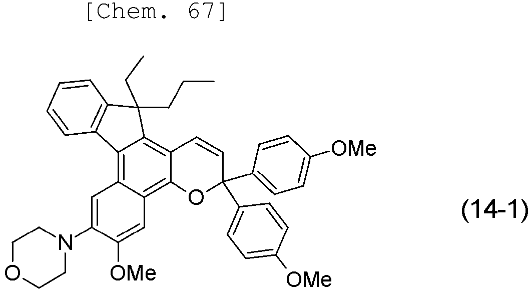

Elemental analysis values for the photochromic compound represented by Formula (14-1) were C: 79.12%, H: 6.77%, N: 2.11%, which are in good agreement with the calculated values for C₄₄H₄₅NO₅ of C: 79.13%, H: 6.79%, N: 2.10%.

Proton nuclear magnetic resonance spectra were measured and showed a 16H peak based on an ethyl group, a propyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 15)

A photochromic compound represented by Formula (15-1) below was obtained at a yield of 65% in the same manner as Example 1, except that 4-methoxy-4'-trifluoromethoxybenzophenone was used instead of 4,4'-dimethylbenzophenone and 3-octanone was used instead of the methyl propyl ketone in the first step and the compound represented by Formula (5-5) was used instead of the compound represented by Formula (1-5) in the fourth step.

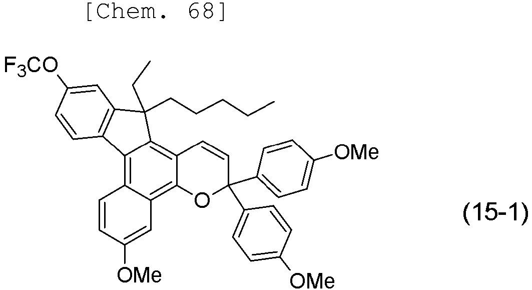

Elemental analysis values for the photochromic compound represented by Formula (15-1) were C: 74.31%, H: 5.93%, which are in good agreement with the calculated values for C₄₃H₄₁F₃O₅ of C: 74.33%, H: 5.95%.

Proton nuclear magnetic resonance spectra were measured and showed a 16H peak based on an ethyl group, a pentyl group around δ: 0.0 to 3.5 ppm, a 9H peak based on a methoxy group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 16)

A photochromic compound represented by Formula (16-1) below was obtained at a yield of 61% in the same manner as Example 6, except that 1,2,3,4-tetrahydroquinoline was used instead of morpholine in the first step and 5-decanone was used instead of 4-octanone and the compound represented by Formula (2-1) was used instead of the compound represented by Formula (1-5) in the second step.

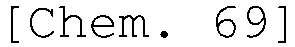

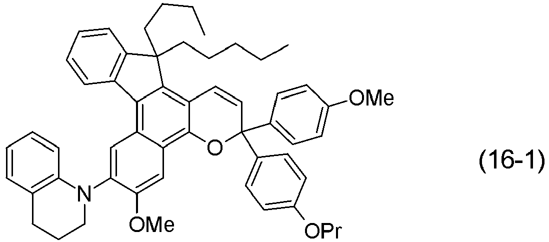

Elemental analysis values for the photochromic compound represented by Formula (16-1) were C: 82.79%, H: 7.44%, N: 1.76%, which are in good agreement with the calculated values for C₅₅H₅₉NO₄ of C: 82.77%, H: 7.45%, N: 1.76%.

Proton nuclear magnetic resonance spectra were measured and showed a 31H peak based on a butyl group, a pentyl group, a propoxy group, a 1,2,3,4-tetrahydroquinoline ring group around δ: 0.0 to 3.5 ppm, a 8H peak based on a methoxy group, a propoxy group around δ: 3.5 to 5.0 ppm, and a 20H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 17)

### First step

3-Bromoanisole was reacted with 4'-(1,1-dimethylethyl) [1,1'-biphenyl]-4-carbonyl chloride with reference to the method described in Patent Document 8 to obtain benzophenone represented by Formula (17-1) below at a yield of 88%.

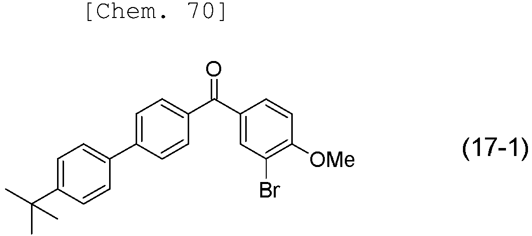

### Second step

A photochromic compound represented by Formula (17-2) below was obtained at a yield of 53% in the same manner as Example 6, except that the benzophenone represented by Formula (17-1) was used instead of 3-bromo-4-methoxybenzophenone, piperidine was used instead of morpholine, and 3-heptanone was used instead of 4-octanone.

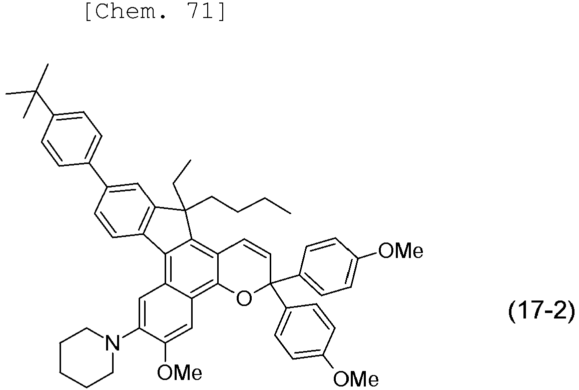

Elemental analysis values for the photochromic compound represented by Formula (17-2) were C: 82.80%, H: 7.58%, N: 1.72%, which are in good agreement with the calculated values for C₅₆H₆₁NO₄ of C: 82.82%, H: 7.57%, N: 1.72%.

Proton nuclear magnetic resonance spectra were measured and showed a 33H peak based on an ethyl group, a butyl group, a tertiary-butyl group, a piperidino group around δ: 0.0 to 3.5 ppm, a 9H peak based on a methoxy group around δ: 3.5 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 18)

A photochromic compound represented by Formula (18-1) below was obtained at a yield of 58% in the same manner as Example 6, except that 3-nonanone was used instead of 4-octanone in the second step.

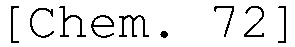

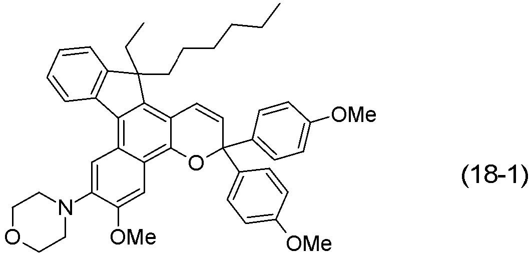

Elemental analysis values for the photochromic compound represented by Formula (18-1) were C: 79.50%, H: 7.23%, N: 1.98%, which are in good agreement with the calculated values for C₄₇H₅₁NO₅ of C: 79.52%, H: 7.24%, N: 1.97%.

Proton nuclear magnetic resonance spectra were measured and showed a 22H peak based on an ethyl group, a hexyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 19)

A photochromic compound represented by Formula (19-1) below was obtained at a yield of 49% in the same manner as Example 6, except that 2-tridecanone was used instead of 4-octanone in the second step.

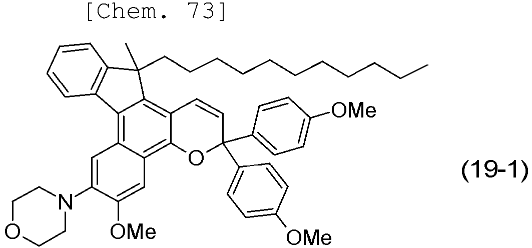

Elemental analysis values for the photochromic compound represented by Formula (19-1) were C: 79.99%, H: 7.74%, N: 1.84%, which are in good agreement with the calculated values for C₅₁H₅₉NO₅ of C: 79.96%, H: 7.76%, N: 1.83%.

Proton nuclear magnetic resonance spectra were measured and showed a 30H peak based on a methyl group, a undecyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 20)

A photochromic compound represented by Formula (20-1) below was obtained at a yield of 51% in the same manner as Example 6, except that 3-methyl-4-octanone was used instead of 4-octanone in the second step.

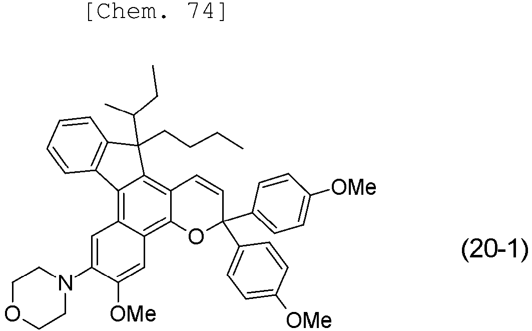

Elemental analysis values for the photochromic compound represented by Formula (20-1) were C: 79.54%, H: 7.24%, N: 1.96%, which are in good agreement with the calculated values for C₄₇H₅₁NO₅ of C: 79.52%, H: 7.24%, N: 1.97%.

Proton nuclear magnetic resonance spectra were measured and showed a 22H peak based on a 2-methylpropyl group, a butyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Evaluation of photochromic plastic lens made by coating method for physical property)

### (Example 21)

### (Preparation of curable composition)

First, the photochromic compound obtained in Example 1, a photoinitiator, and a polymerizable compound were mixed to obtain a curable composition.

The polymerizable compound was a combination of the following radical polymerizable monomers:

Polyethylene glycol dimethacrylate (average molecular weight: 736): 42 parts by mass
Polyethylene glycol dimethacrylate (average molecular weight: 536): 12 parts by mass
Trimethylolpropane methacrylate: 38 parts by mass γ-Methacryloyloxypropyltrimethoxysilane: 2 parts by mass Glycidyl methacrylate: 1 part by mass.

Note that, in the curable composition, the photochromic compound was added in an amount of 0.25 mmol taking a total amount of the radical polymerizable monomers as 100 g.

The following additives were used:

Phenylbis(2,4,6-trimethylbenzoyl)phosphine oxide (photoinitiator: Omnirad 819): 0.3 parts by mass Ethylene bis(oxyethylene) bis[3-(5-tert-butyl-4-hydroxy-m-tolyl) propionate] (stabilizer, Irganox 245): 1 part by mass Bis(1,2,2,6,6-pentamethyl-4-piperidyl)sebacate: 3 parts by mass
Leveling agent (L-7001) manufactured by DuPont Toray Specialty Materials K.K.: 0.1 parts by mass.

Note that, the above-mentioned additives are described with blended ratios taking a total of the radical polymerizable monomers as 100 parts by mass.

### (Production of optical article)

Using this curable composition, a photochromic laminate was obtained by a lamination method including polymerization as follows.

First, a thiourethane plastic lens with a center thickness of 2 mm and a refractive index of 1.60 was prepared as an optical substrate. Note that, the thiourethane plastic lens had been alkaline-etched in a 10% sodium hydroxide aqueous solution at 50°C for 5 minutes in advance, and then thoroughly washed with distilled water.

A spin coater (1H-DX2, manufactured by MIKASA CO., LTD) was used to coat a surface of the above-mentioned plastic lens with a moisture-curing primer (product name: TR-SC-P, manufactured by Tokuyama Corporation) at a rotation speed of 70 rpm for 15 seconds, followed by 1000 rpm for 10 seconds. Then, about 2 g of the photochromic curable composition obtained above was spin-coated at a rotation speed of 60 rpm for 40 seconds, followed by 600 rpm for 10 to 20 seconds so as to achieve a photochromic coating layer with a thickness of 40 µm.

The lens of which surface had been coated with the photochromic curable composition (photochromic coating layer) was irradiated with light for 90 seconds in a nitrogen gas atmosphere using a metal halide lamp with an output of 200 mW/cm² to cure the coating. The resultant was then further heated at 110°C for 1 hour to produce a photochromic laminate with a photochromic layer.

### (Examples 22 to 40)

Photochromic laminates were produced using the photochromic compounds obtained in Examples 2 to 20 in the same manner as in Example 21.

### (Comparative Examples 1 to 4)

Photochromic laminates were obtained using photochromic compounds represented by Formulae (A) to (D) below in the same manner as in Example 21.

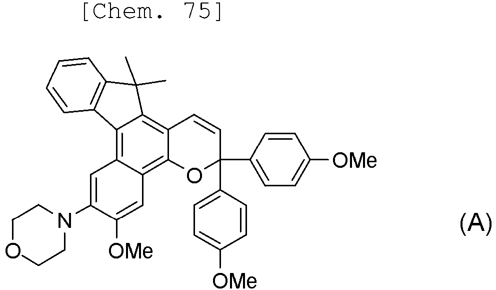

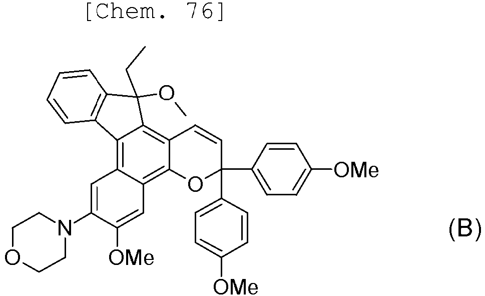

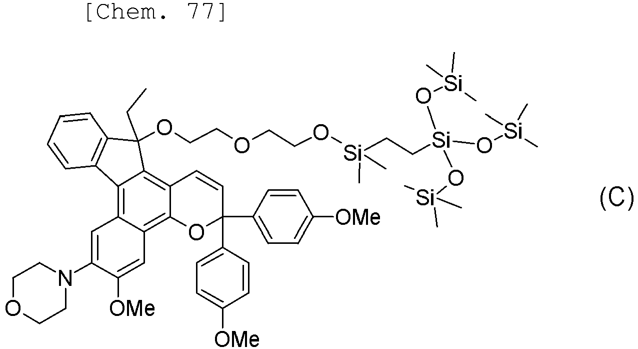

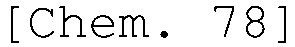

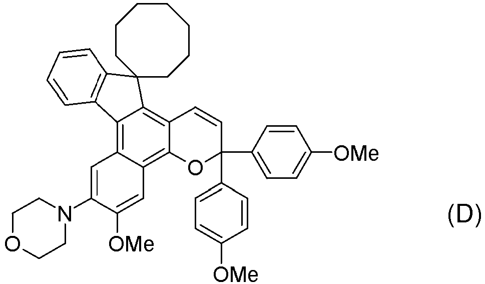

### (Synthesis of Compound A)

Compound (A) was synthesized in the same manner as Example 6, except that acetone was used instead of 4-octanone in the second step.

### (Synthesis of Compound C)

Compound (C) was synthesized by reacting a compound represented by Formula (C-1) below, which was synthesized with reference to the method described in Patent Document 13, with tris(trimethylsiloxy)silyl ethyl chlorosilane with reference to the method described in Patent Document 3.

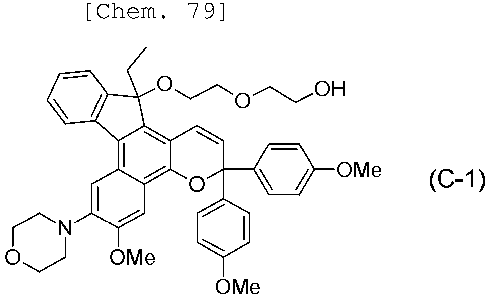

### (Synthesis of Compound D)

Compound (D) was synthesized in the same manner as Example 6, except that cyclooctanone was used instead of 4-octanone in the second step.

### <Evaluation method>

The resulting photochromic laminates were evaluated by the below-mentioned methods.
(1) Photochromic property
   [1] Maximum absorption wavelength (λmax):
      This is the maximum absorption wavelength after color development determined by a spectrophotometer manufactured by Otsuka Electronics Co., Ltd. (instantaneous multichannel photo detector MCPD3000) and was used as an index of a color tone upon color development.
   [2] Color developing density at 23°C (A₂₃):
      This is a difference between an absorbance {ε(240)} after irradiation with light at 23°C for 240 seconds and an absorbance ε(0) when not irradiated with light at the maximum absorption wavelength and was used as an index of a color developing density. The higher this value is, the better a photochromic property is.
   [3] Color fading half-life at 23°C [τ1/2 (sec).)]:
      This is a period of time taken by an absorbance at the maximum absorption wavelength of a sample to decrease to 1/2 of {ε(240)-ε(0)} when irradiation is stopped after irradiation with light at 23°C for 240 seconds, and was used as an index of a color fading rate. The shorter this period of time is, the faster the color fading rate is.
   [4] Residual ratio (A₉₆/A₀ x 100): The resulting photochromic plastic lens was subjected to accelerated degradation for 96 hours using a xenon weather meter X25 manufactured by Suga Test Instruments Co., Ltd. Color developing densities were then evaluated before and after a test, and a color developing density before the test (A₀) and a color developing density after the test (A₉₆) were measured. A ratio (A₉₆/A₀) was determined as a residual ratio and was used as an index of color development durability. The higher the residual ratio is, the more durable the color development is.

The results of Examples 26, 34, 38 to 40, and Comparative Examples 1 to 4 are summarized in Table 1 and the results of Examples 21 to 25, 27 to 33, and 35 to 37 are summarized in Table 2.

### [Table 1]

**table 1**

| | Compound # | Maximum absorption wavelength (nm) | Color developing density at 23°C (-) | Color fading half-life at 23°C (sec) | Residual ratio (%) |
|---|---|---|---|---|---|
| Example 26 | Formula (6-2) | 468 | 1.53 | 120 | 55% |
| | | 568 | 0.72 | 120 | 58% |
| Example 34 | Formula (14-1) | 468 | 1.47 | 110 | 55% |
| | | 566 | 0.70 | 110 | 57% |
| Example 38 | Formula (18-1) | 468 | 1.56 | 130 | 53% |
| | | 567 | 0.73 | 131 | 55% |
| Example 39 | Formula (19-1) | 470 | 1.60 | 150 | 47% |
| | | 569 | 0.77 | 151 | 51% |
| Example 40 | Formula (20-1) | 465 | 1.46 | 160 | 44% |
| | | 566 | 0.66 | 163 | 48% |
| Comparative Example 1 | Formula (A) | 474 | 1.65 | 170 | 47% |
| | | 567 | 0.83 | 172 | 50% |
| Comparative Example 2 | Formula (B) | 471 | 1.29 | 58 | 37% |
| | | 572 | 0.70 | 58 | 41% |
| Comparative Example 3 | Formula (C) | 471 | 1.27 | 63 | 34% |
| | | 572 | 0.70 | 63 | 36% |
| Comparative Example 4 | Formula (D) | 470 | 1.61 | 188 | 51% |
| | | 560 | 0.89 | 188 | 53% |

### [Table 2]

**table 2**

| | Compound # | Maximum absorption wavelength (nm) | Color developing density at 23°C (-) | Color fading half-life at 23°C (sec) | Residual ratio (%) |
|---|---|---|---|---|---|
| Example 21 | Formula (1-5) | 438 | 0.63 | 137 | 62% |
| | | 559 | 1.10 | 137 | 62% |
| Example 22 | Formula (2-2) | 447 | 0.86 | 99 | 68% |
| | | 571 | 0.79 | 99 | 68% |
| Example 23 | Formula (3-2) | 455 | 0.57 | 94 | 52% |
| | | 607 | 1.00 | 96 | 54% |
| Example 24 | Formula (4-5) | 482 | 0.73 | 43 | 69% 67 |
| | | 599 | 0.75 | 43 | % |
| Example 25 | Formula (5-6) | 457 | 1.28 | 102 | 60% |
| | | 581 | 0.84 | 103 | 61% |
| Example 27 | Formula (7-4) | 473 | 0.61 | 68 | 60% |
| | | 602 | 1.12 | 69 | 60% |
| Example 28 | Formula (8-3) | 450 | 0.92 | 100 | 63% |
| | | 621 | 1.03 | 100 | 65% |
| Example 29 | Formula (9-1) | 502 | 1.16 | 79 | 71% |
| Example 30 | Formula (10-1) | 486 | 1.05 | 85 | 61% |
| | | 606 | 1.04 | 85 | 62% |
| Example 31 | Formula (11-1) | 458 | 1.19 | 90 | 69% 69% |
| | | 589 | 1.02 | 91 | |
| Example 32 | Formula (12-2) | 489 | 0.62 | 90 | 58% |
| | | 572 | 1.22 | 95 | 57% |
| Example 33 | Formula (13-2) | 490 | 0.73 | 64 | 63% |
| | | 605 | 1.02 | 65 | 63% |
| Example 35 | Formula (15-1) | 442 | 0.62 | 42 | 68% |
| | | 567 | 0.73 | 43 | 69% |
| Example 36 | Formula (16-1) | 519 | 1.29 | 92 | 59% |
| Example 37 | Formula (17-2) | 477 | 1. 69 | 145 | 52% |
| | | 571 | 0.82 | 142 | 53% |

### <Evaluation of photochromic layer made by binder method for physical property)

### (Example 41)

A binder sheet was produced as follows. The binder sheet was made by laminating a first optical sheet, a first adhesive layer, a photochromic layer, a second adhesive layer, and a second optical sheet in this order. A polycarbonate sheet with a thickness of 400 µm was used as the first optical sheet and the second optical sheet.

### (Production of composition for forming photochromic layer)

A 2 L four-necked flask equipped with a stirring blade, a condense tube, a thermometer, and a nitrogen gas inlet tube was charged with 315 parts by mass of polycarbonate diol with a number average molecular weight of 1000, 100 parts by mass of isophorone diisocyanate, and 72 parts by mass of toluene and reacted under a nitrogen atmosphere at 100°C for 7 hours. Thus, a urethane prepolymer with a terminal isocyanate group was synthesized. After the reaction of the above-mentioned urethane prepolymer was completed, the resulting reaction liquid was cooled to about 0°C and dissolved in 205 parts by mass of tertiary-butyl alcohol and 382 parts by mass of diethyl ketone, and then a temperature of the resulting liquid was kept at 0°C. Next, a mixed solution of 21.3 parts by mass of bis-(4-aminocyclohexyl)methane serving as a chain extender and 20 parts by mass of diethyl ketone was added dropwise thereto within 30 minutes and reacted at 0°C for 1 hour. Then, 8.1 parts by mass of 1,2,2,6,6-pentamethyl-4-aminopiperidine was added dropwise thereto and reacted at 0°C for 1 hour to obtain a solution of a terminal non-reactive urethane urea resin in diethylketone.

One hundred parts by mass of the resulting solution of a terminal non-reactive urethane urea resin, 6.3 parts by mass of Example 1 (a photochromic compound represented by Formula (1-7), an isomer mixture of 4,4'-methylenebis(cyclohexylisocyanate) (polyisocyanate compound)), 0.4 parts by mass of ethylenebis(oxyethylene) bis[3-(5-tert-butyl-4-hydroxy-m-tolyl) propionate] serving as an antioxidant, and 0.06 parts by mass of L-7001 manufactured by DuPont Toray Specialty Materials K.K. serving as a surfactant were added and stirred and mixed at room temperature to obtain a composition for forming a photochromic layer.

Note that, the photochromic compound was added at a concentration of 0.25 mmol relative to 100 parts by mass of the terminal non-reactive urethane urea resin.

### (Production of composition for adhesive layer)

A 5 L separable flask (4-necked) equipped with a stirring blade, a condense tube, a thermometer, and a nitrogen gas inlet tube was prepared and charged with 400 parts by mass of polycarbonate diol with a number average molecular weight of 1000, 175 parts by mass of isophorone diisocyanate, and 120 parts by mass of toluene. These were reacted under a nitrogen atmosphere at 110°C for 7 hours. Thus, a urethane prepolymer with a terminal isocyanate group was synthesized. After the reaction of the urethane prepolymer is completed, the resulting reaction liquid was cooled to about 20°C and dissolved in 2500 parts by mass of propylene glycol-monomethyl ether, and then a temperature of the resulting liquid was kept at 20°C. Next, 60 parts by mass of isophoronediamine serving as a chain extender was added dropwise thereto and reacted at 20°C for 1 hour. Then, 3 parts by mass of n-butylamine was added dropwise thereto and reacted at 20°C for 1 hour to obtain a solution of a terminal non-reactive urethane urea resin in propylene glycol-monomethyl ether.

Then, 0.2 parts by mass of L-7001 manufactured by DuPont Toray Specialty Materials K.K. serving as a surfactant was added to 500 parts by mass of the resulting solution of a terminal non-reactive urethane urea resin and the resultant was stirred and mixed at room temperature to obtain a composition for an adhesive layer.

### (Production of binder sheet)

One main surface of the first optical sheet was coated with the composition for an adhesive layer using a coater (manufactured by TESTER SANGYO CO, LTD.) at a coating speed of 0.5 m/min and dried at a drying temperature of 110°C for 3 minutes to obtain a first optical sheet having a first coating film with a thickness of 5 µm. In the same way, one main surface of the second optical sheet was coated with the composition for an adhesive layer to obtain a second optical sheet having a second coating film.

Then, an oriented polypropylene film (OPP film) with a thickness of 50 µm was coated with the composition for forming a photochromic layer using a coater (manufactured by TESTER SANGYO CO,. LTD.) at a coating speed of 0.3 m/min and dried at a drying temperature of 100°C for 5 minutes. Thus, a third coating film was obtained. The third coating film was then bonded to the first optical sheet so that the third coating film was in contact with the first coating film. The OPP film was peeled off from the resulting structure, and the second optical sheet was bonded to the third coating film so that the thus-exposed main surface of the third coating film was in contact with the second coating film. The resulting laminate was then left to stand at 40°C under vacuum for 24 hours, subjected to heat treatment at 110°C for 60 minutes and humidification treatment at 60°C and 100% RH for 24 hours, and finally left to stand at 40°C under vacuum for 24 hours to obtain a binder sheet. The resulting binder sheet was evaluated in the same manner as for Example 21. The results are shown in Table 3.

### (Examples 42 to 53, Comparative Examples 5, 6)

Binder sheets were produced using the photochromic compounds shown in Table 3 in the same manner as in Example 41.

### [Table 3]

**table 3**

| | Compound # | Maximum absorption wavelength (nm) | Color developing density at 23°C (-) | Color fading half-life at 23°C (sec) | Residual ratio (%) |
|---|---|---|---|---|---|
| Example 41 | Formula (1-5) | 438 | 0.65 | 150 | 82% |
| | | 558 | 1.11 | 152 | 82% |
| Example 42 | Formula (2-2) | 447 | 0.89 | 115 | 91% 90 |
| | | 571 | 0.83 | 116 | % |
| Example 43 | Formula (4-5) | 484 | 0.76 | 49 | 90% |
| | | 599 | 0.75 | 49 | 90% |
| Example 44 | Formula (5-6) | 459 | 1.33 | 120 | 80% |
| | | 580 | 0.84 | 121 | 81% |
| Example 45 | Formula (6-2) | 469 | 1.57 | 141 | 72% |
| | | 568 | 0.71 | 142 | 75% |
| Example 46 | Formula (9-1) | 503 | 1.19 | 87 | 93% |
| Example 47 | Formula (11-1) | 458 | 1.21 | 103 | 92% |
| | | 587 | 1.03 | 104 | 91% |
| Example 48 | Formula (13-2) | 491 | 0.74 | 73 | 82% |
| | | 602 | 1.05 | 73 | 83% |
| Example 49 | Formula (14-1) | 468 | 1.52 | 125 | 75% |
| | | 566 | 0.70 | 126 | 75% |
| Example 50 | Formula (15-1) | 444 | 0.61 | 51 | 90% |
| | | 567 | 0.73 | 51 | 91% |
| Example 51 | Formula (17-2) | 477 | 1.68 | 161 | 68% |
| | | 571 | 0.84 | 159 | 70% |
| Example 52 | Formula (18-1) | 470 | 1.60 | 143 | 71% |
| | | 567 | 0.74 | 145 | 74% |
| Example 53 | Formula (19-1) | 471 | 1.65 | 175 | 62% |
| | | 568 | 0.77 | 177 | 66% |
| Comparative Example 5 | Formula (A) | 474 | 1.67 | 190 | 63% |
| | | 566 | 0.82 | 192 | 65% |
| Comparative Example 6 | Formula (B) | 471 | 1.32 | 65 | 50% |
| | | 572 | 0.71 | 66 | 51% |

### <Evaluation of photochromic cured product made by kneading method for physical property)

### (Example 54)

### (Preparation of curable composition)

First, the photochromic compound obtained in Example 1, an additive, and a polymerizable compound were mixed to obtain a curable composition. The polymerizable compound was a combination of the following polymerizable monomers:
· 1,3-Bis(isocyanatomethyl)cyclohexane: 36.7 parts by mass
· Pentaerythritol tetrakis(3-mercaptopropionate): 39.4 parts by mass
· Polyoxyethylene polyoxypropylene lauryl ether (WONDERSURF 140 manufactured by AOKI OIL INDUSTRIAL Co., Ltd.): 17.4 parts by mass
· 1-Decanethiol: 2.8 parts by mass
· RX-1 prepared by the method described in Patent Document 14: 3.8 parts by mass

Note that, in the curable composition, the photochromic compound was added in an amount of 0.106 mmol taking a total amount of the polymerizable monomers as 100 g.

The following additives were used:
· Dimethyltin dichloride: 0.05 parts by mass
· Irganox 245: 0.1 parts by mass
· 2-Ethylhexyl 4-methoxysilicate: 0.6 parts by mass

### (Production of cured product)

After the thus-prepared curable composition was thoroughly defoamed, it was poured into a glass mold with a gap of 1 mm and polymerized by casting polymerization. The polymerization was performed in an air furnace for 18 hours with a temperature gradually increased from 27°C to 120°C. After the polymerization, the resulting cured product was removed from the glass mold to obtain a photochromic cured product with a thickness of 1 mm. The resulting photochromic cured product was evaluated in the same manner as for Example 23. The results are shown in Table 4.

### (Examples 55 to 66, Comparative Examples 7, 8)

Photochromic cured products were produced using the photochromic compounds shown in Table 4 in the same manner as in Example 50. The results are summarized in Table 4.

### [Table 4]

**table 4**

| | Compound # | Maximum absorption wavelength (nm) | Color developing density at 23°C (-) | Color fading half-life at 23°C (sec) | Residual ratio (%) |
|---|---|---|---|---|---|
| Example 54 | Formula (1-5) | 441 | 0.66 | 191 | 71% |
| | | 564 | 1.10 | 191 | 71% |
| Example 55 | Formula (2-2) | 449 | 0.90 | 140 | 78% |
| | | 579 | 0.89 | 141 | 78% |
| Example 56 | Formula (4-5) | 485 | 0.77 | 64 | 79% |
| | | 605 | 0.74 | 64 | 77% |
| Example 57 | Formula (7-4) | 475 | 0.63 | 94 | 67% |
| | | 605 | 1.13 | 95 | 66% |
| Example 58 | Formula (8-3) | 452 | 0.96 | 141 | 71% |
| | | 623 | 1. 07 | 143 | 72% |
| Example 59 | Formula (9-1) | 505 | 1.20 | 111 | 79% |
| Example 60 | Formula (10-1) | 489 | 1.10 | 125 | 70% |
| | | 609 | 1.07 | 125 | 69% |
| Example 61 | Formula (12-2) | 491 | 0.64 | 132 | 66% |
| | | 578 | 1.23 | 134 | 65% |
| Example 62 | Formula (13-2) | 492 | 0.75 | 91 | 72% |
| | | 609 | 1.05 | 90 | 71% |
| Example 63 | Formula (14-1) | 471 | 1.49 | 162 | 63% |
| | | 575 | 0.72 | 163 | 64% |
| Example 64 | Formula (17-2) | 479 | 1.70 | 210 | 58% |
| | | 579 | 0.83 | 208 | 59% |
| Example 65 | Formula (18-1) | 472 | 1.62 | 186 | 60% |
| | | 574 | 0.76 | 186 | 61% |
| Example 66 | Formula (19-1) | 473 | 1.65 | 221 | 52% |
| | | 576 | 0.79 | 222 | 55% |
| Comparative Example 7 | Formula (A) | 476 | 1.68 | 240 | 54% |
| | | 574 | 0.85 | 241 | 56% |
| Comparative Example 8 | Formula (B) | 474 | 1.32 | 80 | 40% |
| | | 579 | 0.69 | 79 | 42% |

### <Evaluation of photochromic compound for solubility>

The photochromic compounds according to Examples and Comparative examples shown in Table 5 were evaluated for solubility in polymerizable compounds.

Specifically, a sample was prepared by adding each of the photochromic compounds to the composition, which consisted of a radical polymerizable compound, among the curable compositions used in Example 21. The photochromic compounds had a concentration of 35 µmol/g.

Note that, this test was performed using "YOKAI-KUN USS-1" manufactured by NIHONSEIKI KAISHA LTD. under the following conditions:
Dissolution temperature: 65 to 70°C
Stirring condition: Stirring dial of 5. Ultrasonic irradiation condition: Irradiation interval of 3 sec.

Dissolution was checked every 15 minutes up to 1 hour later, the sample was visually checked by transmitting light through the sample, and a degree of dissolution was evaluated according to the following four evaluation criteria.

### (Evaluation criteria)

1: No haze is seen when checked under transmission of light, and the sample is completely dissolved.
2: A very thin haze is seen to the extent that it can be seen under transmission of light.
3: A thin haze that can be seen under transmission of light is seen.
4: A haze can be seen even without transmission of light.

A shorter time required for the evaluation result to be 1 means higher solubility. The evaluation results for solubility are shown in Table 5.

### [Table 5]

**table 5**

| | Compound | Dissolution for 15 min | Dissolution for 30 min | Dissolution for 45 min | Dissolution for 1 hour |
|---|---|---|---|---|---|
| Example 67 | Formula (6-2) | 4 | 2 | 1 | 1 |
| Example 68 | Formula (14-1) | 4 | 2 | 1 | 1 |
| Example 69 | Formula (18-1) | 3 | 2 | 1 | 1 |
| Example 70 | Formula (19-1) | 2 | 1 | 1 | 1 |
| Example 71 | Formula (20-1) | 3 | 2 | 1 | 1 |
| Comparative Example 9 | Formula (A) | 4 | 3 | 3 | 3 |
| Comparative Example 10 | Formula (B) | 4 | 3 | 1 | 1 |
| Comparative Example 11 | Formula (C) | 2 | 1 | 1 | 1 |
| Comparative Example 12 | Formula (D) | 4 | 3 | 2 | 1 |

### Example 72

### First step

First, 27.4 g (211.7 mmol) of N-ethyldiisopropylamine and 360 mL of toluene were added to 36.0 g (73.0 mmol) of the carboxylic acid represented by Formula (5-1) and stirred under reduced pressure to remove dissolved oxygen. Then, 4.5 g (4.9 mmol) of Pd₂(dba)₃ and 5.7 g (9.8 mmol) of 4,5-bis(diphenylphosphino)-9,9-dimethylxanthene were added to the resulting reaction liquid, stirred at room temperature for 30 minutes, and then heated to an internal temperature of 70°C. Then, 25.1 g (141.1 mmol) of 3-trifluoromethylbenzenethiol was slowly added dropwise thereto, after that, the resultant was heated to reflux. Heating was continued until the raw materials were exhausted. After the reaction was completed, the resultant was cooled to room temperature and 540 mL of tetrahydrofuran was added thereto. Then, the resultant was partitioned by adding 360 mL of water thereto and this procedure was repeated 3 times. A solvent was removed from the organic layer, followed by purification by chromatography on silica gel to obtain a carboxylic acid represented by Formula (72-1) below at a yield of 56%.

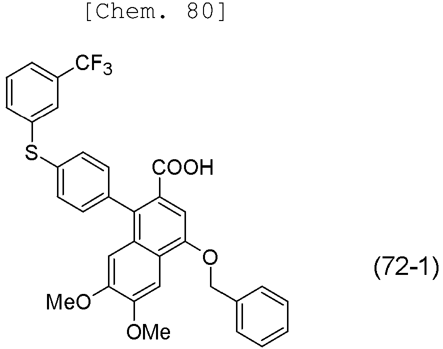

### Second step

A naphthol compound represented by Formula (72-2) below was synthesized in the same manner as Example 5, except that the carboxylic acid represented by Formula (72-1) was used instead of the carboxylic acid represented by Formula (5-2) in the third step and ethyl propyl ketone was used instead of methyl butyl ketone in the fourth step.

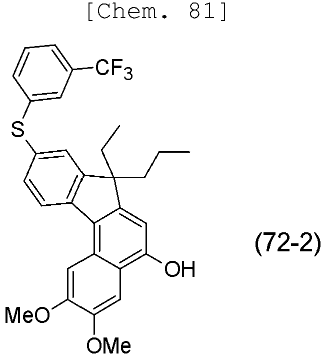

### Third step

A photochromic compound represented by Formula (72-3) below was obtained at a yield of 63% in the same manner as Example 1, except that a compound represented by Formula (72-2) was used instead of the compound represented by Formula (1-4) and the compound represented by Formula (2-1) was used instead of the compound represented by Formula (1-5) in the fourth step of.

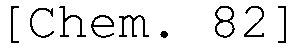

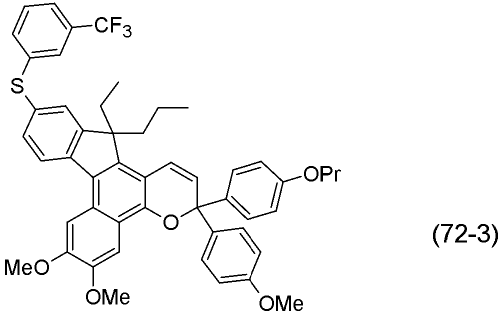

Elemental analysis values for the photochromic compound represented by Formula (72-3) were C: 73.50%, H: 5.78%, S: 3.91%, which are in good agreement with the calculated values for C₅₀H₄₇F₃O₅S of C: 73.51%, H: 5.80%, S: 3.92%. Proton nuclear magnetic resonance spectra were measured and showed a 17H peak based on an ethyl group, a propyl group, a propyloxy group around δ: 0.0 to 3.5 ppm, a 11H peak based on a methoxy group, a propyloxy group around δ: 3.5 to 5.0 ppm, and a 19H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 73

### First step

A naphthol compound represented by Formula (73-1) below was synthesized in the same manner as Example 8, except that a carboxylic acid represented by Formula (72-1) was used instead of the carboxylic acid represented by Formula (8-1) and N-methylaniline was used instead of diphenylamine in the second step of and methyl butyl ketone was used instead of 4-nonanone in the third step.

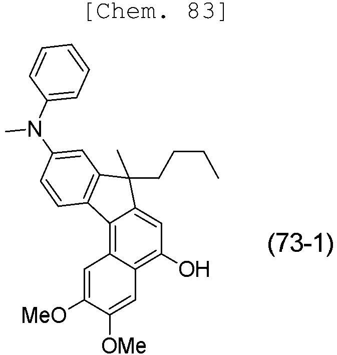

### Second step

A photochromic compound represented by Formula (73-2) below was obtained at a yield of 68% in the same manner as Example 1, except that the compound represented by Formula (73-1) was used instead of the compound represented by Formula (1-4) and the compound represented by Formula (2-1) was used instead of the compound represented by Formula (1-5) in the fourth step.

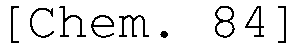

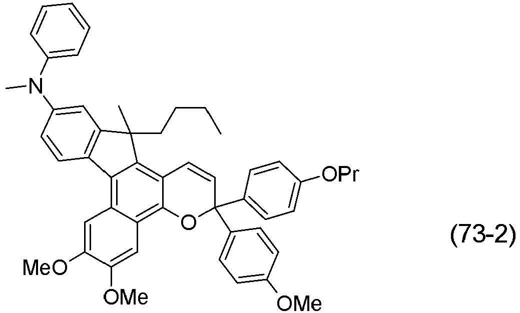

Elemental analysis values for the photochromic compound represented by Formula (73-2) were C: 80.51%, H: 6.91%, N: 1.90%, which are in good agreement with the calculated values for C₅₀H₅₁NO₅ of C: 80.51%, H: 6.89%, N: 1.88%. Proton nuclear magnetic resonance spectra were measured and showed a 20H peak based on a methyl group, a butyl group, a propyloxy group around δ: 0.0 to 3.5 ppm, a 11H peak based on a methoxy group, a propyloxy group around δ: 3.5 to 5.0 ppm, and a 20H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 74

### First step

A naphthol compound represented by Formula (74-1) below was synthesized in the same manner as Example 4, except that 3-bromo-4,4'-dimethoxybenzophenone was used instead of 3-bromo-4-methoxybenzophenone in the first step and methyl ethyl ketone was used instead of ethyl propyl ketone in the second step.

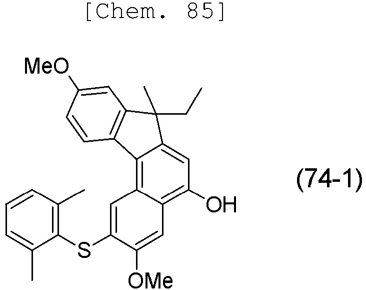

### Second step

A photochromic compound represented by Formula (74-2) below was obtained at a yield of 68% in the same manner as Example 1, except that the compound represented by Formula (74-1) was used instead of the compound represented by Formula (1-4) and the compound represented by Formula (3-1) was used instead of the compound represented by Formula (1-5) in the fourth step.

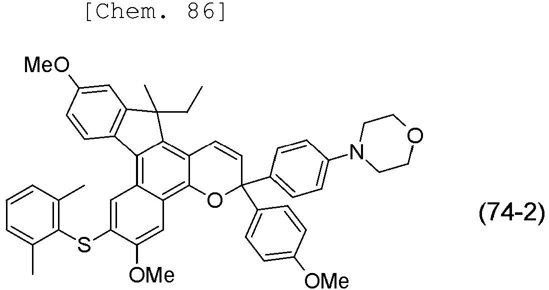

Elemental analysis values for the photochromic compound represented by Formula (74-2) were C: 77.40%, H: 6.36%, N: 1.82%, S: 4.11%, which are in good agreement with the calculated values for C₅₀H₄₉NO₅S of C: 77.39%, H: 6.36%, N: 1.81%, S: 4.13%. Proton nuclear magnetic resonance spectra were measured and showed a 18H peak based on a methyl group, an ethyl group, a morpholino group around δ: 0.0 to 3.5 ppm, a 13H peak based on a methoxy group, a morpholino group around δ: 3.5 to 5.0 ppm, and a 18H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 75

### First step

A naphthol compound represented by Formula (75-1) below was synthesized in the same manner as Example 8, except that 4-bromo-3-methyl-4'-methoxybenzophenone was used instead of 4-bromo-4'-methoxybenzophenone in the first step of, benzmorpholine was used instead of diphenylamine in the second step, and methyl ethyl ketone was used instead of 4-nonanone in the third step.

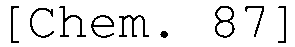

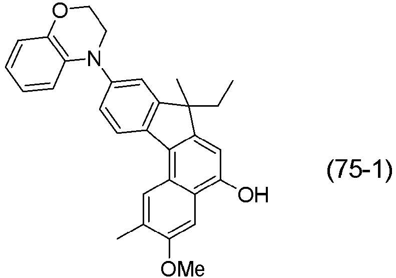

### Second step

A photochromic compound represented by Formula (75-2) below was obtained at a yield of 67% in the same manner as Example 1, except that the compound represented by Formula (75-1) was used instead of the compound represented by Formula (1-4) and the compound represented by Formula (2-1) was used instead of the compound represented by Formula (1-5) in the fourth step.

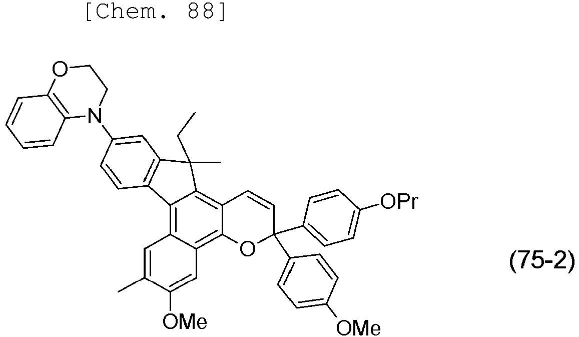

Elemental analysis values for the photochromic compound represented by Formula (75-2) were C: 80.63%, H: 6.51%, N: 1.91%, which are in good agreement with the calculated values for C₄₉H₄₇NO₅ of C: 80.63%, H: 6.49%, N: 1.92%. Proton nuclear magnetic resonance spectra were measured and showed a 18H peak based on a methyl group, an ethyl group, a benzmorpholino group, a propyloxy group around δ: 0.0 to 3.5 ppm, a 10H peak based on a methoxy group, a benzmorpholino group around δ: 3.5 to 5.0 ppm, and a 19H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 76

### First step

A compound represented by Formula (76-1) below was synthesized in the same manner as Example 10, except that 3-bromo-4-methoxybenzophenone was used instead of 3-bromo-4-methoxy-4'-methylbenzophenone, 4-chlorophenylboronic acid was used instead of 4-morpholinophenylboronic acid, and ethyl butyl ketone was used instead of 4-octanone in the first step of.

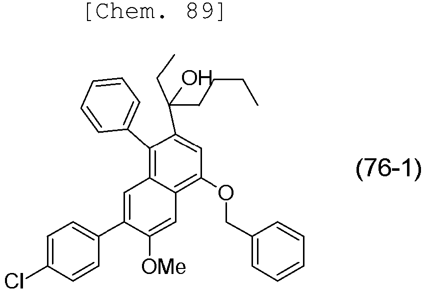

### Second step

The reaction was performed in the same manner as Example 6, except that the compound represented by Formula (76-1) was used instead of the carboxylic acid represented by Formula (4-1) and diphenylamine was used instead of morpholine in the first step. After completion of the reaction, the resultant was partitioned by adding water thereto and repeatedly washed with water until a pH of an aqueous layer was 7. The resulting organic layer was concentrated, followed by purification by chromatography on silica gel to obtain a compound represented by Formula (76-2) below at a yield of 94%.

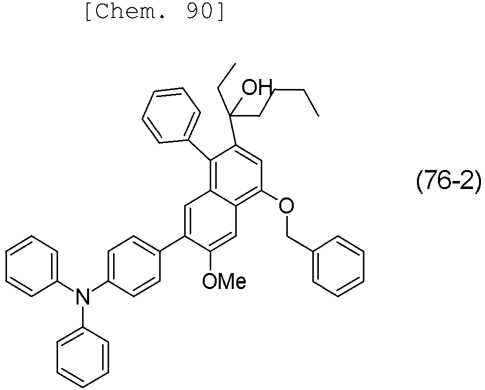

### Third step

A naphthol compound represented by Formula (76-3) below was synthesized in the same manner as Example 1, except that the compound represented by Formula (76-2) was used instead of the compound represented by Formula (1-2) in the second step.

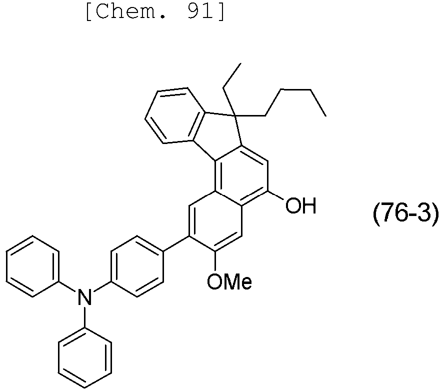

### Fourth step

Benzophenone represented by Formula (76-4) below was synthesized in the same manner Example 17, except that 3-fluoro-4-propoxybenzene was used instead of 3-bromoanisole and 4-bromobenzoylchloride was used instead of 4'-(1,1-dimethylethyl)[1,1'-biphenyl]-4-carbonylchloride in the first step.

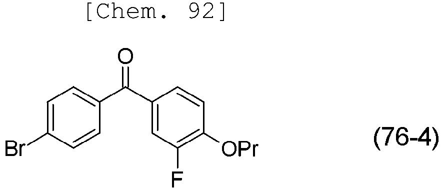

### Fifth step

Propargyl alcohol represented by Formula (76-5) below was synthesized in the same manner as Example 12, except that the benzophenone represented by Formula (76-4) was used instead of 4-bromo-4'-methylbenzophenone and N-methylaniline was used instead of diphenylamine in the first step.

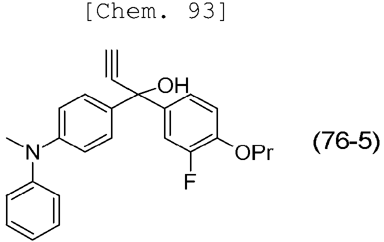

### Sixth step

A photochromic compound represented by Formula (76-6) below was obtained at a yield of 64% in the same manner as Example 1, except that the compound represented by Formula (76-3) was used instead of the compound represented by Formula (1-4) and the compound represented by Formula (76-5) was used instead of the compound represented by Formula (1-5) in the fourth step of.

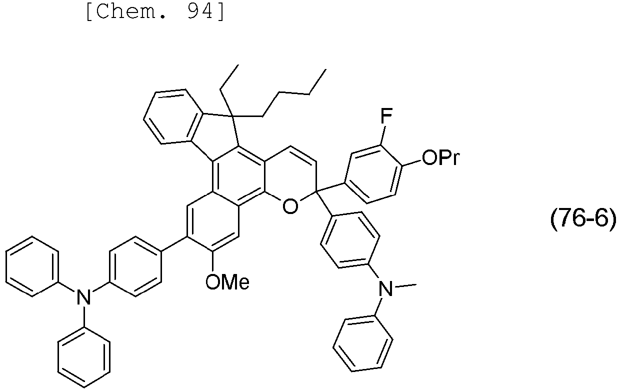

Elemental analysis values for the photochromic compound represented by Formula (76-6) were C: 83.69%, H: 6.41%, N: 2.91%, which are in good agreement with the calculated values for C₆₇H₆₁FN₂O₃ of C: 83.72%, H: 6.40%, N: 2.91%. Proton nuclear magnetic resonance spectra were measured and showed a 22H peak based on a methyl group, an ethyl group, a butyl group, a propyloxy group around δ: 0.0 to 3.5 ppm, a 5H peak based on a methoxy group, a propyloxy group around δ: 3.5 to 5.0 ppm, and a 34H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 77

### First step

A photochromic compound represented by Formula (77-1) below was obtained at a yield of 60% in the same manner as Example 74, except that ethyl butyl ketone was used instead of methyl ethyl ketone in the first step.

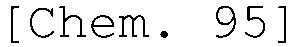

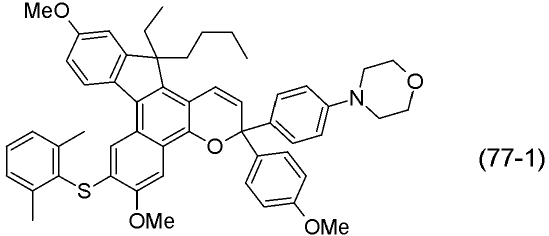

Elemental analysis values for the photochromic compound represented by Formula (77-1) were C: 83.69%, H: 6.41%, N: 2.91%, S: 3.92%, which are in good agreement with the calculated values for C53H55NO₅S of C: 77.81%, H: 6.78%, N: 1.71%, S: 3.92%. Proton nuclear magnetic resonance spectra were measured and showed a 24H peak based on a methyl group, an ethyl group, a butyl group, a morpholino group around δ: 0.0 to 3.5 ppm, a 13H peak based on a methoxy group, a morpholino group around δ: 3.5 to 5.0 ppm, and a 18H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 78

### First step

A naphthol compound represented by Formula (78-1) below was synthesized in the same manner as Example 13, except that 2,4,6-trimethoxyphenylboronic acid was used instead of 2,4-dimethoxyphenylboronic acid and ethyl propyl ketone was used instead of methyl ethyl ketone in the second step.

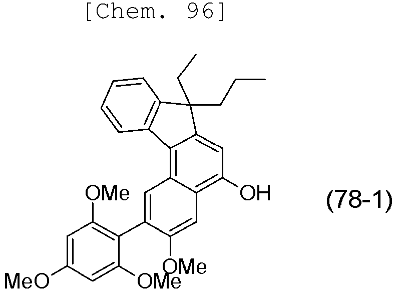

### Second step

A photochromic compound represented by Formula (78-2) below was obtained at a yield of 68% in the same manner as Example 1, except that the compound represented by Formula (78-1) was used instead of the compound represented by Formula (1-4) and the compound represented by Formula (12-1) was used instead of the compound represented by Formula (1-5) in the fourth step.

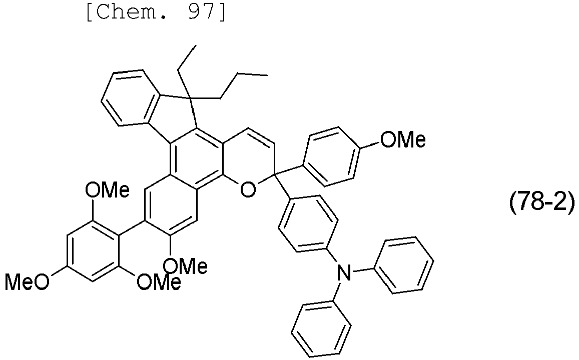

Elemental analysis values for the photochromic compound represented by Formula (78-2) were C: 81.35%, H: 6.25%, N: 1.56%, which are in good agreement with the calculated values for C₆₀H₅₅NO₆ of C: 81.33%, H: 6.26%, N: 1.58%. Proton nuclear magnetic resonance spectra were measured and showed a 12H peak based on an ethyl group, a propyl group around δ: 0.0 to 3.5 ppm, a 15H peak based on a methoxy group around δ: 3.5 to 5.0 ppm, and a 28H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 79

### First step

A naphthol compound represented by Formula (79-1) below was synthesized in the same manner as Example 10, except that ethyl propyl ketone was used instead of 4-octanone.

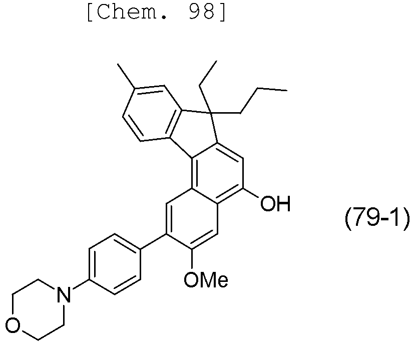

### Second step

A photochromic compound represented by Formula (79-2) below was obtained at a yield of 60% in the same manner as Example 1, except that the compound represented by Formula (79-1) was used instead of the compound represented by Formula (1-4) and the compound represented by Formula (3-1) was used instead of the compound represented by Formula (1-5) in the fourth step.

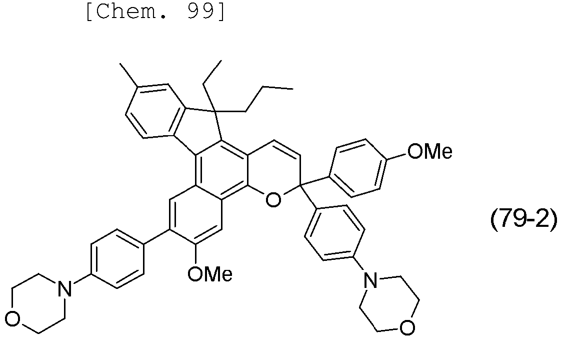

Elemental analysis values for the photochromic compound represented by Formula (79-2) were C: 79.79%, H: 6.93%, N: 3.48%, which are in good agreement with the calculated values for C₅₄H₅₆N₂O₅ of C: 79.77%, H: 6.94%, N: 3.45%. Proton nuclear magnetic resonance spectra were measured and showed a 23H peak based on a methyl group, an ethyl group, a propyl group, a morpholino group around δ: 0.0 to 3.5 ppm, a 14H peak based on a methoxy group, a morpholino group around δ: 3.5 to 5.0 ppm, and a 19H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 80

### First step

A naphthol compound represented by Formula (80-1) below was synthesized in the same manner as Example 9, except that ethyl butyl ketone was used instead of methyl ethyl ketone.

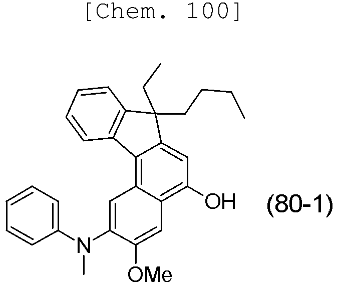

### Second step

A photochromic compound represented by Formula (80-2) below was obtained at a yield of 60% in the same manner as Example 1, except that the compound represented by Formula (80-1) was used instead of the compound represented by Formula (1-4) and the compound represented by Formula (5-5) was used instead of the compound represented by Formula (1-5) in the fourth step.

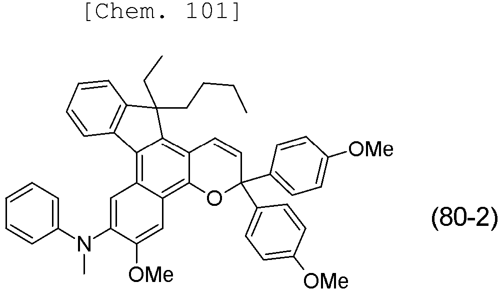

Elemental analysis values for the photochromic compound represented by Formula (80-2) were C: 82.15%, H: 6.77%, N: 1.99%, which are in good agreement with the calculated values for C₄₈H₄₇NO₄ of C: 82.14%, H: 6.75%, N: 2.00%. Proton nuclear magnetic resonance spectra were measured and showed a 17H peak based on a methyl group, an ethyl group, a butyl group around δ: 0.0 to 3.5 ppm, a 9H peak based on a methoxy group around δ: 3.5 to 5.0 ppm, and a 21H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 81

### First step

A naphthol compound represented by Formula (81-1) below was synthesized in the same manner as Example 76, except that 2-chlorophenylboronic acid was used instead of 4-chlorophenylboronic acid in the first step of and morpholine was used instead of diphenylamine in the second step.

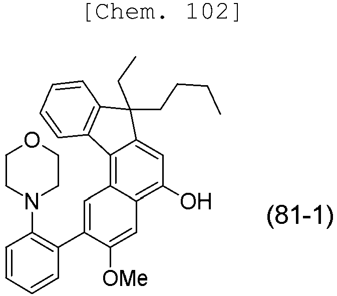

### Second step

A photochromic compound represented by Formula (81-2) below was obtained at a yield of 68% in the same manner as Example 1, except that the compound represented by Formula (81-1) was used instead of the compound represented by Formula (1-4) and the compound represented by Formula (5-5) was used instead of the compound represented by Formula (1-5) in the fourth step.

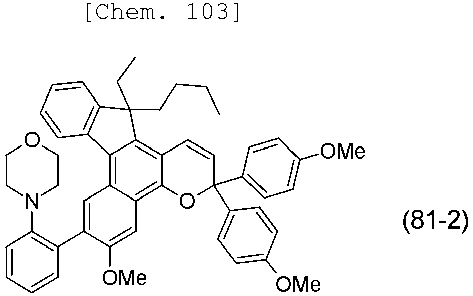

Elemental analysis values for the photochromic compound represented by Formula (81-2) were C: 80.83%, H: 6.77%, N: 1.85%, which are in good agreement with the calculated values for C₅₁H₅₁NO₅ of C: 80.82%, H: 6.78%, N: 1.85%. Proton nuclear magnetic resonance spectra were measured and showed a 18H peak based on an ethyl group, a butyl group, a morpholino group around δ: 0.0 to 3.5 ppm, a 13H peak based on a methoxy group, a morpholino group around δ: 3.5 to 5.0 ppm, and a 20H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 82

### First step

A photochromic compound represented by Formula (82-1) below was obtained at a yield of 71% in the same manner as Example 73, except that ethyl butyl ketone was used instead of methyl butyl ketone in the first step.

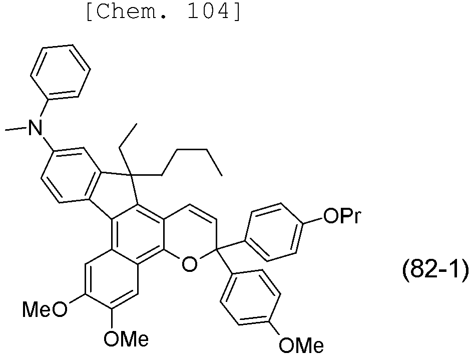

Elemental analysis values for the photochromic compound represented by Formula (82-1) were C: 80.63%, H: 7.01%, N: 1.85%, which are in good agreement with the calculated values for C₅₁H₅₃NO₅ of C: 80.60%, H: 7.03%, N: 1.85%. Proton nuclear magnetic resonance spectra were measured and showed a 22H peak based on a methyl group, an ethyl group, a butyl group, a propyloxy group around δ: 0.0 to 3.5 ppm, a 11H peak based on a methoxy group, a propyloxy group around δ: 3.5 to 5.0 ppm, and a 20H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 83

### First step

A naphthol compound represented by Formula (83-1) below was synthesized in the same manner as Example 9, except that ethyl propyl ketone was used instead of methyl ethyl ketone.

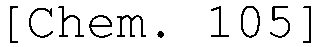

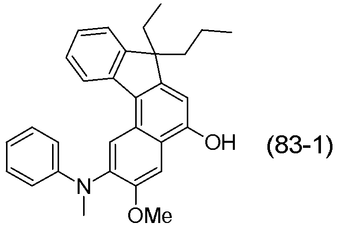

### Second step

A photochromic compound represented by Formula (83-2) below was obtained at a yield of 68% in the same manner as Example 1, except that the compound represented by Formula (83-1) was used instead of the compound represented by Formula (1-4) and the compound represented by Formula (12-1) was used instead of the compound represented by Formula (1-5) in the fourth step.

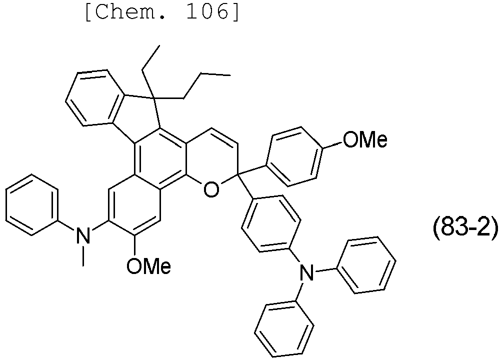

Elemental analysis values for the photochromic compound represented by Formula (83-2) were C: 84.43%, H: 6.33%, N: 3.39%, which are in good agreement with the calculated values for C₅₈H₅₂N₂O₃ of C: 84.43%, H: 6.35%, N: 3.40%. Proton nuclear magnetic resonance spectra were measured and showed a 15H peak based on a methyl group, an ethyl group, a propyl group around δ: 0.0 to 3.5 ppm, a 6H peak based on a methoxy group around δ: 3.5 to 5.0 ppm, and a 31H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 84

A photochromic compound represented by Formula (84-1) below was obtained at a yield of 56% in the same manner as Example 1, except that the compound represented by Formula (79-1) was used instead of the compound represented by Formula (1-4) and the compound represented by Formula (7-2) was used instead of the compound represented by Formula (1-5) in the fourth step.

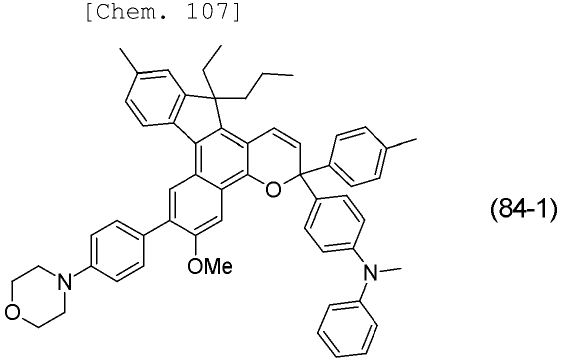

Elemental analysis values for the photochromic compound represented by Formula (84-1) were C: 83.79%, H: 6.93%, N: 3.44%, which are in good agreement with the calculated values for C₅₇H₅₆N₂O₃ of C: 83.79%, H: 6.91%, N: 3.43%. Proton nuclear magnetic resonance spectra were measured and showed a 25H peak based on a methyl group, an ethyl group, a propyl group, a morpholino group around δ: 0.0 to 3.5 ppm, a 7H peak based on a methoxy group, a morpholino group around δ: 3.5 to 5.0 ppm, and a 24H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 85

A photochromic compound represented by Formula (85-1) below was obtained at a yield of 55% in the same manner as Example 74, except that butyl hexyl ketone was used instead of methyl ethyl ketone in the first step.

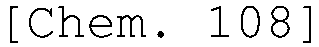

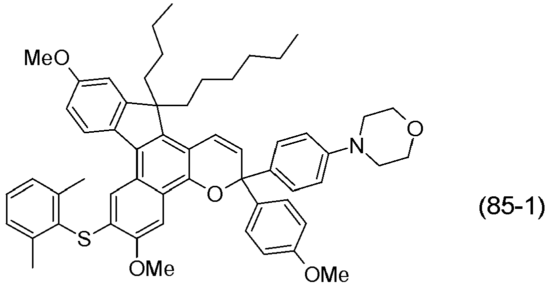

Elemental analysis values for the photochromic compound represented by Formula (85-1) were C: 78.30%, H: 7.28%, N: 1.59%, S: 3.66%, which are in good agreement with the calculated values for C₅₇H₆₃NO₅S of C: 78.31%, H: 7.26%, N: 1.60%, S: 3.67%. Proton nuclear magnetic resonance spectra were measured and showed a 32H peak based on a methyl group, a butyl group, a hexyl group, a morpholino group around δ: 0.0 to 3.5 ppm, a 13H peak based on a methoxy group, a morpholino group around δ: 3.5 to 5.0 ppm, and a 18H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 86

A photochromic compound represented by Formula (86-1) below was obtained at a yield of 63% in the same manner as Example 13, except that the compound represented by Formula (76-5) was used instead of the compound represented by Formula (13-1) in the second step.

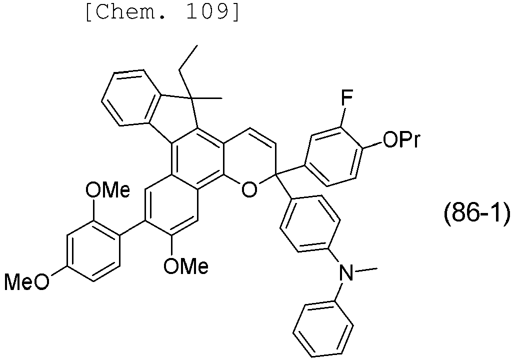

Elemental analysis values for the photochromic compound represented by Formula (86-1) were C: 79.86%, H: 6.20%, N: 1.72%, which are in good agreement with the calculated values for C₅₄H₅₀FNO₅ of C: 79.88%, H: 6.21%, N: 1.73%. Proton nuclear magnetic resonance spectra were measured and showed a 16H peak based on a methyl group, an ethyl group, a propyloxy group around δ: 0.0 to 3.5 ppm, a 11H peak based on a methoxy group, a propyloxy group around δ: 3.5 to 5.0 ppm, and a 23H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 87

### First step

A photochromic compound represented by Formula (87-1) below was obtained at a yield of 72% in the same manner as Example 73, except that ethyl propyl ketone was used instead of methyl butyl ketone in the first step.

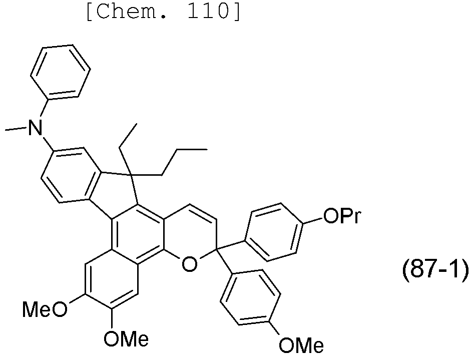

Elemental analysis values for the photochromic compound represented by Formula (87-1) were C: 80.53%, H: 6.89%, N: 1.88%, which are in good agreement with the calculated values for C₅₀H₃₁NO₅ of C: 80.51%, H: 6.89%, N: 1.88%. Proton nuclear magnetic resonance spectra were measured and showed a 20H peak based on a methyl group, an ethyl group, a propyl group, a propyloxy group around δ: 0.0 to 3.5 ppm, a 11H peak based on a methoxy group, a propyloxy group around δ: 3.5 to 5.0 ppm, and a 20H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 88

### First step

A naphthol compound represented by Formula (88-1) below was synthesized in the same manner as Example 4, except that the carboxylic acid compound represented by Formula (8-1) below was used instead of the carboxylic acid represented by Formula (4-1) in the first step.

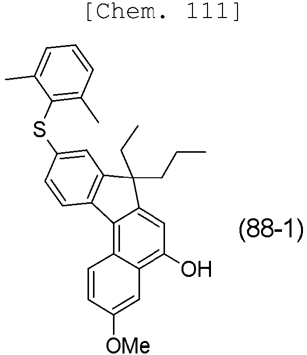

### Second step

A photochromic compound represented by Formula (88-2) below was obtained at a yield of 61% in the same manner as Example 1, except that the compound represented by Formula (88-1) was used instead of the compound represented by Formula (1-4) and the compound represented by Formula (13-1) was used instead of the compound represented by Formula (1-5) in the fourth step of.

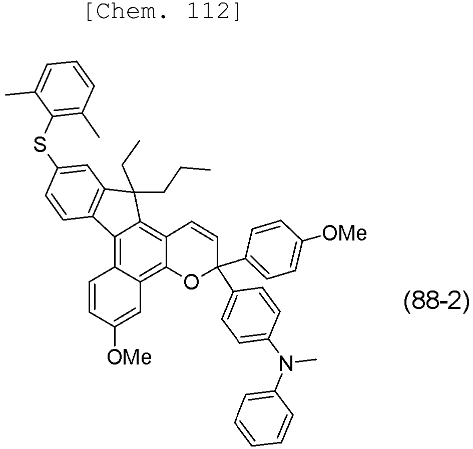

Elemental analysis values for the photochromic compound represented by Formula (88-2) were C: 81.69%, H: 6.45%, N: 1.77%, S: 4.02%, which are in good agreement with the calculated values for C₅₄H₅₁NO₃S of C: 81.68%, H: 6.47%, N: 1.76%, S: 4.04%. Proton nuclear magnetic resonance spectra were measured and showed a 21H peak based on a methyl group, an ethyl group, a propyl group around δ: 0.0 to 3.5 ppm, a 6H peak based on a methoxy group around δ: 3.5 to 5.0 ppm, and a 24H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 89

A photochromic compound represented by Formula (89-1) below was obtained at a yield of 63% in the same manner as Example 78, except that methyl propyl ketone was used instead of ethyl propyl ketone in the first step.

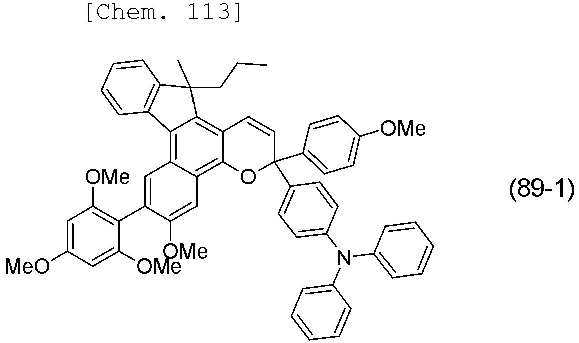

Elemental analysis values for the photochromic compound represented by Formula (89-1) were C: 81.24%, H: 6.16%, N: 1.60%, which are in good agreement with the calculated values for C₅₉H₅₃NO₆ of C: 81.26%, H: 6.13%, N: 1.61%. Proton nuclear magnetic resonance spectra were measured and showed a 10H peak based on a methyl group, a propyl group around δ: 0.0 to 3.5 ppm, a 15H peak based on a methoxy group around δ: 3.5 to 5.0 ppm, and a 28H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 90

A photochromic compound represented by Formula (90-1) below was obtained at a yield of 62% in the same manner as Example 15, except that 2-heptanone was used instead of 3-octanone.

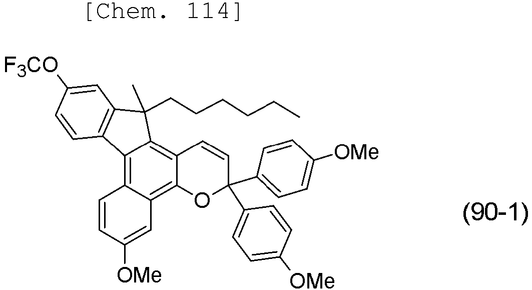

Elemental analysis values for the photochromic compound represented by Formula (90-1) were C: 74.30%, H: 5.93%, which are in good agreement with the calculated values for C₄₃H₄₁F₃O₅ of C: 74.33%, H: 5.95%. Proton nuclear magnetic resonance spectra were measured and showed a 16H peak based on a methyl group, a hexyl group around δ: 0.0 to 3.5 ppm, a 9H peak based on a methoxy group around δ: 3.5 to 5.0 ppm, and a 16H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 91

A photochromic compound represented by Formula (91-1) below was obtained at a yield of 67% in the same manner as Example 3, except that ethyl propyl ketone was used instead of 4-methyl-3-hexanone.

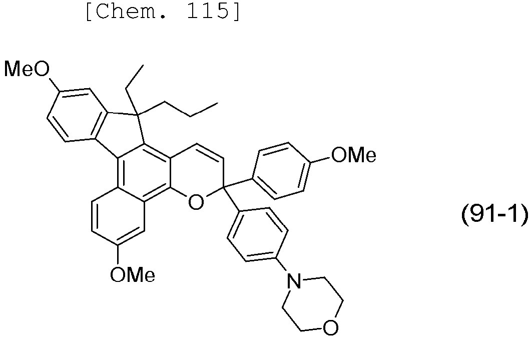

Elemental analysis values for the photochromic compound represented by Formula (91-1) were C: 79.14%, H: 6.81%, N: 2.09%, which are in good agreement with the calculated values for C₄₄H₄₅NO₅ of C: 79.13%, H: 6.79%, N: 2.10%. Proton nuclear magnetic resonance spectra were measured and showed a 16H peak based on an ethyl group, a propyl group, a morpholino group around δ: 0.0 to 3.5 ppm, a 13H peak based on a methoxy group, a morpholino group around δ: 3.5 to 5.0 ppm, and a 16H peak based on an aromatic proton and an alkene proton around δ: 5.0 to 9.0 ppm. Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Examples 92 to 111

Photochromic laminates were prepared using the photochromic compounds obtained in Examples 72 to 91 in the same manner as in Example 21. The evaluation results are summarized in Tables 6 and 7.

### [Table 6]

**table 6**

| | Compound # | Maximum absorption wavelength (nm) | Color developing density at 23°C (-) | Color fading half-life at 23°C (sec) | Residual ratio (%) |
|---|---|---|---|---|---|
| Example 92 | Formula (72-3) | 456 | 1.03 | 42 | 57% |
| | | 574 | 0. 64 | 42 | 57% |
| Example 93 | Formula (73-2) | 477 | 1.50 | 182 | 62% |
| | | 612 | 0.88 | 183 | 62% |
| Example 94 | Formula (74-2) | 487 | 0.83 | 90 | 64% |
| | | 613 | 0.97 | 90 | 64% |
| Example 95 | Formula (75-2) | 453 | 1.08 | 140 | 64% |
| | | 607 | 0.94 | 140 | 64% |
| Example 96 | Formula (76-6) | 509 | 1.06 | 70 | 68% |
| | | 603 | 1.08 | 70 | 68% |
| Example 97 | Formula (77-1) | 484 | 0.82 | 82 | 67% |
| | | 614 | 0.94 | 82 | 67% |
| Example 98 | Formula (78-2) | 496 | 0.75 | 126 | 64% |
| | | 587 | 1.05 | 130 | 64% |
| Example 99 | Formula (79-2) | 485 | 1.04 | 78 | 65% |
| | | 605 | 1.05 | 78 | 66% |
| Example 100 | Formula (80-1) | 500 | 1.09 | 70 | 79% |
| Example 101 | Formula (81-2) | 453 | 0.86 | 100 | 66% |
| | | 574 | 0.85 | 100 | 67% |

### [Table 7]

**table 7**

| | Compound # | Maximum absorption wavelength (nm) | Color developing density at 23°C (-) | Color fading half-life at 23°C (sec) | Residual ratio (%) |
|---|---|---|---|---|---|
| Example 102 | Formula (82-1) | 474 | 1.46 | 152 | 66% 6 |
| | | 610 | 0.84 | 153 | 5% |
| Example 103 | Formula (83-2) | 524 | 1.25 | 92 | 74% |
| Example 104 | Formula (84-1) | 500 | 1.19 | 120 | 62% |
| | | 607 | 1.25 | 120 | 62% |
| Example 105 | Formula (85-1) | 486 | 0.87 | 100 | 63% |
| | | 616 | 0.98 | 100 | 62% |
| Example 106 | Formula (86-1) | 493 | 0.75 | 77 | 58% |
| | | 600 | 1.02 | 78 | 58% |
| Example 107 | Formula (87-1) | 475 | 1. 47 | 159 | 64% |
| | | 609 | 0.84 | 159 | 64% |
| Example 108 | Formula (88-1) | 473 | 0.48 | 43 | 68% |
| | | 619 | 0.93 | 43 | 68% |
| Example 109 | Formula (89-1) | 499 | 0.77 | 160 | 60% |
| | | 588 | 1.10 | 164 | 59% |
| Example 110 | Formula (90-1) | 446 | 0.62 | 54 | 64% |
| | | 567 | 0.75 | 55 | 64% |
| Example 111 | Formula (91-1) | 459 | 0.56 | 75 | 57% |
| | | 610 | 1.07 | 75 | 57% |

### (Examples 112 to 127, Comparative Examples 13 to 20)

The photochromic compounds according to Examples and Comparative examples shown in Tables 8 to 14 were evaluated for solubility in polymerizable compounds in the same manner as in Example 67. Note that, for Comparative Examples 13 to 20, photochromic compounds represented by Formulae (E) to (L) below were evaluated for solubility in polymerizable compounds.

### (Synthesis of compound represented by Formula (E))

A compound represented by Formula (E) below was synthesized in the same manner as Example 3, except that 3,3,5,5-tetramethylcyclohexanone was used instead of 4-methyl-3-hexanone.

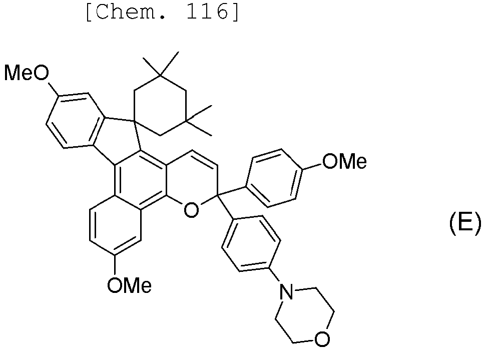

### (Synthesis of compound represented by Formula (F))

A compound represented by Formula (F) below was synthesized in the same manner as Example 9, except that diethyl ketone was used instead of methyl ethyl ketone.

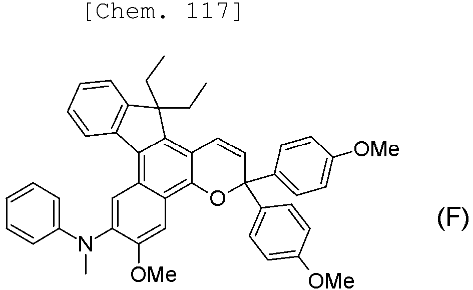

### (Synthesis of compound represented by Formula (G))

A compound represented by Formula (G) below was synthesized in the same manner as Example 9, except that acetone was used instead of 4-octanone.

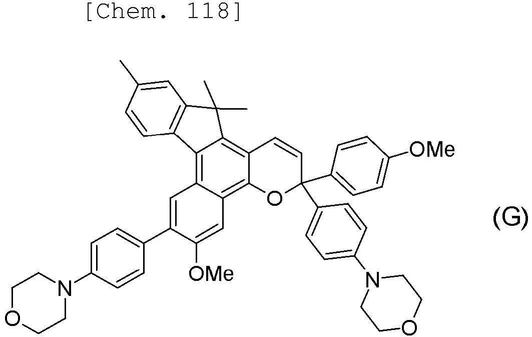

### (Synthesis of compound represented by Formula (H))

A compound represented by Formula (H) below was synthesized in the same manner as Example 15, except that acetone was used instead of 3-octanone.

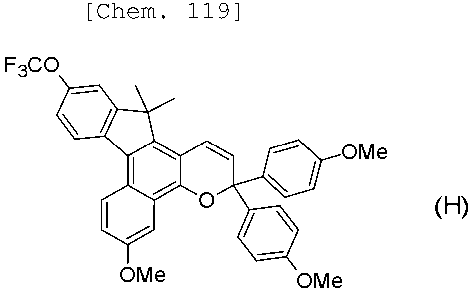

### (Synthesis of compound represented by Formula (I))

A compound represented by Formula (I) below was synthesized in the same manner as Example 73, except that acetone was used instead of methyl butyl ketone.

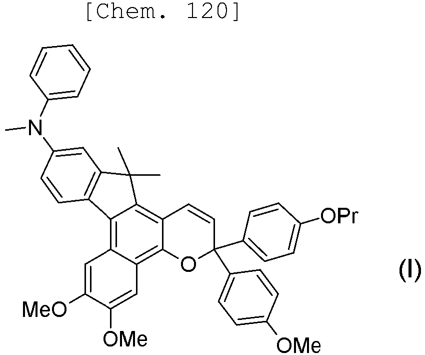

### (Synthesis of compound represented by Formula (J))

A compound represented by Formula (J) below was synthesized in the same manner as Example 74, except that 4,4-diethylcyclohexanone was used instead of methyl ethyl ketone .

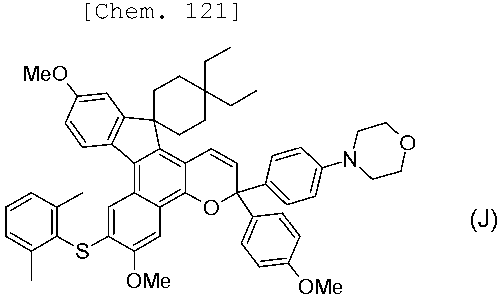

### (Synthesis of compound represented by Formula (K))

A compound represented by Formula (K) below was synthesized in the same manner as Example 74, except that acetone was used instead of methyl ethyl ketone.

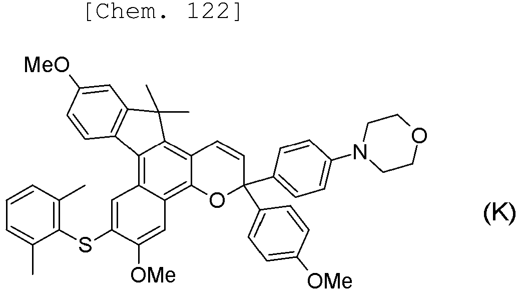

### (Synthesis of compound represented by Formula (L))

A compound represented by Formula (L) below was synthesized in the same manner as Example 78, except that acetone was used instead of ethyl propyl ketone.

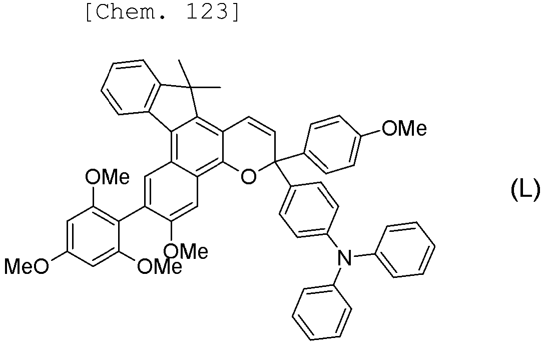

### [Table 8]

**table 8**

| | Compound | Dissolution for 15 min | Dissolution for 30 min | Dissolution for 45 min | Dissolution for 60 min |
|---|---|---|---|---|---|
| Example 112 | Formula (3-2) | 3 | 1 | 1 | 1 |
| Example 113 | Formula (91-1) | 4 | 3 | 1 | 1 |
| Comparative Example 13 | Formula (E) | 4 | 3 | 3 | 2 |

### [Table 9]

**table 9**

| | Compound | Dissolution for 15 min | Dissolution for 30 min | Dissolution for 45 min | Dissolution for 60 min |
|---|---|---|---|---|---|
| Example 114 | Formula (9-1) | 4 | 2 | 2 | 1 |
| Example 115 | Formula (80-2) | 3 | 2 | 1 | 1 |
| Comparative Example 14 | Formula (F) | 4 | 3 | 3 | 2 |

### [Table 10]

**table 10**

| | Compound | Dissolution for 15 min | Dissolution for 30 min | Dissolution for 45 min | Dissolution for 60 min |
|---|---|---|---|---|---|
| Example 116 | Formula (10-1) | 3 | 2 | 1 | 1 |
| Example 117 | Formula (79-2) | 3 | 2 | 1 | 1 |
| Comparative Example 15 | Formula (G) | 4 | 3 | 3 | 2 |

### [Table 11]

**table 11**

| | Compound | Dissolution for 15 min | Dissolution for 30 min | Dissolution for 45 min | Dissolution for 60 min |
|---|---|---|---|---|---|
| Example 118 | Formula (15-1) | 3 | 2 | 1 | 1 |
| Example 119 | Formula (90-1) | 3 | 2 | 1 | 1 |
| Comparative Example 16 | Formula (H) | 4 | 3 | 3 | 2 |

### [Table 12]

**table 12**

| | Compound | Dissolution for 15 min | Dissolution for 30 min | Dissolution for 45 min | Dissolution for 60 min |
|---|---|---|---|---|---|
| Example 120 | Formula (73-2) | 3 | 3 | 2 | 1 |
| Example 121 | Formula (82-1) | 3 | 2 | 1 | 1 |
| Example 122 | Formula (87-1) | 3 | 2 | 1 | 1 |
| Comparative Example 17 | Formula (I) | 4 | 4 | 3 | 3 |

### [Table 13]

**table 13**

| | Compound | Dissolution for 15 min | Dissolution for 30 min | Dissolution for 45 min | Dissolution for 60 min |
|---|---|---|---|---|---|
| Example 123 | Formula (74-2) | 4 | 3 | 2 | 1 |
| Example 124 | Formula (77-1) | 3 | 3 | 1 | 1 |
| Example 125 | Formula (85-1) | 3 | 2 | 1 | 1 |
| Comparative Example 18 | Formula (J) | 4 | 3 | 3 | 2 |
| Comparative Example 19 | Formula (K) | 4 | 4 | 3 | 3 |

### [Table 14]

**table 14**

| | Compound | Dissolution for 15 min | Dissolution for 30 min | Dissolution for 45 min | Dissolution for 60 min |
|---|---|---|---|---|---|
| Example 126 | Formula (78-2) | 3 | 2 | 1 | 1 |
| Example 127 | Formula (89-1) | 4 | 2 | 1 | 1 |
| Comparative Example 20 | Formula (L) | 4 | 3 | 2 | 1 |

Preferred embodiments of the present disclosure will be additionally described.
[1] A photochromic compound having a backbone represented by Formula (3) below: 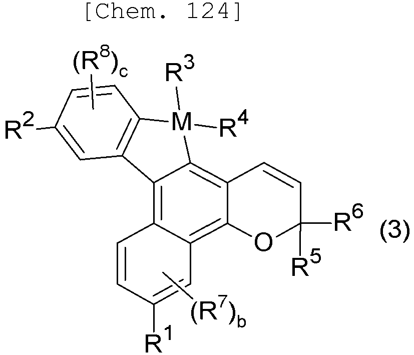 in which
   M is C, Si, or Ge;
   R¹ is a hydrogen atom or a substituent, provided that the substituent is not a halogen atom or a trifluoromethyl group;
   R² is a hydrogen atom or a substituent, provided that the substituent is not a halogen atom;
   R³ and R⁴ are each independently a linear or branched alkyl group and have different structures from each other;
   R⁵ and R⁶ are each independently a hydrogen atom or a substituent;
   R⁷ and R⁸ are each independently a substituent; and b and c are each independently an integer of 0, or 1 or more and 3 or less.
[2] The photochromic compound according to [1], in which, in the Formula (3), R³ is a linear or branched alkyl group having 1 to 19 carbon atoms, R⁴ is a linear or branched alkyl group having 2 to 20 carbon atoms, and a value obtained by subtracting a number of carbon atoms in the alkyl group for R³ from a number of carbon atoms in the alkyl group for R⁴ is 1 or more and 10 or less.
[3] The photochromic compound according to [1] or [2], in which, in the Formula (3), R³ is a linear alkyl group having 1 to 10 carbon atoms.
[4] The photochromic compound according to any one of [1] to [3], in which, in the Formula (3), R³ is a linear alkyl group having 1 to 9 carbon atoms, R⁴ is a linear alkyl group having 2 to 10 carbon atoms, and the value obtained by subtracting a number of carbon atoms in the alkyl group for R³ from a number of carbon atoms in the alkyl group for R⁴ is 1 or more and 9 or less.
[5] The photochromic compound according to any one of [1] to [4], in which, in the Formula (3), R³ is any of a methyl group, an ethyl group, or a propyl group.
[6] The photochromic compound according to any one of [1] to [5], in which, in the Formula (3), one of R³ or R⁴ is an ethyl group.
[7] The photochromic compound according to any one of [1] to [6], in which, in the Formula (3), R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) below, a group represented by Formula (X) below, or a group represented by Formula (X3) below;
   in the Formula (3),
   when b is 2 to 3, a plurality of R⁷s may be the same as or different from each other;
   when c is 2 to 3, a plurality of R⁸s may be the same as or different from each other;
   when b is 2 to 3 and adjacent R⁷s are present, two adjacent R⁷s may be taken together with a carbon atom to which R⁷s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings; and
   when c is 2 to 3 and adjacent R⁸s are present, two adjacent R⁸s may be taken together with a carbon atom to which R⁸s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings;

      -Q¹-(X¹Q²)ₐ-X²Q³ (2a)
   in the Formula (2a),
   Q¹ is an alkylene group or a haloalkylene group;
   Q² is an alkylene group or a haloalkylene group;
   Q³ is an alkyl group or a haloalkylene group;
   X¹ and X² are each independently O, S, NR⁷⁰⁰, PR⁷⁰¹, or P(=O); R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group; and
   a is an integer of 0, or 1 or more and 3 or less;

      L¹-R⁴⁰⁰ (X3)
   in the Formula (X3),
   R⁴⁰⁰ is a hydrogen atom, an alkyl group, an aryl group, a polymerizable group, a photochromic group, or a silyl group having an alkyl group, an alkoxyl group, or an aryl group as a substituent; and
   L¹ is a group represented by Formula (X2) below: 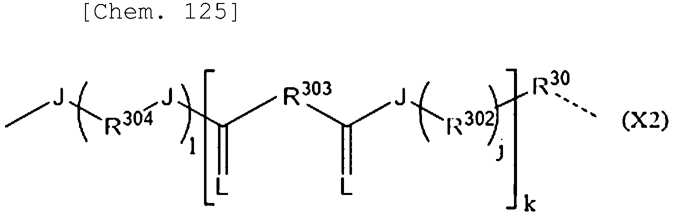
      in the Formula (X2), R³⁰ is a group represented by Formula (X2a) below; 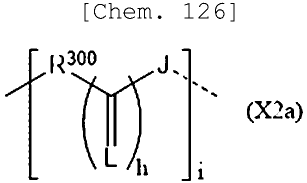 in the Formulae (X2) and (X2a),
         J is a divalent group, each independently being directly attached, a substituted or unsubstituted methylene group, an oxygen atom, a sulfur atom, or NR³⁰¹ in which R³⁰¹ is a hydrogen atom or an alkyl group;
         L is an oxygen atom or a sulfur atom;
         R³⁰⁰ is an alkylene group, or a silylene group having an alkyl group or an aryl group as a substituent;
         R³⁰², R³⁰³, and R³⁰⁴ are each independently an alkylene group;
         h, j, k, and l are each independently 0 or 1;
         i is an integer from 0 or more and 200 or less; when i is 2 or more, a plurality of R³⁰s may be the same as or different from each other; and a dashed line represents an attachment to R⁴⁰⁰; 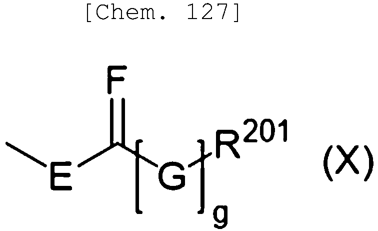 in the Formula (X),
         E is an oxygen atom or NR¹⁰¹ in which R¹⁰¹ is a hydrogen atom or an alkyl group;
         F is an oxygen atom or a sulfur atom;
         G is an oxygen atom, a sulfur atom, or NR²⁰² in which R²⁰² is a hydrogen atom, alkyl group, a cycloalkyl group, an aryl group, or a heteroaryl group;
         g is 0 or 1;
         R²⁰¹ is a hydrogen atom, an alkyl group, a cycloalkyl group, an aryl group, or a heteroaryl group; and
         when G is an oxygen atom or a sulfur atom, R²⁰¹ is a group other than a hydrogen atom.
[8] The photochromic compound according to any one of [1] to [7], in which, in the Formula (3), R⁵ and R⁶ are each independently a substituted or unsubstituted aryl group or a substituted or unsubstituted heteroaryl group.
[9] The photochromic compound according to any one of [1] to [8] represented by Formula (4) below: 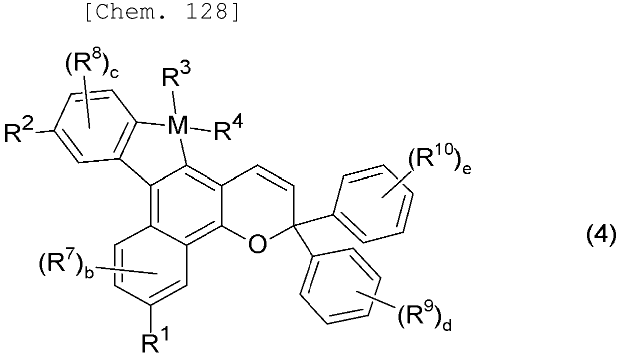 in which
   R¹, R², R³, R⁴, R⁷, R⁸, b, c, and M are each independently the same as in the Formula (3);
   R⁹ and R¹⁰ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by the Formula (2a), or a group represented by the Formula (X3);
   d is an integer of 0 or more and 5 or less;
   when d is 2 to 5, a plurality of R⁹s may be the same as or different from each other;
   when adjacent R⁹s are present, two adjacent R⁹s may be taken together with a carbon atom to which R⁹s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings;
   e is an integer of 0 or more and 5 or less;
   when e is 2 to 5, a plurality of R¹⁰s may be the same as or different from each other; and
   when adjacent R¹⁰s are present, two adjacent R¹⁰s may be taken together with a carbon atom to which R¹⁰s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings.
[10] The photochromic compound according to any one of [1] to [9], in which, in the Formula (3), R¹ is a hydrogen atom, a hydroxyl group, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3); and
   R² is a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).
[11] The photochromic compound according to any one of [1] to [10], in which, in the Formula (3), R¹ is a hydrogen atom, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a substituted or unsubstituted aryloxy group, a substituted or unsubstituted aryl group, a substituted or unsubstituted heteroaryl group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).
[12] The photochromic compound according to any one of [1] to [11] represented by Formula (5) below: 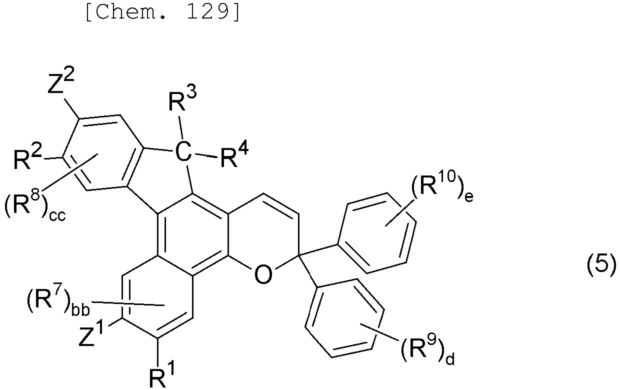 in which
   R¹, R², R³, R⁴, R⁷, R⁸, R⁹, R¹⁰, d, and e are each independently the same as in the Formula (4);
   Z¹ and Z² are each independently a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3);
   bb is an integer of 0 or more and 2 or less; cc is an integer of 0 or more and 2 or less;
   when bb is 2, a plurality of R⁷s may be the same as or different from each other; and
   when cc is 2, a plurality of R⁸s may be the same as or different from each other.
[13] The photochromic compound according to [12], in which, in the Formula (5), R² is a hydrogen atom, bb is 0, and cc is 0.
[14] The photochromic compound according to [12] or [13], in which, in the Formula (5), Z² is a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).
[15] A curable composition including:
   the photochromic compound according to any one of [1] to [14]; and
   at least one selected from the group consisting of a radical polymerizable monomer, a cationic polymerizable monomer, a compound having a polymerization reactive group, and a (thio)urethane(urea) polymer.
[16] A cured product of the curable composition according to [15].
[17] An optical article including the cured product according to [16].
[18] A lens including the photochromic compound according to any one of [1] to [14].
[19] Eyeglasses including the lens according to [18].

## Claims

1. A photochromic compound represented by Formula (3) below: 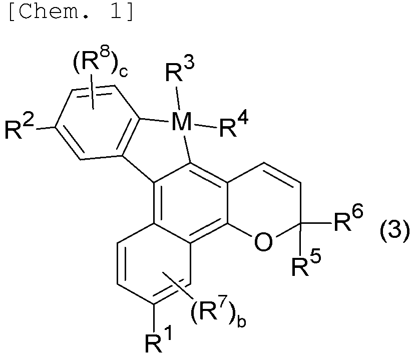
wherein
M is C, Si, or Ge;
R¹ is a hydrogen atom or a substituent, provided that the substituent is not a halogen atom or a trifluoromethyl group;
R² is a hydrogen atom or a substituent, provided that the substituent is not a halogen atom;
R³ and R⁴ are each independently a linear or branched alkyl group and have different structures from each other;
R⁵ and R⁶ are each independently a hydrogen atom or a substituent;
R⁷ and R⁸ are each independently a substituent; and
b and c are each independently an integer of 0, or 1 or more and 3 or less.

2. The photochromic compound according to claim 1, wherein, in the Formula (3), R³ is a linear or branched alkyl group having 1 to 19 carbon atoms, R⁴ is a linear or branched alkyl group having 2 to 20 carbon atoms, and a value obtained by subtracting a number of carbon atoms in the alkyl group for R³ from a number of carbon atoms in the alkyl group for R⁴ is 1 or more and 10 or less.

3. The photochromic compound according to claim 1, wherein, in the Formula (3), R³ is a linear alkyl group having 1 to 10 carbon atoms.

4. The photochromic compound according to claim 1, wherein, in the Formula (3), R³ is a linear alkyl group having 1 to 9 carbon atoms, R⁴ is a linear alkyl group having 2 to 10 carbon atoms, and a value obtained by subtracting a number of carbon atoms in the alkyl group for R³ from a number of carbon atoms in the alkyl group for R⁴ is 1 or more and 9 or less.

5. The photochromic compound according to claim 1, wherein, in the Formula (3), R³ is any of a methyl group, an ethyl group, or a propyl group.

6. The photochromic compound according to claim 1, wherein, in the Formula (3), one of R³ or R⁴ is an ethyl group.

7. The photochromic compound according to claim 1, wherein, in the Formula (3), R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) below, a group represented by Formula (X) below, or a group represented by Formula (X3) below;
in the Formula (3),
when b is 2 to 3, a plurality of R⁷s may be the same as or different from each other;
when c is 2 to 3, a plurality of R⁸s may be the same as or different from each other;
when b is 2 to 3 and adjacent R⁷s are present, two adjacent R⁷s may be taken together with a carbon atom to which R⁷s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings; and
when c is 2 to 3 and adjacent R⁸s are present, two adjacent R⁸s may be taken together with a carbon atom to which R⁸s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings;
-Q¹-(X¹Q²)ₐ-X²Q³ (2a)
in the Formula (2a),
Q¹ is an alkylene group or a haloalkylene group;
Q² is an alkylene group or a haloalkylene group;
Q³ is an alkyl group or a haloalkylene group;
X¹ and X² are each independently O, S, NR⁷⁰⁰, PR⁷⁰¹, or P (=O) ;
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group; and
a is an integer of 0, or 1 or more and 3 or less;
L¹-R⁴⁰⁰ (X3)
in the Formula (X3),
R⁴⁰⁰ is a hydrogen atom, an alkyl group, an aryl group, a polymerizable group, a photochromic group, or a silyl group having an alkyl group, an alkoxyl group, or an aryl group as a substituent; and
L¹ is a group represented by Formula (X2) below; 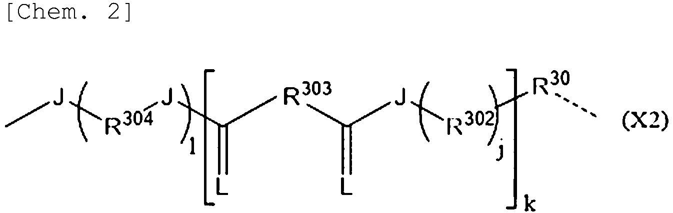
in the Formula (X2), R³⁰ is a group represented by Formula (X2a) below; 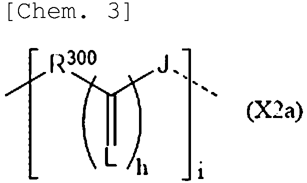
in the Formulae (X2) and (X2a),
J is a divalent group, each independently being directly attached, a substituted or unsubstituted methylene group, an oxygen atom, a sulfur atom, or NR³⁰¹ in which R³⁰¹ is a hydrogen atom or an alkyl group;
L is an oxygen atom or a sulfur atom;
R³⁰⁰ is an alkylene group, or a silylene group having an alkyl group or an aryl group as a substituent;
R³⁰², R³⁰³, and R³⁰⁴ are each independently an alkylene group;
h, j, k, and l are each independently 0 or 1;
i is an integer from 0 or more and 200 or less; when i is 2 or more, a plurality of R³⁰s may be the same as or different from each other; and a dashed line represents an attachment to R⁴⁰⁰; 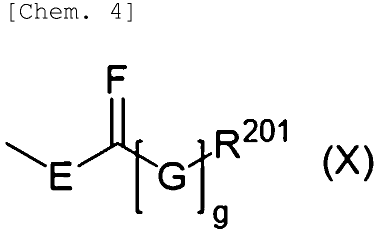
in the Formula (X),
E is an oxygen atom or NR¹⁰¹ in which R¹⁰¹ is a hydrogen atom or an alkyl group;
F is an oxygen atom or a sulfur atom;
G is an oxygen atom, a sulfur atom, or NR²⁰² in which R²⁰² is a hydrogen atom, alkyl group, a cycloalkyl group, an aryl group, or a heteroaryl group;
g is 0 or 1;
R²⁰¹ is a hydrogen atom, an alkyl group, a cycloalkyl group, an aryl group, or a heteroaryl group; and
when G is an oxygen atom or a sulfur atom, R²⁰¹ is a group other than a hydrogen atom.

8. The photochromic compound according to claim 1, wherein, in the Formula (3), R⁵ and R⁶ are each independently a substituted or unsubstituted aryl group or a substituted or unsubstituted heteroaryl group.

9. The photochromic compound according to claim 7, represented by Formula (4) below: 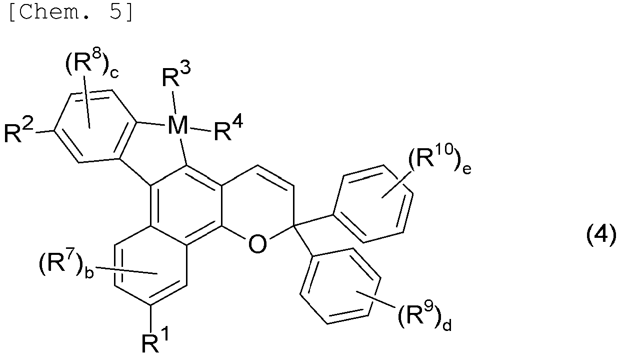 wherein
R¹, R², R³, R⁴, R⁷, R⁸, b, c, and M are each independently the same as in the Formula (3);
R⁹ and R¹⁰ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by the Formula (2a), or a group represented by the Formula (X3);
d is an integer of 0 or more and 5 or less;
when d is 2 to 5, a plurality of R⁹s may be the same as or different from each other;
when adjacent R⁹s are present, two adjacent R⁹s may be taken together with a carbon atom to which R⁹s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings;
e is an integer of 0 or more and 5 or less;
when e is 2 to 5, a plurality of R¹⁰s may be the same as or different from each other; and
when adjacent R¹⁰s are present, two adjacent R¹⁰s may be taken together with a carbon atom to which R¹⁰s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring constituted of an aromatic ring or an aromatic heterocyclic ring fused to any of the above-mentioned rings.

10. The photochromic compound according to claim 7, wherein, in the Formula (3), R¹ is a hydrogen atom, a hydroxyl group, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3); and
R² is a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).

11. The photochromic compound according to claim 7, wherein, in the Formula (3), R¹ is a hydrogen atom, a substituted or unsubstituted alkyl group, a substituted or unsubstituted cycloalkyl group, a substituted or unsubstituted alkoxy group, a substituted or unsubstituted amino group, a substituted or unsubstituted heterocyclic group, a cyano group, a substituted or unsubstituted alkylthio group, a substituted or unsubstituted arylthio group, a substituted or unsubstituted aryloxy group, a substituted or unsubstituted aryl group, a substituted or unsubstituted heteroaryl group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).

12. The photochromic compound according to claim 9 represented by Formula (5) below: 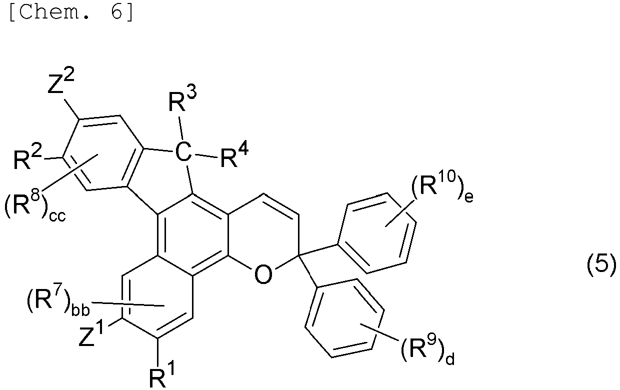 wherein
R¹, R², R³, R⁴, R⁷, R⁸, R⁹, R¹⁰, d, and e are each independently the same as in the Formula (4);
Z¹ and Z² are each independently a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3);
bb is an integer of 0 or more and 2 or less; cc is an integer of 0 or more and 2 or less;
when bb is 2, a plurality of R⁷s may be the same as or different from each other; and
when cc is 2, a plurality of R⁸s may be the same as or different from each other.

13. The photochromic compound according to claim 12, wherein, in the Formula (5), R² is a hydrogen atom, bb is 0, and cc is 0.

14. The photochromic compound according to claim 12, wherein, in the Formula (5), Z² is a hydrogen atom, a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by the Formula (2a), a group represented by the Formula (X), or a group represented by the Formula (X3).

15. A curable composition comprising:
the photochromic compound according to claim 1; and
at least one selected from the group consisting of a radical polymerizable monomer, a cationic polymerizable monomer, a compound having a polymerization reactive group, and a (thio)urethane(urea) polymer.

16. A cured product of the curable composition according to claim 15.

17. An optical article comprising the cured product according to claim 16.

18. A lens comprising the photochromic compound according to claim 1.

19. Eyeglasses comprising the lens according to claim 18.
